# EUROPEAN PATENT APPLICATION

(11) **EP 4 722 216 A1**
(43) Date of publication of application: **08.04.2026**
(21) Application number: 24818679.3
(22) Date of filing: 05.06.2024
(51) Int. Cl.: C07D 487/04, A61K 31/519, A61P 35/00

(54) **PYRROLOPYRIMIDINE DERIVATIVE AND USE THEREOF IN MEDICINE**

(30) Priority: 05.06.2023 CN 202310658738; 19.06.2023 CN 202310723848; 11.09.2023 CN 202311164718; 25.10.2023 CN 202311397456; 25.10.2023 CN 202311397521; 10.01.2024 CN 202410036765; 07.02.2024 CN 202410173207; 18.03.2024 CN 202410306868
(71) Applicant: Kangbaida (Sichuan) Biotechnology Co., Ltd., Chengdu, Sichuan 610000 (CN)
(72) Inventor: ZHANG, Jing, Chengdu, Sichuan 610000 (CN); WEI, Yonggang, Chengdu, Sichuan 610000 (CN); ZHOU, Xibing, Chengdu, Sichuan 610000 (CN); CHU, Hongzhu, Chengdu, Sichuan 610000 (CN); SUN, Yi, Chengdu, Sichuan 610000 (CN)
(74) Representative: Dehns
(86) International application number: PCT/CN2024/097505
(87) International publication number: WO 2024/251147

(57) **Abstract**

The present invention relates to a pyrrolopyrimidine derivative represented by general formula (I) or a pharmaceutically acceptable salt, stereoisomer or deuterated compound thereof, a composition thereof, and a use thereof in medicine.

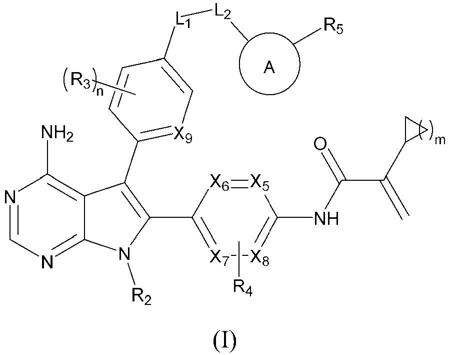

## Description

### TECHNICAL FIELD

The present invention relates to a pyrrolopyrimidine derivative and a use thereof in medicine.

### BACKGROUND ART

FGFRs, i.e., fibroblast growth factor receptors, are a family composed of four homologous and highly conserved transmembrane tyrosine kinase receptors (FGFR1-4). Upon binding of FGFRs to their ligands, i.e., fibroblast growth factors (FGFs), FGFRs undergo dimerization and autophosphorylation, leading to the activation of downstream signaling pathways such as JAK/STAT pathway and phospholipase C pathway. These signaling pathways play critical roles in various physiological processes, such as cell proliferation, differentiation, migration, and apoptosis. FGFR fusion and dysregulated expression are closely associated with the development and progression of various cancers. To date, FGFR gene alterations have been reported in more than ten types of malignant tumors.

Currently available drugs towards the target on the market are all Pan-FGFR inhibitors and have shown relatively limited therapeutic effects, with more than 50% of patients exhibiting insufficient response. These drugs have shown acquired drug resistance problem. In addition, these drugs have relatively highly toxic and side effects, primarily manifested as hyperphosphatemia, diarrhea, etc., which are caused by the targeting effect of FGFR1/4 inhibition. Therefore, there remains a need for the development of highly active and highly selective next-generation FGFR2 and/or FGFR3 inhibitors, as well as methods for treating cancer and other diseases.

### SUMMARY OF THE INVENTION

An object of the present invention is to provide a pyrrolopyrimidine derivative as an FGFR2 and/or FGFR3 inhibitor and a use thereof in the preparation of an anti-tumor drug.

One or more embodiments of the present invention provide a compound represented by general formula (I), or a pharmaceutically acceptable salt, stereoisomer, or deuterated compound thereof: 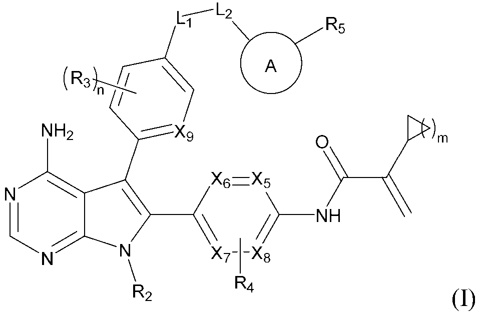 wherein:
X₅ is CH or N;
X₆ is CH or N;
X₇ is CH or N;
X₈ is CH or N;
X₉ is CH or N;
R₂ is C₁₋₆ alkyl;
each R₃ is independently H, halogen, cyano, C₁₋₆ alkyl, or C₁₋₆ alkoxy, wherein the C₁₋₆ alkyl is optionally substituted with 1 to 3 halogens;
R₄ is H, halogen, cyano, C₁₋₆ alkyl, or C₁₋₆ alkoxy, wherein the C₁₋₆ alkyl is optionally substituted with 1 to 3 halogens;
R₅ is H, halogen, cyano, C₁₋₆ alkyl, C₁₋₆ alkoxy, C₂₋₆ alkenyl, or C₂₋₆ alkynyl, wherein the C₁₋₆ alkyl, C₂₋₆ alkenyl, and C₂₋₆ alkynyl are optionally substituted with 1 to 3 substituents selected from halogen or C₁₋₆ alkyl;
L₁ is O, 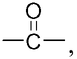 or -O(CO)-;
L₂ is a bond, 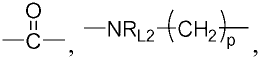 or C₁₋₆ alkyl;
R_{L2} is H or C₁₋₆ alkyl;
A is C₃₋₈ heterocycle or C₃₋₈ cycloalkyl, wherein the C₃₋₈ heterocycle contains 1 to 3 heteroatoms selected from N, O, or S, and the C₃₋₈ heterocycle is optionally substituted with carbonyl;
n is 0, 1, 2, or 3;
m is 1, 2, 3, or 4; and
p is 0, 1, or 2.

In one or more embodiments of the present invention,
A is

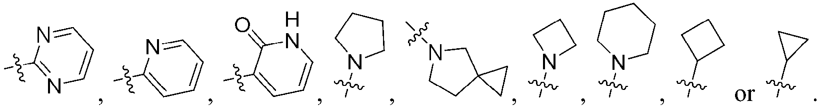

In one or more embodiments of the present invention, the compound has a structure represented by formula (I-1): 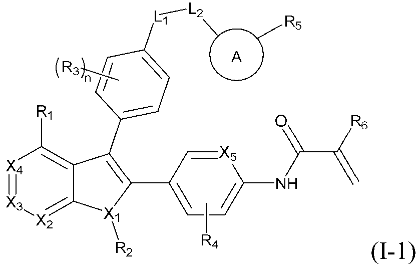 wherein:
X₁ is N;
X₂ is N;
X₃ is CH;
X₄ is N;
X₅ is CH or N;
R₁ is NH₂;
R₂ is C₁₋₆ alkyl;
each R₃ is independently H, halogen, cyano, or C₁₋₆ alkyl, wherein the C₁₋₆ alkyl is optionally substituted with 1 to 3 halogens;
R₄ is H or halogen;
R₅ is H, halogen, cyano, C₁₋₆ alkyl, C₂₋₆ alkenyl, or C₂₋₆ alkynyl, wherein the C₁₋₆ alkyl, C₂₋₆ alkenyl, and C₂₋₆ alkynyl are optionally substituted with 1 to 3 substituents selected from halogen or C₁₋₆ alkyl;
R₆ is C₃₋₆ cycloalkyl, preferably 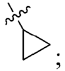
L₁ is O;
L₂ is a bond or 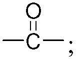
A is C₃₋₈ heterocycle, preferably 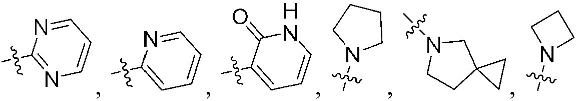 or 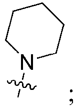
n is 0, 1, 2, or 3.

In one or more embodiments of the present invention, the compound has a structure represented by formula (I-2): 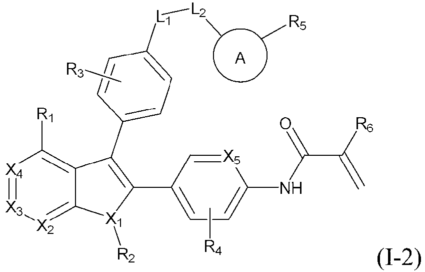 wherein:
X₁ is N;
X₂ is N;
X₃ is CH;
X₄ is N;
X₅ is CH or N;
R₁ is NH₂;
R₂ is C₁₋₆ alkyl;
R₃ is H, halogen, cyano, or C₁₋₆ alkyl, wherein the C₁₋₆ alkyl is optionally substituted with 1 to 3 halogens;
R₄ is H or halogen;
R₅ is H, halogen, cyano, C₁₋₆ alkyl, C₂₋₆ alkenyl, or C₂₋₆ alkynyl, wherein the C₁₋₆ alkyl, C₂₋₆ alkenyl, and C₂₋₆ alkynyl are optionally substituted with 1 to 3 substituents selected from halogen or C₁₋₆ alkyl;
R₆ is 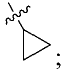
L₁ is O;
L₂ is a bond; and
A is

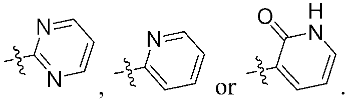

In one or more embodiments of the present invention, the compound has a structure represented by formula (1-2): 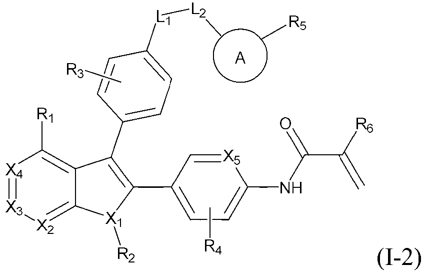 wherein:
X₁ is N;
X₂ is N;
X₃ is CH;
X₄ is N;
X₅ is CH or N;
R₁ is NH₂;
R₂ is C₁₋₆ alkyl;
R₃ is H, halogen, cyano, or C₁₋₆ alkyl, wherein the C₁₋₆ alkyl is optionally substituted with 1 to 3 halogens;
R₄ is H or halogen;
R₅ is H, halogen, cyano, C₁₋₆ alkyl, C₂₋₆ alkenyl, or C₂₋₆ alkynyl, wherein the C₁₋₆ alkyl, C₂₋₆ alkenyl, and C₂₋₆ alkynyl are optionally substituted with 1 to 3 substituents selected from halogen or C₁₋₆ alkyl;
R₆ is 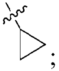
L₁ is -O(CO)-;
L₂ is a bond, NR_{L2}, or C₁₋₆ alkyl;
R_{L2} is H or C₁₋₆ alkyl;
A is C₃₋₈ cycloalkyl or C₃₋₈ heterocycloalkyl, wherein the C₃₋₈ heterocycloalkyl contains 1 to 3 heteroatoms selected from N, O, or S.

In one or more embodiments of the present invention, the compound or the pharmaceutically acceptable salt, stereoisomer, or deuterated compound thereof is selected from, but not limited to, the following structures:

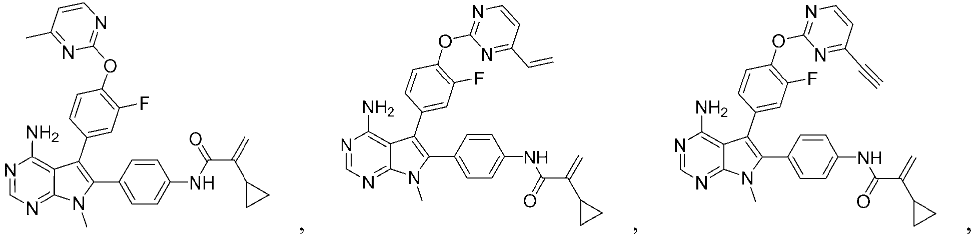

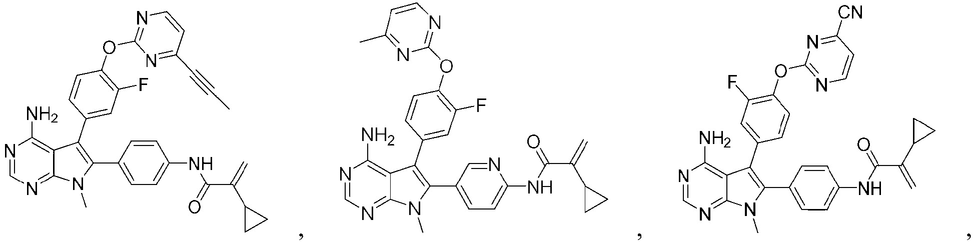

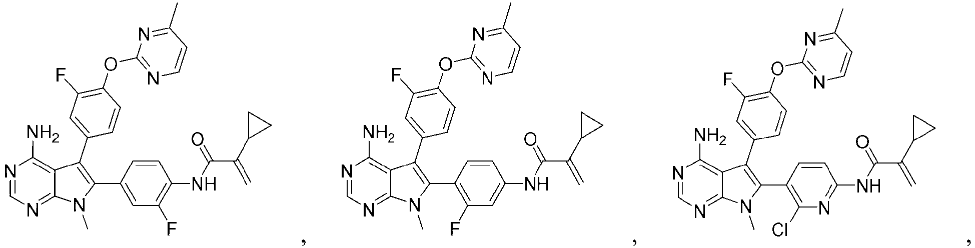

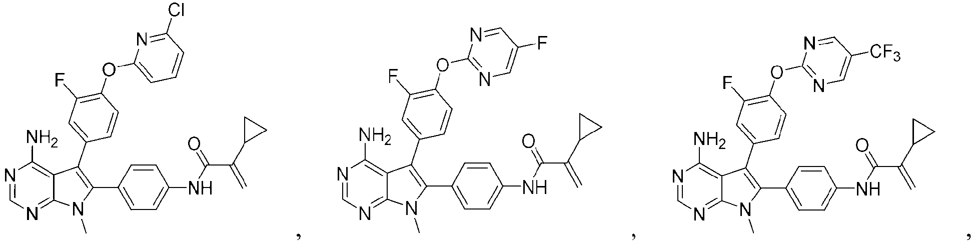

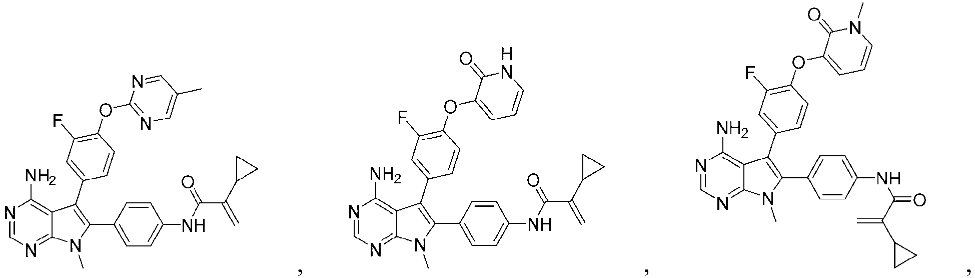

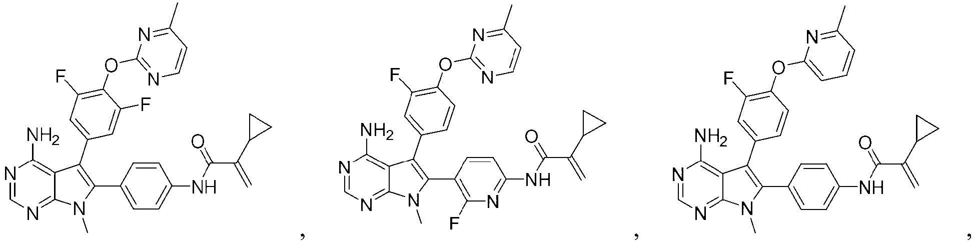

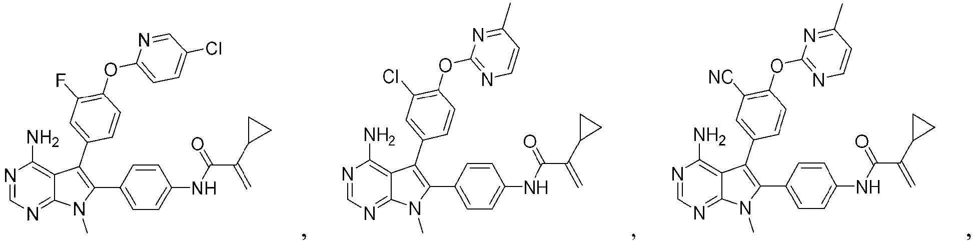

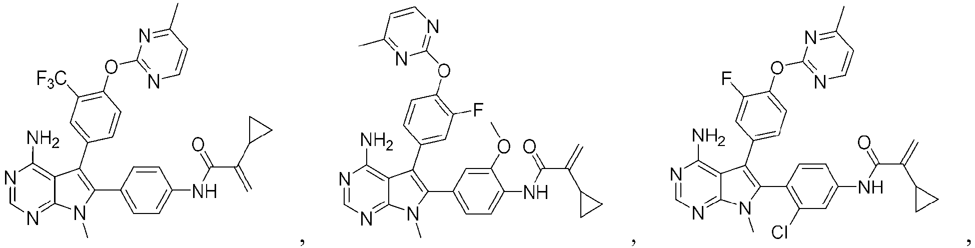

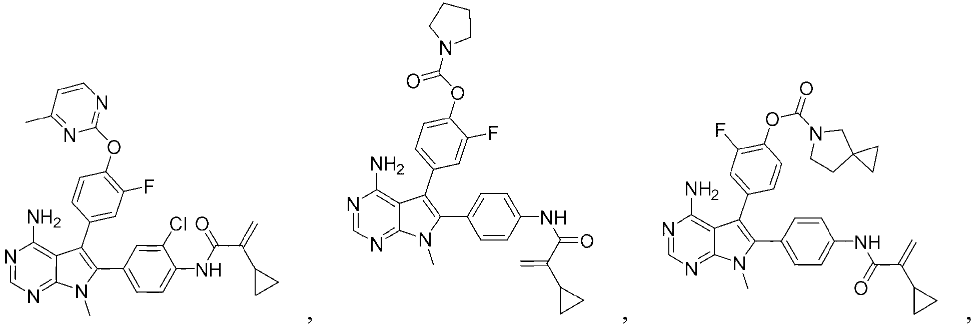

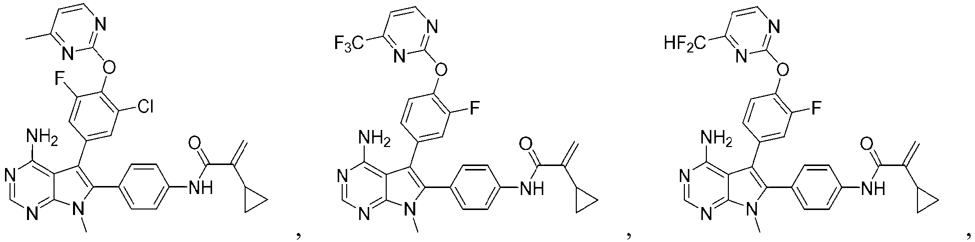

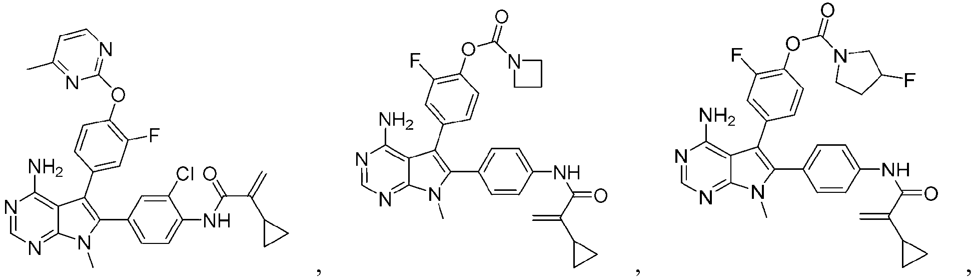

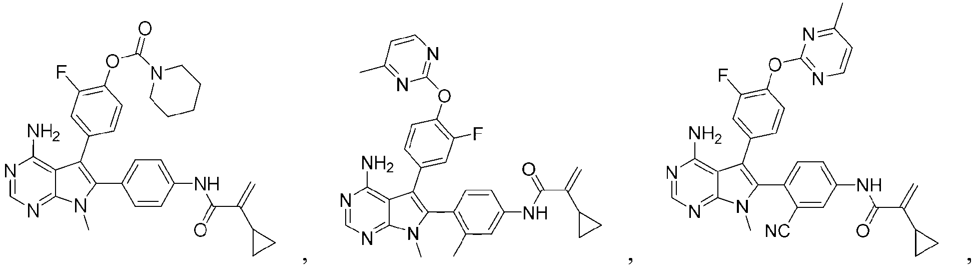

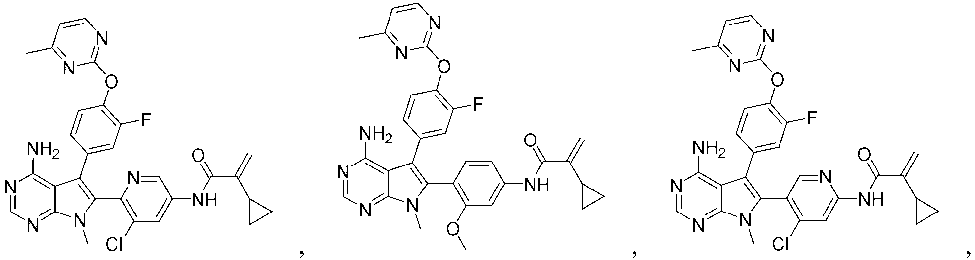

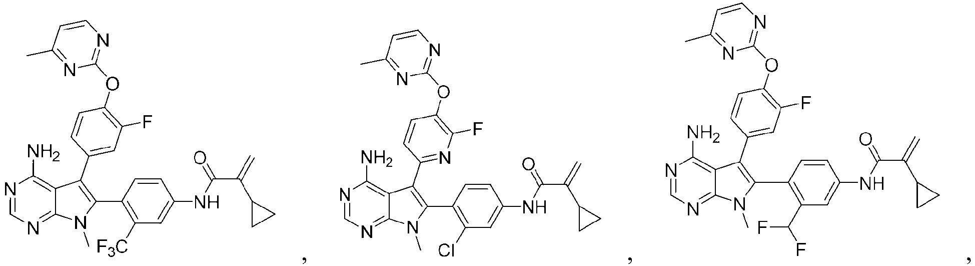

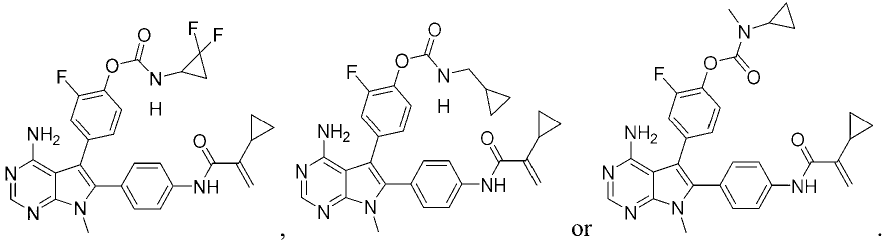

One or more embodiments of the present invention provide a pharmaceutical composition comprising:
(1) the compound or the pharmaceutically acceptable salt, stereoisomer, or deuterated compound thereof according to the present invention:
(2) optionally one or more additional active ingredients; and
(3) a pharmaceutically acceptable carrier and/or excipient.

One or more embodiments of the present invention provide a use of the pharmaceutical composition, or the compound or the pharmaceutically acceptable salt, stereoisomer, or deuterated compound thereof according to the present invention in the preparation of an anti-tumor drug.

Unless stated to the contrary, the terms used in the specification and claims have the following meanings.

The carbon, hydrogen, oxygen, sulfur, nitrogen, F, Cl, Br, and I involved in the groups and compounds described in the present invention all include isotopes thereof, and the carbon, hydrogen, oxygen, sulfur, or nitrogen involved in the groups and compounds described in the present invention is optionally further replaced by one or more corresponding isotopes thereof, wherein isotopes of carbon include ¹²C, ¹³C, and ¹⁴C, isotopes of hydrogen include protium (H), deuterium (D, also called heavy hydrogen), and tritium (T, also called superheavy hydrogen), isotopes of oxygen include ¹⁶O, ¹⁷O, and ¹⁸O, isotopes of sulfur include ³²S, ³³S, ³⁴S, and ³⁶S, isotopes of nitrogen include ¹⁴N and ¹⁵N, isotopes of fluorine include ¹⁷F and ¹⁹F, isotopes of chlorine include ³⁵Cl and ³⁷Cl, and isotopes of bromine include ⁷⁹Br and ⁸¹Br.

"Alkyl" refers to a linear or branched saturated aliphatic hydrocarbon group having 1 to 20 carbon atoms, preferably an alkyl group having 1 to 8 (e.g., 1, 2, 3, 4, 5, 6, 7, or 8) carbon atoms, more preferably an alkyl group having 1 to 6 carbon atoms, further preferably an alkyl group having 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl, and various branched isomers thereof. When the alkyl is substituted, it may be optionally further substituted with 1 or more substituents.

"Alkoxy" refers to a group formed by the substitution of at least 1 carbon atom of an alkyl group with an oxygen atom. Non-limiting examples include methoxy, ethoxy, n-propoxy, isopropoxy, n-butoxy, sec-butoxy, tert-butoxy, n-pentoxy, n-hexoxy, cyclopropoxy, and cyclobutoxy. The alkyl is defined in the same way as for the "alkyl" described above.

"Alkenyl" refers to a linear or branched unsaturated aliphatic hydrocarbon group containing 1 to 10 (e.g., 1, 2, 3, 4, 5, 6, 7, 8, 9, or 10) carbon-carbon double bonds and consisting of 2 to 20 carbon atoms, preferably an alkenyl group having 2 to 12 (e.g., 2, 3, 4, 5, 6, 7, 8, 9, 10, 11, or 12) carbon atoms, more preferably an alkenyl group having 2 to 8 carbon atoms, further preferably an alkenyl group having 2 to 6 carbon atoms. Non-limiting examples include vinyl, propen-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl, and undecen-3-yl. The alkenyl may be optionally further substituted with 1 or more substituents.

"Alkynyl" refers to a linear or branched unsaturated aliphatic hydrocarbon group containing 1 to 10 (e.g., 1, 2, 3, 4, 5, 6, 7, 8, 9, or 10) carbon-carbon triple bonds and consisting of 2 to 20 carbon atoms, preferably an alkynyl group having 2 to 12 (e.g., 2, 3, 4, 5, 6, 7, 8, 9, 10, 11, or 12) carbon atoms, more preferably an alkynyl group having 2 to 8 carbon atoms, further preferably an alkynyl group having 2 to 6 carbon atoms. Non-limiting examples include ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyn-3-yl, nonyn-3-yl, decyn-4-yl, undec-3-yl, and dodecyn-4-yl. The alkynyl may be optionally further substituted with one or more substituents.

"Cycloalkyl" refers to a saturated cyclic hydrocarbon group, the ring of which may be a 3- to 10-membered (e.g., 3-, 4-, 5-, 6-, 7-, 8-, 9-, or 10-membered) monocyclic ring, a 4- to 12-membered (e.g., 4-, 5-, 6-, 7-, 8-, 9-, 10-, 11-, or 12-membered) bicyclic ring or a 10- to 20-membered (e.g., 10-, 11-, 12-, 13-, 14-, 15-, 16-, 17-, 18-, 19-, or 20-membered) polycyclic ring system, and has preferably 3 to 10 ring carbon atoms, and more preferably 3 to 8 ring carbon atoms. Non-limiting examples of "cycloalkyl" include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl, cycloheptatrienyl, etc. When the cycloalkyl is substituted, it may be optionally further substituted with 1 or more substituents.

"Heterocyclyl" or "heterocycle" refers to a saturated or unsaturated aromatic heterocyclic or non-aromatic heterocyclic ring. When it is an aromatic heterocyclic ring, the definition thereof is the same as that of "heteroaryl" above; and when it is a non-aromatic heterocyclic ring, it may be a 3- to 10-membered (e.g., 3, 4, 5, 6, 7, 8, 9, or 10-membered) monocyclic, 4- to 12-membered (e.g., 4, 5, 6, 7, 8, 9, 10, 11, or 12-membered) bicyclic, or 10- to 15-membered (e.g., 10, 11, 12, 13, 14, or 15 membered) tricyclic ring system containing 1 to 4 (e.g., 1, 2, 3, or 4) heteroatoms selected from N, O, or S, preferably 3- to 8-membered heterocyclyl. 1 to 4 (e.g., 1, 2, 3, or 4) N and S optionally substituted in the ring of the "heterocyclyl" or "heterocycle" can be oxidized into various oxidation states; "heterocyclyl" or "heterocycle" may be attached to a heteroatom or a carbon atom; and "heterocyclyl" or "heterocycle" may be a bridged ring or a spiro ring. Non-limiting examples of "heterocyclyl" or "heterocycle" include epoxyethyl, epoxypropyl, aziridinyl, oxetanyl, azetidinyl, thietanyl, 1,3-dioxolanyl, 1,4-dioxolanyl, 1,3-dioxanyl, azepanyl, oxepanyl, thiepanyl, oxoazepinyl, diazepinyl, thiazepinyl, pyridinyl, homopiperidinyl, furanyl, thienyl, pyranyl, N-alkylpyrrolyl, pyrimidinyl, pyrazinyl, pyridazinyl, piperazinyl, homopiperazinyl, imidazolyl, piperidinyl, morpholinyl, thiomorpholinyl, oxathianyl, 1,3-dithianyl, dihydrofuranyl, dithiacyclopentyl, tetrahydrofuranyl, tetrahydrothienyl, tetrahydropyridinyl, tetrahydrothiopyranyl, tetrahydropyrrolyl, tetrahydroimidazolyl, tetrahydrothiazolyl, tetrahydropyranyl, benzimidazolyl, benzopyridinyl, pyrrolopyridinyl, pyrazolopyrimidinyl, imidazopyrazinyl, benzodihydrofuranyl, 2-pyrrolinyl, 3-pyrrolinyl, indolinyl, 2H-pyranyl, 4H-pyranyl, dioxacyclohexyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3H-indolylquinolizinyl, N-pyridinylurea, 1,1-dioxothiomorpholinyl, azabicyclo[3.2.1]octyl, azabicyclo[5.2.0]nonyl, oxatricyclo[5.3.1.1]dodecyl, aza-adamantyl, and oxaspiro[3.3]heptyl. The "heterocyclyl" or "heterocycle" may be optionally further substituted with one or more substituents.

When the "alkyl", "alkoxy", "alkenyl", "alkynyl", "cycloalkyl", "heterocyclyl", or "heterocycle" described above is substituted, it can be further substituted with 0, 1, 2, 3, 4, 5, 6, 7, 8, 9, or 10 substituents selected from F, Cl, Br, I, hydroxyl, mercapto, nitro, cyano, amino, C₁₋₆ alkylamino, =O, C₁₋₆ alkyl, C₁₋₆ alkoxy, C₂₋₆ alkenyl, C₂₋₆ alkynyl, -NR_{q4}R_{q5}, =NR_{q6}, -C(=O)OC₁₋₆ alkyl, -OC(=O)C₁₋₆ alkyl, -C(=O)NR_{q4}R_{q5}, C₃₋₈ cycloalkyl, C₃₋₈ heterocycloalkyl, C₆₋₁₀ aryl, C₅₋₁₀ heteroaryl, -C(=O)OC₆₋₁₀ aryl, -OC(=O)C₆₋₁₀ aryl, -OC(=O)C₅₋₁₀ heteroaryl, -C(=O)OC₅₋₁₀ heteroaryl, -OC(=O)C₃₋₈ heterocycloalkyl, -C(=O)OC₃₋₈ heterocycloalkyl, -OC(=O)C₃₋₈ cycloalkyl, -C(=O)OC₃₋₈ cycloalkyl, -NHC(=O)C₃₋₈ heterocycloalkyl, -NHC(=O)C₆₋₁₀ aryl, -NHC(=O)C₅₋₁₀ heteroaryl, -NHC(=O)C₃₋₈ cycloalkyl, -NHC(=O)C₃₋₈ heterocycloalkyl, -NHC(=O)C₂₋₆ alkenyl, or -NHC(=O)C₂₋₆ alkynyl, and the C₁₋₆ alkyl, C₁₋₆ alkoxy, C₂₋₆ alkenyl, C₂₋₆ alkynyl, C₃₋₈ cycloalkyl, C₃₋₈ heterocycloalkyl, C₆₋₁₀ aryl, C₅₋₁₀ heteroaryl, -NHC(=O)C₆₋₁₀ aryl, -NHC(=O)C₅₋₁₀ heteroaryl, -NHC(=O)C₃₋₈ heterocycloalkyl, or -NHC(=O)C₃₋₈ cycloalkyl substituent is optionally further substituted with 1 to 3 substituents selected from OH, F, Cl, Br, I, C₁₋₆ alkyl, C₁₋₆ alkoxy, -NR_{q4}R_{q5}, or =O; R_{q1} is selected from C₁₋₆ alkyl, C₁₋₆ alkoxy, or C₆₋₁₀ aryl; R_{q2} and R_{q3} are selected from H or C₁₋₆ alkyl; R_{q4} and R_{q5} are selected from H, C₁₋₆ alkyl, -NH(C=NR_{q1})NR_{q2}R_{q3}, -S(=O)₂NR_{q2}R_{q3}, - C(=O)R_{q1}, or -C(=O)NR_{q2}R_{q3}, wherein the C₁₋₆ alkyl is optionally further substituted with 1 or more substituents selected from OH, F, Cl, Br, I, C₁₋₆ alkyl, C₁₋₆ alkoxy, C₆₋₁₀ aryl, C₅₋₁₀ heteroaryl, C₃₋₈ cycloalkyl, or C₃₋₈ heterocycloalkyl; or R_{q4} and R_{q5}, together with an N atom, form a 3- to 8-membered heterocycle, which may contain 1 or more heteroatoms selected from N, O, or S.

Halogen includes F, Cl, Br, and I.

"Pharmaceutically acceptable salt" or "pharmaceutically acceptable salt thereof" refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention or pharmaceutically acceptable salts or prodrugs thereof and other chemical components, wherein "other chemical components" refer to pharmaceutically acceptable carriers, excipients and/or one or more other therapeutic agents.

"Carrier" refers to a material that does not cause significant irritation to an organism and does not eliminate the biological activity and characteristics of the administered compound.

"Excipient" refers to an inert substance added to a pharmaceutical composition to facilitate administration of a compound. Non-limiting examples include calcium carbonate, calcium phosphate, sugar, starch, cellulose derivatives (including microcrystalline cellulose), gelatin, vegetable oils, polyethylene glycols, diluents, granulating agents, lubricants, adhesives and disintegrants.

"Stereoisomer" refers to isomers resulting from different spatial arrangements of atoms in a molecule, including cis/trans isomers, enantiomers, and conformers.

"Optional", "optionally", "selective", or "selectively" means that the subsequently described event or circumstance may, but not necessarily, occur, and the description includes cases where the event or circumstance occurs and cases where it does not. For example, "heterocyclyl optionally substituted with alkyl" means that the alkyl may be, but not necessarily, present, and the description includes the case where the heterocyclyl is substituted with alkyl and the case where the heterocyclyl is not substituted with alkyl.

### BRIEF DESCRIPTION OF THE DRAWINGS

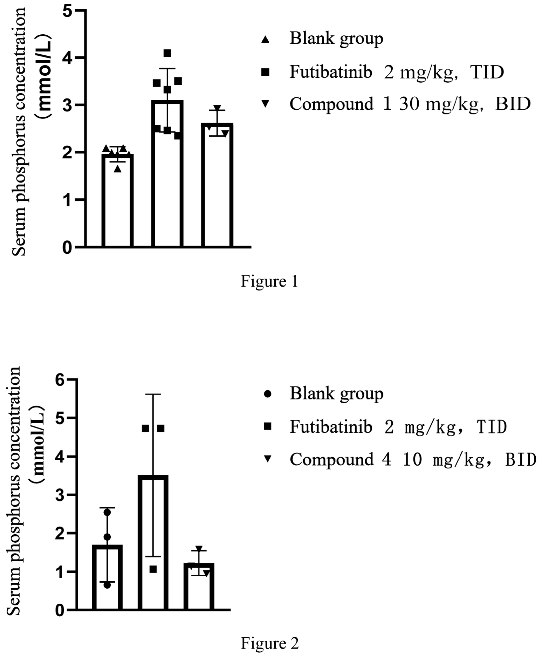

FIG. 1 shows serum phosphorus concentration I in tumor-bearing mice.
FIG. 2 shows serum phosphorus concentration II in tumor-bearing mice.

### DETAILED DESCRIPTION OF EMBODIMENTS

The technical solution of the present invention will be explained in detail by the following examples; however, the scope of protection of the present invention includes but is not limited thereto.

The structures of the compounds are determined by nuclear magnetic resonance (NMR) or (and) mass spectrometry (MS). The NMR shift (δ) is given in 10-6 (ppm). NMR is determined with (Bruker Avance III 400 and Bruker Avance 300) nuclear magnetic resonance instrument; the solvents for determination are deuterated dimethyl sulfoxide (DMSO-d6), deuterated chloroform (CDCl3) and deuterated methanol (CD3OD); and the internal standard is tetramethylsilane (TMS).

MS measurement is carried out using Agilent 6120B (ESI) and Agilent 6120B (APCI).

HPLC measurement is carried out using Agilent 1260DAD high-pressure liquid chromatograph (Zorbax SB-C18 100 × 4.6 mm, 3.5 µM).

Yantai Huanghai HSGF254 or Qingdao GF254 silica gel plate is used as a thin layer chromatography silica plate. The specification of the silica gel plate for thin layer chromatography (TLC) is 0.15 mm-0.20 mm. The specification used when product separation and purification are carried out by thin layer chromatography is 0.4 mm-0.5 mm.

For column chromatography, Yantai Huanghai silica gel of 200-300 mesh silica gel is generally used as a carrier.

Known starting materials in the present invention can be synthesized by or according to methods known in the art, or can be purchased from companies, such as Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd., and Jiangsu Aikon Biopharmaceutical R&D Co., Ltd.

### Intermediate 1

### 2-Cyclopropyl-N-(5-(4,4,5,5-tetramethyl-1,3,2-dioxaborolan-2-yl)pyridin-2-yl)acrylamide intermediate 1

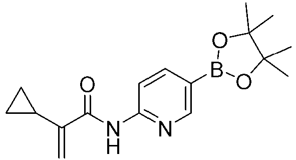

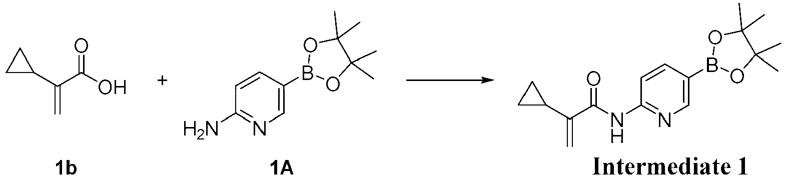

With reference to the synthesis method for an intermediate of compound **2** in a reference (Organic Letters, 2014. 16, 22. 5956 - 5959.), 2-cyclopropylacrylic acid **1b** is obtained.

2-Cyclopropylacrylic acid **1b** (500 mg, 4.9 mmol) was dissolved in acetonitrile (15 mL), and N,N,N',N'-tetramethylchloroformamidinium hexafluorophosphate (1.37 g, 4.9 mmol) and N-methylimidazole (837 mg, 10 mmol) were added. After the mixture was reacted at room temperature for 30 minutes, compound **1A** (900 mg, 4.08 mmol) was added. After the reaction flask underwent nitrogen displacement, a reaction was carried out at room temperature for 2 hours. The reaction mixture was diluted with water (15 mL) and extracted with ethyl acetate (3 × 30 mL). After the extraction was complete, the organic phase was washed with a saturated aqueous sodium chloride solution (3 × 30 mL), dried over anhydrous sodium sulfate, and filtered. The organic phases were combined and concentrated under reduced pressure to obtain **intermediate 1** (as a brown solid, 280 mg, yield 21.7%).

LCMS m/z = 315.18 [M+1].

### Intermediate 2

### 2-Cyclopropylacryloyl chloride intermediate 2

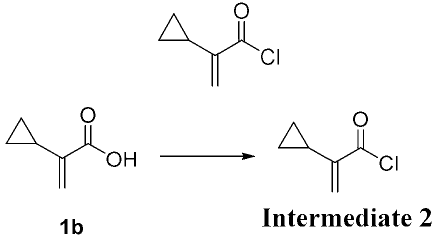

2-Cyclopropylacrylic acid **1b** (2.5 g, 22.2 mmol) was dissolved in dichloromethane (70 mL). After the system was cooled to 0°C, oxalyl chloride (2.54 g, 20 mmol) and 3 drops of N,N-dimethylformamide were added. The mixture was reacted at room temperature for 4 hours to obtain a solution of **intermediate 2,** which was directly used in the next reaction after concentration.

### Intermediate 3

### 7-Methyl-5-(4,4,5,5-tetramethyl-1,3,2-dioxaborolan-2-yl)-7H-pyrrolo[2,3-d]pyrimidin-4-amine intermediate 3

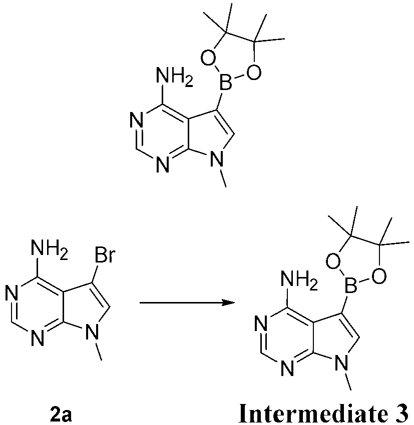

5-Bromo-7-methyl-7H-pyrrolo[2,3-d]pyrimidin-4-amine **2a** (1.5 g, 3.51 mmol), pinacol borane (2.7 g, 21 mmol), triethylamine (2.13 g, 21 mmol), tris(dibenzylideneacetone)dipalladium (644 mg, 0.7 mmol), and 2-dicyclohexylphosphino-2',4',6'-triisopropylbiphenyl (670 mg, 1.4 mmol) were dissolved in 1,4-dioxane (20 mL). After nitrogen displacement, the reaction flask was placed in an oil bath at 80°C for a reaction. After 8 hours of reaction, the reaction solution was filtered and concentrated. Water (300 mL) and ethyl acetate (3 × 150 mL) were added, and layers were separated. The organic layer was washed with brine (400 mL), dried and concentrated. The crude product was purified by column chromatography (petroleum ether: ethyl acetate = 5 : 1) to obtain **intermediate 3** (as a black solid, 1 g, yield 60%).

LCMS m/z = 275.16 [M+1].

### Example 1

### N-(4-(4-amino-5-(3-fluoro-4-((4-methylpyrimidin-2-yl)oxy)phenyl)-7-methyl-7H-pyrrolo[2,3-d]pyrimidin-6-yl)phenyl]-2-cyclopropylacrylamide compound 1

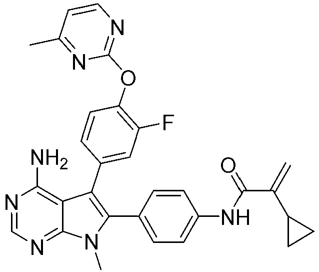

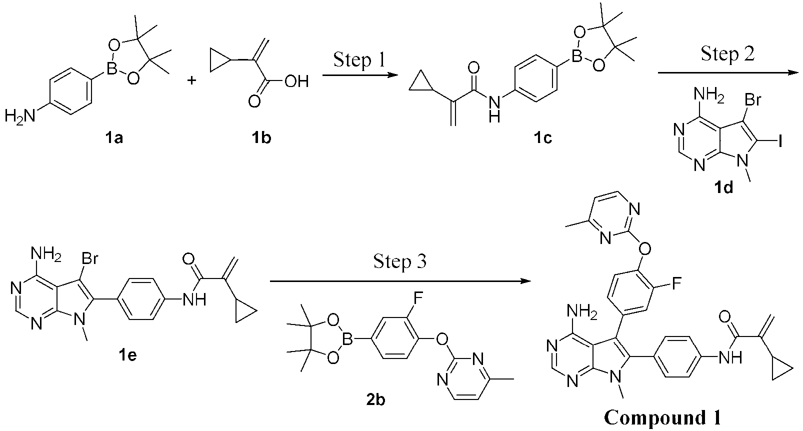

### Step 1

### 2-Cyclopropyl-N-(4-(4,4,5,5-tetramethyl-1,3,2-dioxaborolan-2-yl)phenyl)acrylamide 1c

Compound **1a** (1.96 g, 8.9 mmol) and 2-cyclopropylacrylic acid **1b** (1 g, 9.0 mmol) were dissolved in acetonitrile (20 mL). Diisopropylethylamine (2.3 g, 17.8 mmol) and 2-(7-azabenzotriazole)-N,N,N',N'-tetramethyluronium hexafluorophosphate (4.07 g, 10.7 mmol) were added. After nitrogen displacement, the reaction flask was placed at 50°C for a reaction. After 2 hours of reaction, the reaction mixture was concentrated, followed by the addition of water (40 mL) and ethyl acetate (6 × 30 mL) for extraction. Organic phases were combined and concentrated under reduced pressure to obtain a crude product, which was purified by column chromatography (petroleum ether : ethyl acetate = 5 : 1) to obtain compound **1c** (as a white solid, 1.2 g, yield 63%).

LCMS m/z = 314.20 [M+1].

### Step 2:

### N-(4-(4-amino-5-bromo-7-methyl-7H-pyrrolo[2,3-d]pyrimidin-6-yl)phenyl)-2-cyclopropylacrylamide 1e

Compound **1c** (550 mg, 1.7 mmol) and 5-bromo-6-iodo-7-methyl-7H-pyrrolo[2,3-d]pyrimidin-4-amine **1d** (470 mg, 1.3 mmol) were dissolved in 1,4-dioxane (5.0 mL). Potassium phosphate (847 mg, 4.0 mol) and 1,1-bis(diphenylphosphino)ferrocene palladium dichloride (123 mg, 18.5 mmol) were added. After nitrogen displacement, the reaction flask was placed in an oil bath at 100°C for a reaction. After 3 hours of reaction, the reaction mixture was quenched with water (40 mL) and extracted with ethyl acetate (3×25 mL). Organic phases were combined and concentrated under reduced pressure to obtain a crude product, which was purified by column chromatography (dichloromethane : methanol = 8 : 1) to obtain compound **1e** (as a yellow solid, 160 mg, yield 29%).

LCMS m/z = 412.34 [M+1].

### Step 3:

### N-(4-(4-amino-5-(3-fluoro-4-((4-methylpyrimidin-2-yl)oxy)phenyl)-7-methyl-7H-pyrrolo[2,3-d]pyrimidin-6-yl)phenyl]-2-cyclopropylacrylamide compound 1

Compound **1e** (160 mg, 0.39 mmol) and 2-(2-fluoro-4-(4,4,5,5-tetramethyl-1,3,2-dioxaborolan-2-yl)phenoxy)-4-methylpyrimidine **2b** (170 mg, 0.52 mmol) were dissolved in N,N-dimethylformamide (3.0 mL). Potassium phosphate (210 mg, 0.99 mmol) and 1,1-bis(diphenylphosphino)ferrocene palladium dichloride (20 mg, 0.03 mmol) were added. After nitrogen displacement, the reaction flask was placed in an oil bath at 90°C for a reaction. After 3 hours, the reaction mixture was diluted with water (40 mL) and extracted with ethyl acetate (3 × 30 mL). The organic phase was washed with a saturated aqueous sodium chloride solution (3 × 30 mL). Organic phases were combined and concentrated under reduced pressure. The crude product was purified by column chromatography (dichloromethane : methanol = 12 : 1) to obtain **compound 1** (as a white solid, 1 mg, yield 2%).

¹H NMR (400 MHz, DMSO-d₆) δ 9.68 (s, 1H), 8.18 (d, J = 5.0 Hz, 1H), 7.91 (s, 1H), 7.86 (s, 2H), 7.50 - 7.42 (m, 2H), 7.08 - 7.00 (m, 3H), 6.92 - 6.87 (m, 2H), 6.81 (dd, J = 8.2, 2.0 Hz, 1H), 5.33 (s, 1H), 4.96 (s, 1H), 3.30 (s, 3H), 2.12 (s, 3H), 1.46 (m, 1H), 0.58 - 0.41 (m, 2H), 0.36 - 0.24 (m, 2H).

LCMS m/z = 536.23 [M+1].

### Example 2

### N-(5-(4-amino-5-(3-fluoro-4-((4-methylpyrimidin-2-yl)oxy)phenyl)-7-methyl-7H-pyrrolo[2,3-d]pyrimidin-6-yl)pyridin-2-yl]-2-cyclopropylacrylamide compound 2

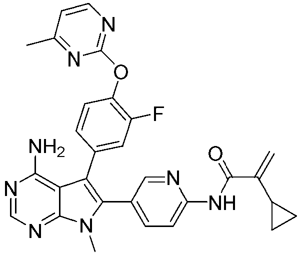

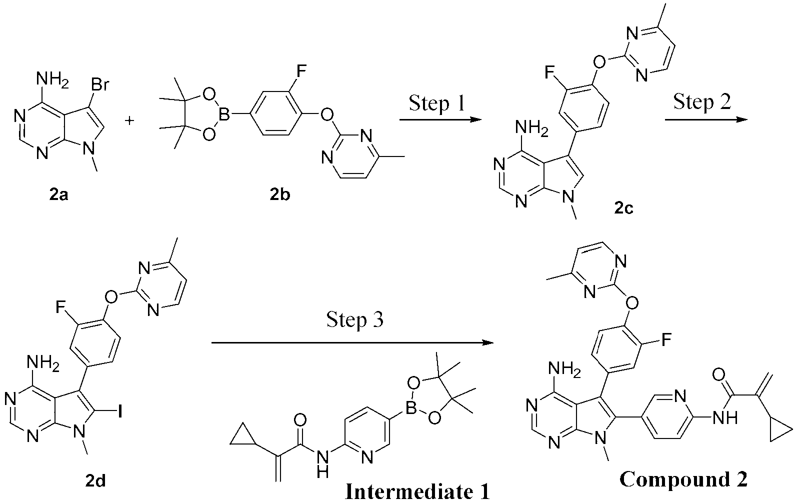

### Step 1

### 5-(3-Fluoro-4-((4-methylpyrimidin-2-yl)oxy)phenyl)-7-methyl-7H-pyrrolo[2,3-d]pyrimidin-4-amine 2c

Compound **2a** (6.55 g, 28.8 mmol) and **2b** (10 g, 30.3 mmol) were dissolved in N,N-dimethylformamide : water = 5 : 1 (168 mL). Potassium phosphate (18.35 g, 86.5 mmol) and 1,1-bis(diphenylphosphino)ferrocene palladium dichloride (2.33 g, 0.3 mmol) were added. After nitrogen displacement, the reaction flask was placed in an oil bath at 90°C for a reaction. After 4 hours of reaction, the reaction mixture was diluted with water (60 mL) and extracted with ethyl acetate (3 × 120 mL). After the extraction was complete, the organic phase was washed with a saturated aqueous sodium chloride solution (3 × 40 mL), dried over anhydrous sodium sulfate, and filtered. Organic phases were combined and concentrated under reduced pressure. The crude product was purified by column chromatography (dichloromethane : methanol = 20 : 1) to obtain compound **2c** (as a light yellow solid, 7.37 g, yield 82%).

LCMS m/z = 351.13 [M+1].

### Step 2

### 5-(3-Fluoro-4-((4-methylpyrimidin-2-yl)oxy)phenyl)-6-iodo-7-methyl-7H-pyrrolo[2,3-d]pyrimidin-4-amine 2d

Compound **2c** (7.37 g, 21 mmol) was dissolved in dichloromethane. Trifluoroacetic acid (4.7 mL, 63.2 mmol) was added. After cooling to 0°C, N-iodosuccinimide (5.7 g, 25.3 mmol) was added. The reaction flask underwent nitrogen displacement. After 3 hours of reaction, the reaction system was quenched with an aqueous sodium sulfite solution and extracted with dichloromethane (3 × 120 mL). The organic phase was washed with a saturated aqueous sodium chloride solution (3 × 40 mL), dried over anhydrous sodium sulfate, and filtered. Organic phases were combined and concentrated under reduced pressure. The crude product was pulped with ethanol : acetonitrile = 1 : 1 (20 mL) at 50°C for 30 minutes and then suction-filtered to obtain compound 2d (as a white solid, 6.5 g, yield 65%).

LCMS m/z = 477.03 [M+1].

### Step 3

### N-(5-(4-amino-5-(3-fluoro-4-((4-methylpyrimidin-2-yl)oxy)phenyl)-7-methyl-7H-pyrrolo[2,3-d]pyrimidin-6-yl)pyridin-2-yl)-2-cyclopropylacrylamide compound 2

Compound **2d** (280 mg, 0.89 mmol) and **intermediate 1** (354 mg, 0.74 mmol) were dissolved in a mixed solution of N,N-dimethylformamide : water = 20 : 1 (4 mL). Potassium phosphate (474 mg, 2.23 mmol) and 1,1-bis(diphenylphosphino)ferrocene palladium dichloride (60 mg, 0.074 mmol) were added. After nitrogen displacement, the reaction flask was placed in an oil bath at 90°C for a reaction. After 2 hours of reaction, the reaction mixture was quenched with water (5 mL) and extracted with ethyl acetate (3 × 15 mL). The organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (ethyl acetate : methanol = 30 : 1) to obtain **compound 2** (as a white solid, 88 mg, yield 14.6%).

¹H NMR (400 MHz, DMSO-d₆) δ 10.34 (s, 1H), 8.51 (s, 1H), 8.47 (d, *J =* 8.0 Hz, 1H), 8.32 (d, *J* = 4.0 Hz, 1H), 8.20 (d, *J* = 8.0 Hz, 1H), 7.92 (dd, *J =* 8.0, 4.0 Hz, 1H), 7.54 (d, *J =* 6.4 Hz, 2H), 7.38 (t, *J* = 8.4 Hz, 1H), 7.31 (dd, *J=* 11.2, 4.0 Hz, 1H), 7.19 (d, *J* = 5.0 Hz, 1H), 7.14 - 7.08 (m, 1H), 5.81 (s, 1H), 5.35 (d, *J= 4.0* Hz, 1H), 3.72 (s, 3H), 2.42 (s, 3H),1.79 (m, 1H), 0.82 - 0.73 (m, 2H), 0.59 - 0.48 (m, 2H).

LCMS m/z = 537.58 [M+1].

### Example 3

### N-(4-(4-amino-5-(3-fluoro-4-((4-methylpyrimidin-2-yl)oxy)phenyl)-7-methyl-7H-pyrrolo[2,3-d]pyrimidin-6-yl)-2-methoxyphenyl)-2-cyclopropylacrylamide compound 3

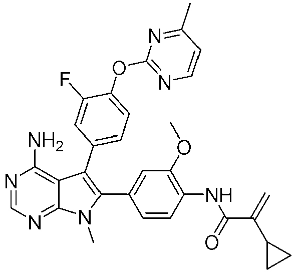

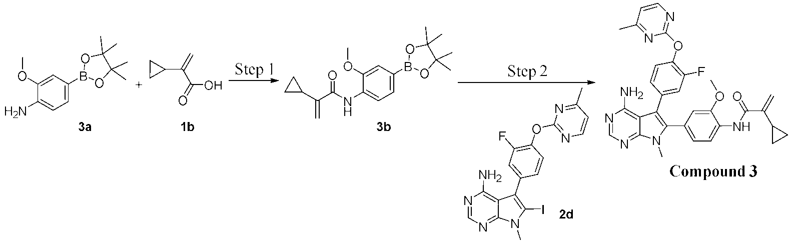

### Step 1

### 2-Cyclopropyl-N-(2-methoxy-4-(4,4,5,5-tetramethyl-1,3,2-dioxaborolan-2-yl)phenyl)acrylamide 3b

2-Cyclopropylacrylic acid **1b** (300 mg, 2.6 mmol) was dissolved in N,N-dimethylformamide (8 mL). 2-(7-Azobenzotriazole)-N,N,N',N'-tetramethyluronium hexafluorophosphate (1.38 g, 3.6 mmol) and triethylamine (614 mg, 6.07 mmol) were added. After the mixture was reacted at room temperature for 30 minutes, compound **3a** (617 mg, 2.2 mmol) was added. After nitrogen displacement in a reaction flask at room temperature for 2 hours, the mixture was diluted with water (8 mL) and extracted with ethyl acetate (3 × 15 mL). The organic phase was washed with a saturated aqueous sodium chloride solution (3 × 15 mL), dried over anhydrous sodium sulfate, and filtered. The organic phases were combined and concentrated under reduced pressure to obtain compound **3b** (as a yellow solid, 290 mg, yield 31.5%).

LCMS m/z = 344.20 [M+1].

### Step 2

### N-(4-(4-amino-5-(3-fluoro-4-((4-methylpyrimidin-2-yl)oxy)phenyl)-7-methyl-7H-pyrrolo[2,3-d]pyrimidin-6-yl)-2-methoxyphenyl)-2-cyclopropylacrylamide compound 3

Compound **2d** (298 mg, 0.62 mmol) and compound **3b** (280 mg, 0.8 mmol) were dissolved in a mixed solution of N,N-dimethylformamide : water = 20 : 1 (4 mL). Potassium phosphate (400 mg, 1.87 mmol) and 1,1-bis(diphenylphosphino)ferrocene palladium dichloride (45 mg, 0.062 mmol) were added. After nitrogen displacement, the reaction flask was placed in an oil bath at 90°C for a reaction. After 3 hours of reaction, the reaction mixture was quenched with water (5 mL) and extracted with ethyl acetate (3 × 15 mL). The organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (ethyl acetate : methanol = 30 : 1) to obtain **compound 3** (as a white solid, 100 mg, yield 28.3%).

¹H NMR (400 MHz, Chloroform-d) δ 8.91 (s, 1H), 8.58 (d, *J =* 8.3 Hz, 1H), 8.38 (s, 1H), 8.34 (d, *J* = 4.0 Hz, 1H), 7.23 (d, *J* = 8.0 Hz, 1H), 7.11 (d, J = 2.3 Hz, 1H), 7.10 - 7.07 (m, 1H), 7.04 (dd, J = 8.4, 1.6 Hz, 1H), 6.94 (d, *J =* 4.0 Hz, 1H), 6.63 (d, *J =* 1.6 Hz, 1H), 6.15 (d, *J =* 1.1 Hz, 1H), 5.40 (d, *J* = 1.2 Hz, 1H), 5.33 (s, 2H), 3.78 (s, 3H), 3.71 (s, 3H), 2.50 (s, 3H), 1.68 - 1.63 (m, 1H), 0.93-0.95 (m, 2H), 0.69 - 0.63 (m, 2H).

LCMS m/z = 566.61 [M+1].

### Example 4

### N-(4-(4-amino-5-(3-fluoro-4-((4-methylpyrimidin-2-yl)oxy)phenyl)-7-methyl-7H-pyrrolo[2,3-d]pyrimidin-6-yl)-3-chlorophenyl)-2-cyclopropylacrylamide compound 4

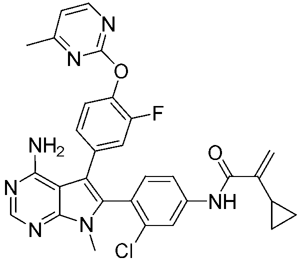

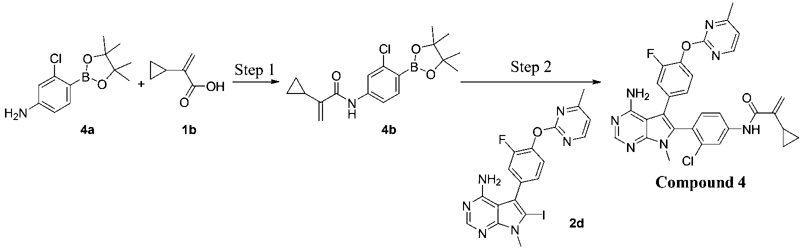

### Step 1

### N-(3-chloro-4-(4,4,5,5-tetramethyl-1,3,2-dioxaborolan-2-yl)phenyl)-2-cyclopropylacrylamide 4b

2-Cyclopropylacrylic acid **1b** (300 mg, 2.6 mmol) was dissolved in N,N-dimethylformamide (8 mL). 2-(7-Azobenzotriazole)-N,N,N',N'-tetramethyluronium hexafluorophosphate (1.38 g, 3.6 mmol) and triethylamine (614 mg, 6.07 mmol) were added. After the mixture was reacted at room temperature for 30 minutes, compound **4a** (616 mg, 2.4 mmol) was added. After nitrogen displacement in a reaction flask at room temperature for 2 hours, the mixture was diluted with water (8 mL) and extracted with ethyl acetate (3 × 15 mL). The organic phase was washed with a saturated aqueous sodium chloride solution (3 × 15 mL), dried over anhydrous sodium sulfate, and filtered. The organic phases were combined and concentrated under reduced pressure to obtain compound **4b** (as a white solid, 200 mg, yield 23.7%).

LCMS m/z = 348.65 [M+1].

### Step 2

### N-(4-(4-amino-5-(3-fluoro-4-((4-methylpyrimidin-2-yl)oxy)phenyl)-7-methyl-7H-pyrrolo[2,3-d]pyrimidin-6-yl)-3-chlorophenyl)-2-cyclopropylacrylamide compound 4

Compound **2d** (211 mg, 0.44mmol) and compound **4b** (200 mg, 0.57 mmol) were dissolved in a mixed solution of N,N-dimethylformamide : water = 20 : 1 (4 mL). Potassium phosphate (280 mg, 1.31 mmol) and 1,1-bis(diphenylphosphino)ferrocene palladium dichloride (32 mg, 0.044 mmol) were added. After nitrogen displacement, the reaction flask was placed in an oil bath at 90°C for a reaction. After 3 hours, the reaction mixture was quenched with water (5 mL) and extracted with ethyl acetate (3 × 15 mL). The organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (ethyl acetate : methanol = 25 : 1) to obtain **compound 4** (as a white solid, 5 mg, yield 1.5%).

¹H NMR (400 MHz, DMSO-d₆) δ 10.21 (s, 1H), 8.50 (s, 1H), 8.46 (d, *J =* 8.4 Hz, 1H), 8.09 (d, *J* = 4.0 Hz, 1H), 7.74 (dd, *J* = 4.0, 8.0 Hz, 1H), 7.45 (d, *J* = 8.4 Hz, 1H), 7.35 (t, *J* = 8.4 Hz, 1H), 7.59 (d, *J* = 6.4 Hz, 2H),7.26 - 7.15 (m, 2H), 7.09 - 7.01 (m, 1H), 5.67 (s, 1H), 5.33 (s, 1H), 3.55 (s, 3H), 2.40 (s, 3H), 1.79 (m, 1H), 0.86 - 0.72 (m, 2H), 0.62 - 0.51 (m, 2H).

LCMS m/z = 570.17 [M+1].

### Example 5

### N-(4-(4-amino-5-(3-fluoro-4-((4-methylpyrimidin-2-yl)oxy)phenyl)-7-methyl-7H-pyrrolo[2,3-d]pyrimidin-6-yl)-3-fluorophenyl)-2-cyclopropylacrylamide compound 5

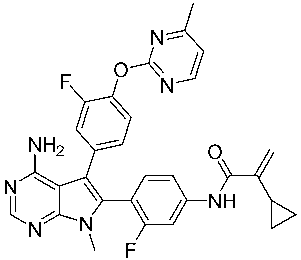

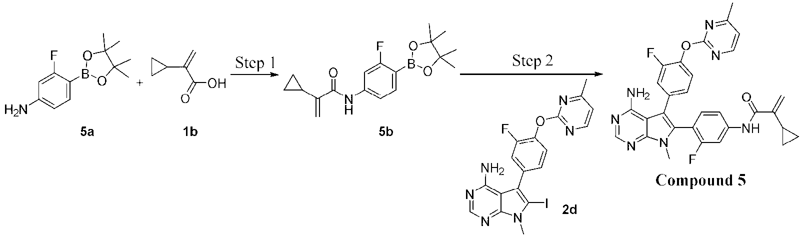

### Step 1

### 2-Cyclopropyl-N-(3-fluoro-4-(4,4,5,5-tetramethyl-1,3,2-dioxaborolan-2-yl)phenyl)acrylamide 5b

2-Cyclopropylacrylic acid **1b** (800 mg, 7.14 mmol) was dissolved in N,N-dimethylformamide (10 mL). 2-(7-Azobenzotriazole)-N,N,N',N'-tetramethyluronium hexafluorophosphate (2.4 g, 6.31 mmol) and triethylamine (2.18 g, 21.05 mmol) were added. After the mixture was reacted at room temperature for 30 minutes, compound **5a** (1 g, 4.21 mmol) was added. After nitrogen displacement in a reaction flask at room temperature for 2 hours, the mixture was diluted with water (8 mL) and extracted with ethyl acetate (3 × 15 mL). The organic phase was washed with a saturated aqueous sodium chloride solution (3 × 15 mL), dried over anhydrous sodium sulfate, and filtered. The organic phases were combined and concentrated under reduced pressure to obtain compound **5b** (as a yellow solid, 1.16 g, yield 83%).

LCMS m/z = 332.19 [M+1].

### Step 2

### N-(4-(4-amino-5-(3-fluoro-4-((4-methylpyrimidin-2-yl)oxy)phenyl)-7-methyl-7H-pyrrolo[2,3-d]pyrimidin-6-yl)-3-fluorophenyl)-2-cyclopropylacrylamide compound 5

Compound **2d** (100 mg, 0.22 mmol) and compound **5b** (104 mg, 0.31 mmol) were dissolved in a mixed solution of N,N-dimethylformamide : water = 20 : 1 (4 mL). Potassium phosphate (133 mg, 0.63 mmol) and 1,1-bis(diphenylphosphino)ferrocene palladium dichloride (15 mg, 0.02 mmol) were added. After nitrogen displacement, the reaction flask was placed in an oil bath at 90°C for a reaction. After 3 hours, the reaction mixture was quenched with water (5 mL) and extracted with ethyl acetate (3 × 15 mL). The organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (ethyl acetate : methanol = 25 : 1) to obtain **compound 5** (as a white solid, 16 mg, yield 13%).

¹H NMR (400 MHz, DMSO-d₆) δ 10.23 (s, 1H), 8.47 (d, *J =* 8.0 Hz, 1H), 8.43 (s, 1H), 7.82 (dd, *J* = 4.0*,* 12.0 Hz, 1H), 7.57 (dd, *J* = 4.0, 12 Hz, 1H), 7.41 - 7.34 (m, 2H), 7.26 (s, 1H), 7.25 - 7.21 (m, 1H), 7.19 (d, *J* = 4.0 Hz, 1H), 7.13 (s, 1H), 7.08 (d, *J* = 8.0 Hz, 1H), 5.65 (s, 1H), 5.33 (s, 1H), 3.60 (s, 3H), 2.41 (s, 3H), 1.79 - 1.72 (m, 1H), 0.81 - 0.77 (m, 2H), 0.58 - 0.54 (m, 2H).

LCMS m/z = 554.20 [M+1].

### Example 6

### N-(4-(4-amino-5-(4-((5-chloropyridin-2-yl)oxy)-3-fluorophenyl)-7-methyl-7H-pyrrolo[2,3-d]pyrimidin-6-yl)phenyl)-2-cyclopropylacrylamide compound 6

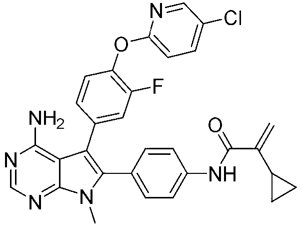

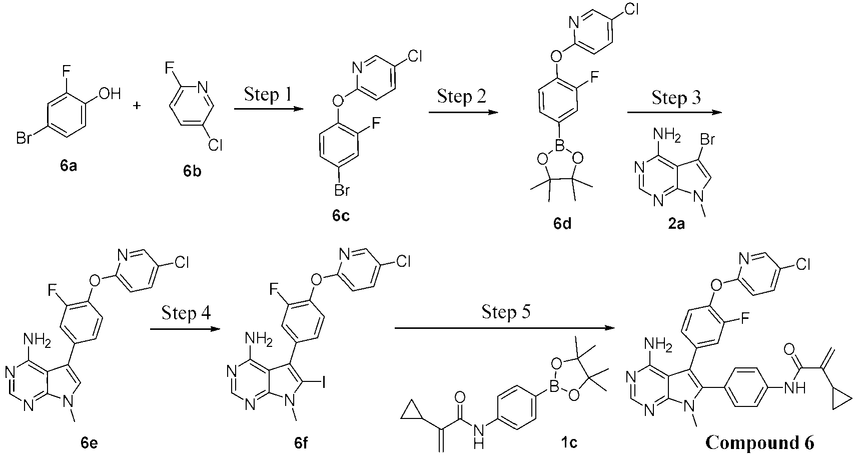

### Step 1

### 2-(4-Bromo-2-fluorophenoxy)-5-chloropyridine 6c

4-Bromo-2-fluorophenol compound **6a** (3.48 g, 18.24 mmol) was dissolved in dimethylsulfoxide (20 mL). 5-Chloro-2-fluoropyridine compound **6b** (2.00 g, 15.2 mmol) was added, followed by sodium hydroxide (0.91 g, 22.8 mmol). After nitrogen displacement, the reaction flask was placed in an oil bath at 110°C for 7 hours of reaction. After the reaction was complete, the reaction mixture was diluted with water (20 mL) and extracted with ethyl acetate (3 × 40 mL). The organic phase was washed with a saturated aqueous sodium chloride solution (3 × 40 mL), dried over anhydrous sodium sulfate, and filtered. The organic phase was concentrated under reduced pressure. The crude product was purified by column chromatography (petroleum ether : ethyl acetate = 20 : 1) to obtain compound **6c** (as a light yellow solid, 2.00 g, yield 37.03%).

LCMS m/z = 303.99 [M+1].

### Step 2

### 5-Chloro-2-(2-fluoro-4-(4,4,5,5-tetramethyl-1,3,2-dioxaborolan-2-yl)phenoxy)pyridine 6d

Compound **6c** (2.000 g, 6.65 mmol) was dissolved in 1,4-dioxane (30 mL). Bis(pinacolato)diboron (2.530 g, 9.97 mmol) was added, followed by potassium acetate (1.950 g, 19.95 mmol) and 1,1-bis(diphenylphosphino)ferrocene palladium dichloride (0.486 g, 0.665 mmol). After the reaction flask underwent nitrogen displacement, a reaction was carried out for 2 hours. After the reaction was complete, the reaction mixture was diluted with water (30 mL) and extracted with ethyl acetate (50 mL × 3). The organic phase was washed with a saturated aqueous sodium chloride solution (50 mL × 3), dried over anhydrous sodium sulfate, and filtered. The organic phase was concentrated under reduced pressure to obtain a crude product. The crude product was purified by column chromatography (petroleum ether : ethyl acetate = 20 : 1) to obtain compound **6d** (as a white solid, 2 g, yield 86.1%).

LCMS m/z = 350.11 [M+1].

### Step 3

### 5-(4-((5-Chloropyridin-2-yl)oxy)-3-fluorophenyl)-7-methyl-7H-pyrrolo[2,3-d]pyrimidin-4-amine 6e

5-Bromo-7-methyl-4-aminopyrrolo[2,3-d]pyrimidine **2a** (591 mg, 2.6 mmol) and compound **6d** (1.000 g, 2.86 mmol) were dissolved in a mixed solution of N,N-dimethylformamide : water = 10 : 1 (22 mL). Potassium phosphate (1.650 g, 7.80 mmol) and 1,1-bis(diphenylphosphino)ferrocene palladium dichloride (210 mg, 0.26 mmol) were added. After nitrogen displacement, the reaction flask was placed in an oil bath at 90°C for 4 hours of reaction. After the reaction was complete, the reaction mixture was diluted with water (20 mL) and extracted with ethyl acetate (30 mL × 3). The organic phase was washed with a saturated aqueous sodium chloride solution (40 mL × 3), dried over anhydrous sodium sulfate, and filtered. Organic phases were combined and concentrated under reduced pressure. The crude product was purified by column chromatography (dichloromethane : methanol = 20 : 1) to obtain compound **6e** (as a light yellow solid, 744 mg, yield 77%).

LCMS m/z = 370.10 [M+1].

### Step 4

### 5-(4-((5-Chloropyridin-2-yl)oxy)-3-fluorophenyl)-6-iodo-7-methyl-7H-pyrrolo[2,3-d]pyrimidin-4-amine compound 6f

Compound **6e** (500 mg, 1.35 mmol) was dissolved in dichloromethane (10 mL). Trifluoroacetic acid (0.3 mL, 4.06 mmol) was added. The system was cooled to 0°C. N-iodosuccinimide (457 mg, 2.03 mmol) was added. After the reaction flask underwent nitrogen displacement, a reaction was carried out for 3 hours. After the reaction was complete, the reaction system was quenched with a sodium sulfite solution. The mixture was extracted with dichloromethane (20 mL × 3). The organic phase was washed with a saturated aqueous sodium chloride solution (20 mL × 3), dried over anhydrous sodium sulfate, and filtered. Organic phases were combined and concentrated under reduced pressure. The crude product was pulped with ethanol : acetonitrile = 1 : 1 (10 mL) at 50°C for 30 minutes and then suction-filtered to obtain compound **6f** (as a brown solid, 233 mg, yield 35%).

LCMS m/z = 495.98 [M+1].

### Step 5

### N-(4-(4-amino-5-(4-((5-chloropyridin-2-yl)oxy)-3-fluorophenyl)-7-methyl-7H-pyrrolo[2,3-d]pyrimidin-6-yl)phenyl)-2-cyclopropylacrylamide compound 6

Compound **6f** (233 mg, 0.47 mmol) and compound **1c** (175 mg, 0.56 mmol) were dissolved in a mixed solution of N,N-dimethylformamide : water = 20 : 1 (6 mL). Potassium phosphate (298 mg, 1.41 mmol) and 1,1-bis(diphenylphosphino)ferrocene palladium dichloride (34 mg, 0.047 mmol) were added. After nitrogen displacement, the reaction flask was placed in an oil bath at 90°C for 2 hours of reaction. After the reaction was complete, the reaction mixture was diluted with water (6 mL) and extracted with ethyl acetate (10 mL × 3). The organic phase was washed with a saturated aqueous sodium chloride solution (10mL × 3), dried over anhydrous sodium sulfate, and filtered. Organic phases were combined and concentrated under reduced pressure. The crude product was purified by column chromatography (ethyl acetate : methanol = 30 : 1) to obtain **compound 6** (as a white solid, 70 mg, yield 26.8%).

¹HNMR (400 MHz, DMSO-d₆) δ 9.97 (s, 1H), 8.22 (d, *J=* 4.0 Hz, 1H), 8.20 (s, 1 H), 7.99 (dd, *J* = 12.0 Hz, 4.0 Hz, 1H), 7.75 (d, *J*=8.0 Hz, 2H), 7.34-7.29 (m, 3 H), 7.21-7.16 (m, 2 H), 7.10-7.08 (m, 1 H), 5.99 (s, 2H), 5.63 (s, 1H), 5.28 (s, 1H), 3.58 (s, 3H), 1.73 (m, 1 H), 0.81-0.76 (m, 2H), 0.58-0.54(m, 2H).

LCMS m/s = 555.16 [M+1].

### Example 7

### 4-(4-Amino-6-(4-(2-cyclopropylacrylamido)phenyl)-7-methyl-7H-pyrrolo[2,3-d]pyrimidin-5-yl)-2-fluorophenyl pyrrolidine-1-carboxylate compound 7

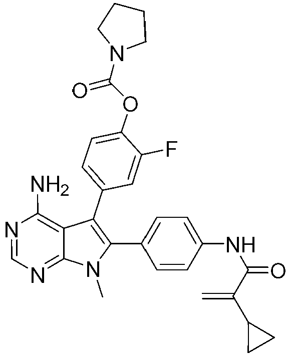

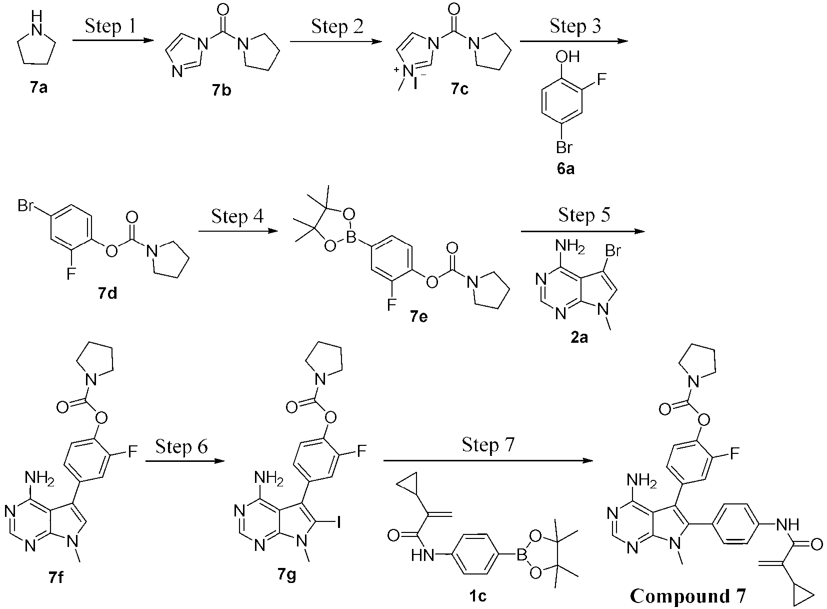

### Step 1

### (1H-imidazol-1-yl)(pyrrolidin-1-yl)methanone 7b

Compound **7a** (3.00 g, 42.2 mmol) and N,N'-carbonyldiimidazole (7.54 g, 46.5 mmol) were dissolved in tetrahydrofuran (80 mL). The mixture was reacted at 75°C under reflux for 24 hours. After the reaction was complete, the reaction solution was subjected to rotary evaporation to dryness. The crude product was dissolved with dichloromethane and washed with water (50 mL × 3). The organic phase was dried over anhydrous sodium sulfate and filtered. Organic phases were combined and concentrated under reduced pressure to obtain compound **7b** (as a yellow liquid, 3.60 g, yield 52%).

LCMS m/z = 166.10 [M+1].

### Step 2

### 3-Methyl-1-(pyrrolidine-1-carbonyl)-1H-imidazol-3-ium 7c

Compound **7b** (3.600 g, 21.8 mmol) was dissolved in acetonitrile (40 mL). Iodomethane (5.5 mL, 87.2 mmol) was slowly dropwise added to the system. The mixture was reacted at room temperature for 24 hours. After the reaction was complete, the reaction solution was concentrated to obtain compound **7c** (as a yellow liquid, 7.466 g, yield 99%).

LCMS m/z = 181.10 [M+1].

### Step 3

### 4-Bromo-2-fluorophenyl pyrrolidine-1-carboxylate 7d

Compound **7c** (7.466 g, 24.32 mmol) was dissolved in dichloromethane (80 mL). Compound **6a** (4.670 g, 24.32 mmol) and triethylamine (3.5 mL, 3.48 mmol) were added. The mixture was reacted at room temperature for 3 hours. After the reaction was complete, the reaction solution was concentrated. The crude product was purified by column chromatography (petroleum ether : ethyl acetate = 10 : 1) to obtain compound **7d** (as a light yellow liquid, 5.637 g, yield 81%).

LCMS m/z = 288.00 [M+1].

### Step 4

### 2-Fluoro-4-(4,4,5,5-tetramethyl-1,3,2-dioxaborolan-2-yl)phenyl pyrrolidine-1-carboxylate 7e

Compound **7d** (5.637 g, 19.64 mmol), bis(pinacolato)diboron (7.500 g, 29.46 mmol), and potassium acetate (5.800 g, 58.92 mmol) were dissolved in 1,4-dioxane (50 mL). 1,1-Bis(diphenylphosphino)ferrocene palladium dichloride (1.440 g, 1.96 mmol) was then added. After nitrogen displacement, the reaction flask was placed in an oil bath at 90°C for 3 hours of reaction. After the reaction was complete, the reaction mixture was quenched with water (100 mL) and extracted with ethyl acetate (80 mL × 3). The organic phase was washed with a saturated aqueous sodium chloride solution (50 mL × 3), dried over anhydrous sodium sulfate, and filtered. Organic phases were combined and concentrated under reduced pressure. The crude product was purified by column chromatography (petroleum ether : ethyl acetate = 10 : 1) to obtain compound **7e** (as a white solid, 6.672 g, yield 91%).

LCMS m/z = 336.17 [M+1].

### Step 5

### 4-(4-Amino-7-methyl-7H-pyrrolo[2,3-d]pyrimidin-5-yl)-2-fluorophenyl pyrrolidine-1-carboxylate 7f

Compound **7e** (2.000 g, 5.97 mmol), compound **2a** (1.35 g, 5.97 mmol), and potassium phosphate (3.800 g, 18.01 mmol) were dissolved in N,N-dimethylformamide : water = 20 : 1 (21 mL). 1,1-Bis(diphenylphosphino)ferrocene palladium dichloride (440 mg, 0.61 mmol) was added. After nitrogen displacement, the reaction flask was placed in an oil bath at 90°C for 3 hours of reaction. After the reaction was complete, the reaction mixture was diluted with water (20 mL) and extracted with ethyl acetate (30 mL × 3). The organic phase was washed with a saturated aqueous sodium chloride solution (30 mL × 3), dried over anhydrous sodium sulfate, and filtered. Organic phases were combined and concentrated under reduced pressure. The crude product was purified by column chromatography (ethyl acetate : methanol = 50 : 1) to obtain compound **7f** (as a yellow solid, 1.458 g, yield 69%).

LCMS m/z = 356.14 [M+1].

### Step 6

### 4-(4-Amino-6-iodo-7-methyl-7H-pyrrolo[2,3-d]pyrimidin-5-yl)-2-fluorophenyl pyrrolidine-1-carboxylate 7g

Compound **7f** (1.458 g, 4.13 mmol) was dissolved in dichloromethane (20 mL). Trifluoroacetic acid (1.405 g, 12.35 mmol) was added to the mixed solution. After the system was cooled to 0°C, N-iodosuccinimide (1.391 g, 4.18 mmol) was slowly added. The system was heated to room temperature and reacted for 2 hours. After the reaction was complete, the reaction mixture was quenched with a saturated solution (20 mL). The system was extracted with dichloromethane (30 mL × 3). The organic phase was washed with a saturated aqueous sodium chloride solution (30 mL × 3), dried over anhydrous sodium sulfate, and filtered. Organic phases were combined and concentrated under reduced pressure. The crude product was purified by column chromatography (ethyl acetate : methanol = 30 : 1) to obtain compound **7g** (as a white solid, 1.320 g, yield 67%).

LCMS m/z = 482.10 [M+1].

### Step 7

### 4-(4-Amino-6-(4-(2-cyclopropylacrylamido)phenyl)-7-methyl-7H-pyrrolo[2,3-d]pyrimidin-5-yl)-2-fluorophenyl pyrrolidine-1-carboxylate compound 7

Compound **7g** (200 mg, 0.416 mmol) and compound **1c** (130 mg, 0.416 mmol) were dissolved in N,N-dimethylformamide : water = 20 : 1 (5.5 mL). Potassium phosphate (265 mg, 1.250 mmol) and 1,1-bis(diphenylphosphino)ferrocene palladium dichloride (31 mg, 0.044 mmol) were added. After nitrogen displacement, the reaction flask was placed in an oil bath at 90°C for 3 hours of reaction. After the reaction was complete, the reaction mixture was quenched with water (10 mL) and extracted with ethyl acetate (15 mL × 3). The organic phase was washed with a saturated aqueous sodium chloride solution (15 mL × 3), dried over anhydrous sodium sulfate, and filtered. Organic phases were combined and concentrated under reduced pressure. The crude product was purified by column chromatography (dichloromethane : methanol = 10 : 1) to obtain **compound 7** (as a white solid, 85 mg, yield 38%).

¹H NMR (400 MHz, DMSO-d₆) δ 10.00 (s, 1H), 8.50 (s, 1H), 7.76 (d, *J* = 8.8 Hz, 2H), 7.55 (s, 2H), 7.33 - 7.29 (m, 2H), 7.26 (t, *J* = 8.4 Hz, 1H), 7.19 (dd, *J* = 12.0, 2.4 Hz, 1H), 7.02 (dd, *J* = 8.4, 2.0 Hz, 1H), 5.64 (s, 1H), 5.28 (s, 1H), 3.66 (s, 3H), 3.49 (t, *J* = 4.0 Hz, 2H), 3.33 (t, *J* = 6.4 Hz, 2H), 1.97 - 1.81 (m, 4H), 1.75 (m, 1H), 0.82 - 0.74 (m, 2H), 0.59 - 0.52 (m, 2H).

LCMS m/z = 541.23 [M+1].

### Example 8

### 4-(4-Amino-6-(4-(2-cyclopropylacrylamido)phenyl)-7-methyl-7H-pyrrolo[2,3-d]pyrimidin-5-yl)-2-fluorophenyl-5-azaspiro[2.4]heptane-5-carboxylate compound 8

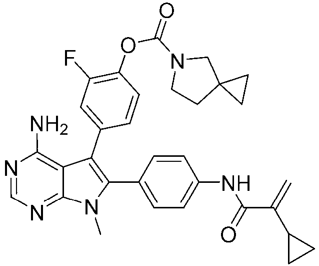

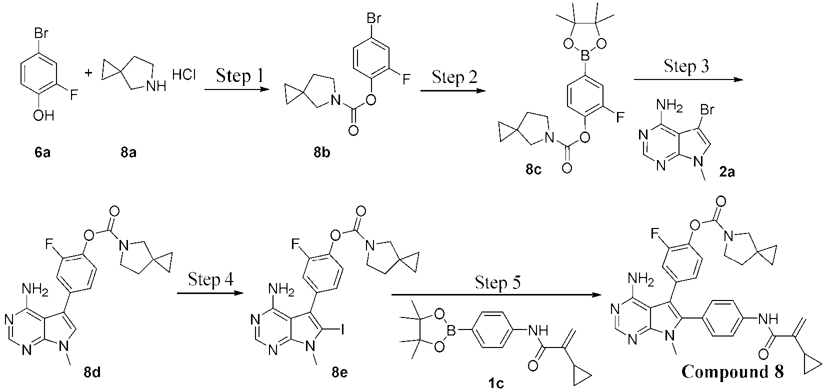

### Step 1

### 4-Bromo-2-fluorophenyl-5-azaspiro[2.4]heptane-5-carboxylate 8b

Triphosgene (2.64 g, 8.90 mmol) was dissolved in dichloromethane (10 mL). At 0°C, a solution of 4-bromo-2-fluorophenol **6a** (5.00 g, 26.17 mmol) and N,N-diisopropylethylamine (3.38 g, 26.17 mmol) in dichloromethane (20 mL) was slowly dropwise added to the system. The system was heated to room temperature and reacted for 2 hours. The system was cooled to 0°C. A solution of 5-azaspiro[2.4]heptane **8a** (3.67 g, 26.17 mmol) and N,N-diisopropylethylamine (7.10 g, 54.86 mmol) in dichloromethane (20 mL) was slowly dropwise added to the system. The system was heated to room temperature and reacted for 2 hours. After the reaction was complete, the reaction solution was concentrated. The crude product was purified by column chromatography (petroleum ether : ethyl acetate = 8 : 1) to obtain compound **8b** (as a light yellow liquid, 5.70 g, yield 69%).

LCMS m/z = 314.16 [M+1].

### Step 2

### 2-Fluoro-4-(4,4,5,5-tetramethyl-1,3,2-dioxaborolan-2-yl)phenyl-5-azaspiro[2.4]heptane-5-carboxylate 8c

### 4-Bromo-2-fluorophenyl-5-azaspiro[2.4]heptane-5-carboxylate 8b (3.000 g, 9.54 mmol) and bis(pinacolato)diboron (3.640 g, 14.32 mmol) were dissolved in 1,4-dioxane (30 mL). Potassium acetate (2.820 g, 28.65 mmol) and 1,1-bis(diphenylphosphino)ferrocene palladium dichloride (700 mg, 0.95 mmol) were added. After nitrogen displacement, the reaction flask was placed in an oil bath at 95°C for 5 hours of reaction. After the reaction was complete, the reaction solution was concentrated. The crude product was purified by column chromatography (petroleum ether : ethyl acetate = 8 : 1) to obtain compound 8c (as a white solid, 3.00 g, yield 87%).

LCMS m/z = 362.20 [M+1].

### Step 3

### 4-(4-Amino-7-methyl-7H-pyrrolo[2,3-d]pyrimidin-5-yl)-2-fluorophenyl 5-azaspiro[2.4]heptane-5-carboxylate 8d

5-Bromo-7-methyl-4-aminopyrrolo[2,3-d]pyrimidine **2a** (571 mg, 2.52 mmol) and 2-fluoro-4-(4,4,5,5-tetramethyl-1,3,2-dioxaborolan-2-yl)phenyl-5-azaspiro[2.4]heptane-5-carboxylate **8c** (1.000 g, 2.77 mmol) were dissolved in N,N-dimethylformamide : water= 5 : 1 (10 mL). Potassium phosphate (1.600 g, 7.56 mmol) and 1,1-bis(diphenylphosphino)ferrocene palladium dichloride (185 mg, 0.25 mmol) were added. After nitrogen displacement, the reaction flask was placed in an oil bath at 90°C for 4 hours of reaction. After the reaction was complete, the reaction mixture was diluted with water (10 mL) and extracted with ethyl acetate (10 mL × 3). The organic phase was washed with a saturated aqueous sodium chloride solution (10 mL × 3), dried over anhydrous sodium sulfate, and filtered. Organic phases were combined and concentrated under reduced pressure. The crude product was purified by column chromatography (dichloromethane : methanol = 30 : 1) to obtain compound **8d** (as a yellow solid, 550 mg, yield 57%).

LCMS m/z = 382.10 [M+1].

### Step 4

### 4-(4-Amino-6-iodo-7-methyl-7H-pyrrolo[2,3-d]pyrimidin-5-yl)-2-fluorophenyl 5-azaspiro[2.4]heptane-5-carboxylate 8e

Compound **8d** (550 mg, 1.44 mmol) was dissolved in dichloromethane (5 mL). Trifluoroacetic acid (493 mg, 4.32 mmol) was added. After the system was cooled to 0°C, N-iodosuccinimide (486 mg, 2.16 mmol) was added. The reaction flask underwent nitrogen displacement for 3 hours of reaction. After the reaction was complete, the system was quenched with an aqueous sodium sulfite solution and extracted with dichloromethane (5 × 3 mL). The organic phase was washed with a saturated aqueous sodium chloride solution (5 mL × 3), dried over anhydrous sodium sulfate, and filtered. Organic phases were combined and concentrated under reduced pressure. The crude product was purified by column chromatography (dichloromethane : methanol = 40 : 1) to obtain compound **8e** (as a brown solid, 292 mg, yield 40%).

LCMS m/z = 508.10 [M+1].

### Step 5

### 4-(4-Amino-6-(4-(2-cyclopropylacrylamido)phenyl)-7-methyl-7H-pyrrolo[2,3-d]pyrimidin-5-yl)-2-fluorophenyl-5-azaspiro[2.4]heptane-5-carboxylate compound 8

Compound **8e** (200 mg, 0.39 mmol) and compound **1c** (160 mg, 0.51 mmol) were dissolved in N,N-dimethylformamide : water = 20 : 1 (4 mL). Potassium phosphate (250 mg, 1.18 mmol) and 1,1-bis(diphenylphosphino)ferrocene palladium dichloride (30 mg, 0.04 mmol) were added. After nitrogen displacement, the reaction flask was placed in an oil bath at 90°C for 2 hours of reaction. After the reaction was complete, the reaction mixture was quenched with water (5 mL) and extracted with ethyl acetate (10 mL × 3). The organic phase was washed with a saturated aqueous sodium chloride solution (10 mL × 3), dried over anhydrous sodium sulfate, and filtered. Organic phases were combined and concentrated under reduced pressure. The crude product was purified by column chromatography (dichloromethane : methanol = 30 : 1) to obtain **compound 8** (as a white solid, 55 mg, yield 24.8%).

¹H NMR (400 MHz, DMSO-d₆) δ 9.95 (s, 1H), 8.20 (s, 1H), 7.76 - 7.69 (m, 2H), 7.32 - 7.21 (m, 3H), 7.13 (m, 1H), 7.04 (dd, *J =* 8.4, 2.0 Hz, 1H), 5.95 (s, 2H), 5.63 (s, 1H), 5.27 (d, *J =* 2.0 Hz, 1H), 3.66 (t, *J* = 6.4 Hz, 1H), 3.59 (s, 3H), 3.50 (t, *J* = 6.4 Hz, 1H), 3.42 (s, 1H), 3.26 (s, 1H), 1.90 - 1.70 (m, 3H), 0.83 - 0.72 (m, 2H), 0.68 - 0.52 (m, 6H).

LCMS m/z = 567.30 [M+1].

### Example 9

### N-(4-(4-amino-5-(4-((6-chloropyridin-2-yl)oxy)-3-fluorophenyl)-7-methyl-7H-pyrrolo[2,3-d]pyrimidin-6-yl)phenyl)-2-cyclopropylacrylamide compound 9

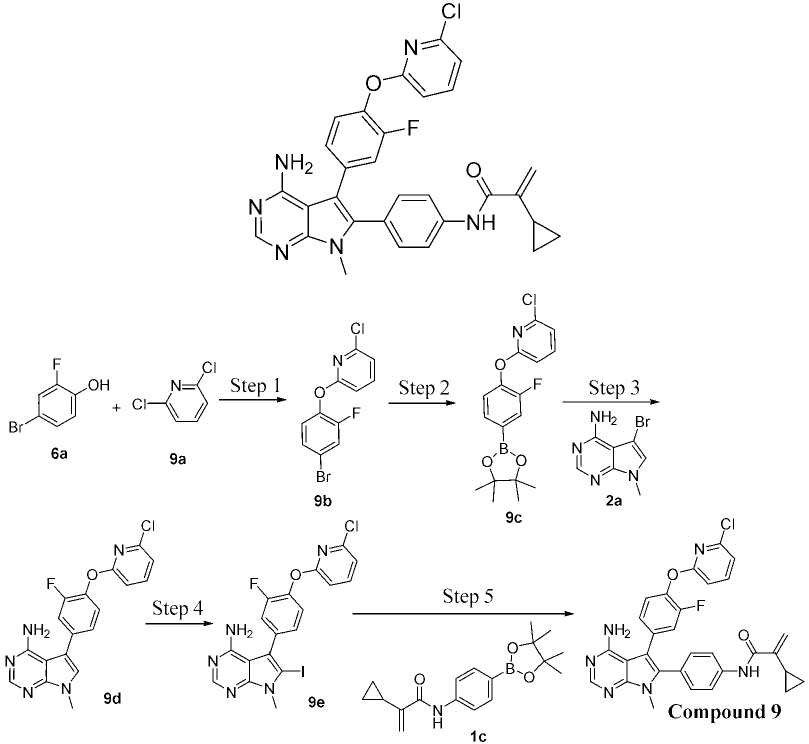

### Step 1

### 2-(4-Bromo-2-fluorophenoxy)-6-chloropyridine 9b

4-Bromo-2-fluorophenol **6a** (4.58 g, 24 mmol) was dissolved in dimethylsulfoxide (20 mL). 2,6-Dichloropyridine compound **9a** (3.00 g, 20 mmol) and sodium hydroxide (1.20 g, 30 mmol) were added. After nitrogen displacement, the reaction flask was placed in an oil bath at 110°C for 7 hours of reaction. After the reaction was complete, the reaction mixture was diluted with water (20 mL) and extracted with ethyl acetate (40 mL × 3). The organic phase was washed with a saturated aqueous sodium chloride solution (40 mL × 3), dried over anhydrous sodium sulfate, and filtered. Organic phases were combined and concentrated under reduced pressure. The crude product was purified by column chromatography (petroleum ether : ethyl acetate = 20 : 1) to obtain compound **9b** (as a light yellow solid, 1.10 g, yield 15.2%).

LCMS m/z = 303.93 [M+1].

### Step 2

### 2-Chloro-6-(2-fluoro-4-(4,4,5,5-tetramethyl-1,3,2-dioxaborolan-2-yl)phenoxy)pyridine 9c

Compound **9b** (1.100 g, 3.65 mmol) was dissolved in 1,4-dioxane (18 mL). Bis(pinacolato)diboron (1.390 g, 5.48 mmol) was added, followed by potassium acetate (1.073 g, 10.95 mmol) and 1,1-bis(diphenylphosphino)ferrocene palladium dichloride (267 mg, 0.36 mmol). After nitrogen displacement, the reaction flask was placed in an oil bath at 90°C for 2 hours of reaction. After the reaction was complete, the reaction mixture was diluted with water (20 mL) and extracted with ethyl acetate (40 mL × 3). The organic phase was washed with a saturated aqueous sodium chloride solution (40 mL × 3), dried over anhydrous sodium sulfate, and filtered. Organic phases were combined and concentrated under reduced pressure. The crude product was purified by column chromatography (petroleum ether : ethyl acetate = 20 : 1) to obtain compound **9c** (as a white solid, 1 g, yield 78.7%).

LCMS m/z = 350.11 [M+1].

### Step 3

### 5-(4-((6-Chloropyridin-2-yl)oxy)-3-fluorophenyl)-7-methyl-7H-pyrrolo[2,3-d]pyrimidin-4-amine 9d

5-Bromo-7-methyl-4-aminopyrrolo[2,3-d]pyrimidine compound **2a** (591 mg, 2.60 mmol) and compound **9c** (1 g, 2.86 mmol) were dissolved in N,N-dimethylformamide : water = 10 : 1 (22 mL). Potassium phosphate (1.650 g, 7.81 mmol) and 1,1-bis(diphenylphosphino)ferrocene palladium dichloride (210 mg, 0.26 mmol) were added. After nitrogen displacement, the reaction flask was placed in an oil bath at 90°C for 4 hours of reaction. After the reaction was complete, the reaction mixture was diluted with water (20 mL) and extracted with ethyl acetate (30 mL × 3). The organic phase was washed with a saturated aqueous sodium chloride solution (40 mL × 3), dried over anhydrous sodium sulfate, and filtered. Organic phases were combined and concentrated under reduced pressure. The crude product was purified by column chromatography (dichloromethane : methanol = 20 : 1) to obtain compound **9d** (as a light yellow solid, 502 mg, yield 57%).

LCMS m/z = 370.08 [M+1].

### Step 4

### 5-(4-((6-Chloropyridin-2-yl)oxy)-3-fluorophenyl)-6-iodo-7-methyl-7H-pyrrolo[2,3-d]pyrimidin-4-amine 9e

Compound **9d** (502 mg, 1.35 mmol) was dissolved in dichloromethane (10 mL). Trifluoroacetic acid (0.3 mL, 4.06 mmol) was added. After the system was cooled to 0°C, N-iodosuccinimide (457 mg, 2.03 mmol) was added. The reaction flask underwent nitrogen displacement, and a reaction was carried out for 3 hours. After the reaction was complete, the system was quenched with an aqueous sodium sulfite solution and extracted with dichloromethane (20 mL × 3). The organic phase was washed with a saturated aqueous sodium chloride solution (20 mL × 3), dried over anhydrous sodium sulfate, and filtered. Organic phases were combined and concentrated under reduced pressure. The crude product was pulped with ethanol : acetonitrile = 1 : 1 (10 mL) at 50°C for 30 minutes and then suction-filtered to obtain compound **9e** (as a brown solid, 300 mg, yield 45%).

LCMS m/z = 495.98 [M+1].

### Step 5

### N-(4-(4-amino-5-(4-((6-chloropyridin-2-yl)oxy)-3-fluorophenyl)-7-methyl-7H-pyrrolo[2,3-d]pyrimidin-6-yl)phenyl)-2-cyclopropylacrylamide compound 9

Compound **9e** (300 mg, 0.61 mmol) and compound **1c** (228 mg, 0.73 mmol) were dissolved in N,N-dimethylformamide : water = 20 : 1 (6 mL). Potassium phosphate (388 mg, 1.83 mmol) and 1,1-bis(diphenylphosphino)ferrocene palladium dichloride (45 mg, 0.05 mmol) were added. After nitrogen displacement, the reaction flask was placed in an oil bath at 90°C for 2 hours of reaction. After the reaction was complete, the reaction mixture was quenched with water (10 mL) and extracted with ethyl acetate (10 mL × 3). The organic phase was washed with a saturated aqueous sodium chloride solution (10 mL × 3), dried over anhydrous sodium sulfate, and filtered. Organic phases were combined and concentrated under reduced pressure. The crude product was purified by column chromatography (ethyl acetate : methanol = 30 : 1) to obtain **compound 9** (as a white solid, 11 mg, yield 3.28%).

¹H NMR (400 MHz, DMSO-d₆) δ 9.95 (s, 1H), 8.21 (s, 1H), 7.93 (s, 1H), 7.76 - 7.74 (m, 2H), 7.36 - 7.10 (m, 7H), 6.04 (s, 1H), 5.63 (s, 1H), 5.28 (s, 1H), 3.61 (s, 3H), 1.76 (s, 1 H), 1.24 (s, 1H), 0.88 (s, 2H), 0.63 (s, 2H).

LCMS m/z = 555.30 [M+1].

### Example 10

### N-(4-(4-amino-5-(3-cyano-4-((4-methylpyrimidin-2-yl)oxy)phenyl)-7-methyl-7H-pyrrolo[2,3-d]pyrimidin-6-yl)phenyl]-2-cyclopropylacrylamide compound 10

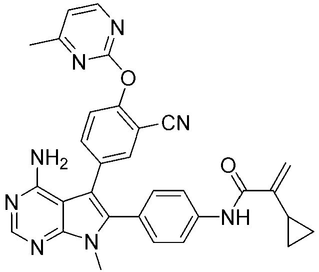

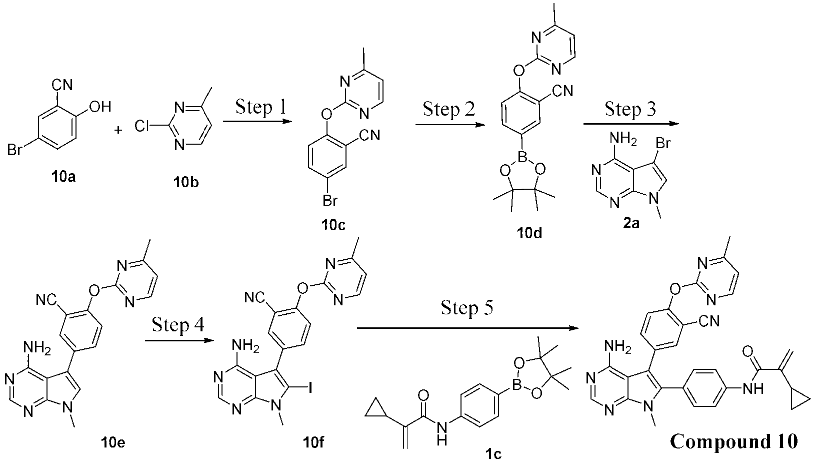

### Step 1

### 5-Bromo-2-((4-methylpyrimidin-2-yl)oxy)benzonitrile 10c

5-Bromo-2-hydroxybenzonitrile **10a** (4.80 g, 24.24 mmol), 2-chloro-4-methylpyrimidine **10b** (3.1 g, 24.24 mmol), and potassium hydroxide (1.36 g, 24.24 mmol) were added to dimethyl sulfoxide (40 mL). The mixture was heated in an oil bath at 150°C and reacted for 4 hours. After the reaction was complete, the system was cooled to room temperature, diluted with water (40 mL), and extracted with ethyl acetate (20 mL × 3). The organic layer was washed with brine (40 mL), dried over anhydrous sodium sulfate, and filtered. The organic phase was concentrated. The crude product was purified by column chromatography (petroleum ether : ethyl acetate = 5 : 1) to obtain compound **10c** (as a yellow solid, 4.76 g, yield 67%).

LCMS m/z = 290.10 [M+1].

### Step 2

### 2-((4-Methylpyrimidin-2-yl)oxy)-5-(4,4,5,5-tetramethyl-1,3,2-dioxaborolan-2-yl)benzonitrile 10d

5-Bromo-2-((4-methylpyrimidin-2-yl)oxy)benzonitrile **10c** (2.810 g, 9.70 mmol) and bis(pinacolato)diboron (3.700 g, 14.57 mmol) were dissolved in 1,4-dioxane (40 mL). Potassium acetate (2.860 g, 29.14 mmol) and 1,1-bis(diphenylphosphino)ferrocene palladium dichloride (711 mg, 0.97 mmol) were added. After nitrogen displacement, the reaction flask was placed in an oil bath at 95°C for 5 hours of reaction. After the reaction was complete, the reaction solution was concentrated. The crude product was purified by column chromatography (petroleum ether : ethyl acetate = 5 : 1) to obtain compound **10d** (as a yellow solid, 2.600 g, yield 80%).

LCMS m/z = 338.10 [M+1].

### Step 3

### 5-(4-Amino-7-methyl-7H-pyrrolo[2,3-d]pyrimidin-5-yl)-2-((4-methylpyrimidin-2-yl)oxy)benzonitrile 10e

5-Bromo-7-methyl-4-aminopyrrolo[2,3-d]pyrimidine compound **2a** (1.520 g, 6.72 mmol) and compound **10d** (2.600 g, 7.71 mmol) were dissolved in N,N-dimethylformamide : water = 5 : 1 (20 mL). Potassium phosphate (4.300 g, 20.26 mmol) and 1,1-bis(diphenylphosphino)ferrocene palladium dichloride (500 mg, 0.67 mmol) were added. After nitrogen displacement, the reaction flask was placed in an oil bath at 90°C for 4 hours of reaction. After the reaction was complete, the reaction mixture was diluted with water (20 mL) and extracted with ethyl acetate (20 mL × 3). The organic phase was washed with a saturated aqueous sodium chloride solution (20 mL × 3), dried over anhydrous sodium sulfate, and filtered. Organic phases were combined and concentrated under reduced pressure. The crude product was purified by column chromatography (dichloromethane : methanol = 30 : 1) to obtain compound **10e** (as a yellow solid, 720 mg, yield 30%).

LCMS m/z = 358.10 [M+1].

### Step 4

### 5-(4-Amino-6-iodo-7-methyl-7H-pyrrolo[2,3-d]pyrimidin-5-yl)-2-((4-methylpyrimidin-2-yl)oxy)benzonitrile 10f

Compound **10e** (570 mg, 1.59 mmol) was dissolved in dichloromethane (5 mL). Trifluoroacetic acid (545 mg, 4.78 mmol) was added. After the system was cooled to 0°C, N-iodosuccinimide (538 mg, 2.39 mmol) was added. The reaction flask underwent nitrogen displacement, and a reaction was carried out for 3 hours. After the reaction was complete, the reaction system was quenched with an aqueous sodium sulfite solution and extracted with dichloromethane (5 mL × 3). The organic phase was washed with a saturated aqueous sodium chloride solution (5 mL × 3), dried over anhydrous sodium sulfate, and filtered. Organic phases were combined and concentrated under reduced pressure. The crude product was purified by column chromatography (dichloromethane : methanol = 40 : 1) to obtain compound **10f** (as a yellow solid, 270 mg, yield 35%).

LCMS m/z = 484.10 [M+1].

### Step 5

### N-(4-(4-amino-5-(3-cyano-4-((4-methylpyrimidin-2-yl)oxy)phenyl)-7-methyl-7H-pyrrolo[2,3-d]pyrimidin-6-yl)phenyl]-2-cyclopropylacrylamide compound 10

Compound **10f** (200 mg, 0.41mmol) and compound **1c** (160 mg, 0.50 mmol) were dissolved in N,N-dimethylformamide : water = 20 : 1 (4 mL). Potassium phosphate (263 mg, 1.24 mmol) and 1,1-bis(diphenylphosphino)ferrocene palladium dichloride (30 mg, 0.04 mmol) were added. After nitrogen displacement, the reaction flask was placed in an oil bath at 90°C for 2 hours of reaction. After the reaction was complete, the reaction mixture was quenched with water (5 mL) and extracted with ethyl acetate (10 mL × 3). The organic phase was washed with a saturated aqueous sodium chloride solution (10 mL × 3), dried over anhydrous sodium sulfate, and filtered. Organic phases were combined and concentrated under reduced pressure. The crude product was purified by column chromatography (dichloromethane : methanol = 30 : 1) to obtain **compound 10** (as a white solid, 100 mg, yield 38%).

¹H NMR (400 MHz, DMSO-d₆) δ 10.02 (s, 1H), 8.53 - 8.49 (m, 2H), 7.82 (d, *J* = 2.0 Hz, 1H), 7.80 (d, *J* = 2.0 Hz, 1H), 7.78 (d, *J =* 2.0 Hz, 1H), 7.51 (dd, *J* = 8.4, 2.0 Hz, 1H), 7.42 (d, *J* = 8.4 Hz, 1H), 7.37 - 7.33 (m, 2H), 7.25 (d, *J* = 4.8 Hz, 1H), 7.01 (s, 2H), 5.63 (s, 1H), 5.28 (d, *J* = 1.2 Hz, 1H), 3.67 (s, 3H), 2.44 (s, 3H), 1.79 - 1.73 (m, 1H), 0.81 - 0.76 (m, 2H), 0.57 - 0.54 (m, 2H).

LCMS m/z = 543.20 [M+1].

### Example 11

### N-(4-(4-amino-5-(3-chloro-4-((4-methylpyrimidin-2-yl)oxy)phenyl)-7-methyl-7H-pyrrolo[2,3-d]pyrimidin-6-yl)phenyl]-2-cyclopropylacrylamide compound 11

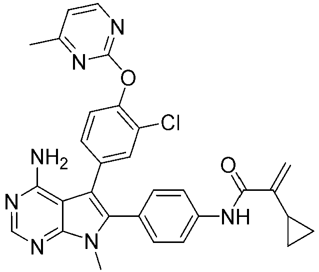

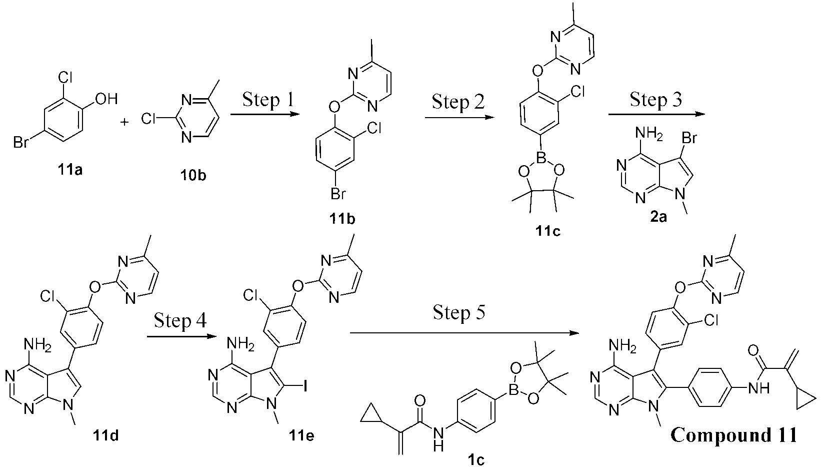

### Step 1

### 2-(4-Bromo-2-chlorophenoxy)-4-methylpyrimidine 11b

4-Bromo-2-chlorophenol **11a** (3.0 g, 14.46 mmol), compound **10b** (1.86 g, 14.46 mmol), and potassium hydroxide (0.812 g, 14.46 mmol) were added to dimethyl sulfoxide (20 mL). The mixture was heated in an oil bath at 150°C and reacted for 4 hours. The mixture was cooled to room temperature and reacted. Water (40 mL) and ethyl acetate (3 × 20 mL) were added, and layers were separated. The organic layer was washed with brine (40 mL), dried, and concentrated. The crude product was purified by column chromatography (petroleum ether : ethyl acetate = 5 : 1) to obtain compound **11b** (as a yellow solid, 3.1 g, yield 70%).

LCMS m/z = 299.10 [M+1].

### Step 2

### 2-(2-Chloro-4-(4,4,5,5-tetramethyl-1,3,2-dioxaborolan-2-yl)phenoxy)-4-methylpyrimidine 11c

Compound **11b** (3.1 g, 8.9 mmol) and bis(pinacolato)diboron (3.4 g, 13.41 mmol) were dissolved in 1,4-dioxane (30 mL). Potassium acetate (2.86 g, 26.83 mmol) and 1,1-bis(diphenylphosphino)ferrocene palladium dichloride (651 mg, 0.89 mmol) were added. After nitrogen displacement, the reaction flask was placed in an oil bath at 95°C for a reaction. After 5 hours, the reaction system was concentrated. The crude product was purified by column chromatography (petroleum ether : ethyl acetate = 5 : 1) to obtain compound **11c** (as a yellow solid, 2.4 g, yield 80%).

LCMS m/z = 347.10 [M+1].

### Step 3

### 5-(3-Chloro-4-((4-methylpyrimidin-2-yl)oxy)phenyl)-7-methyl-7H-pyrrolo[2,3-d]pyrimidin-4-amine 11d

Compound **2a** (1.11 g, 4.88 mmol) and compound **11c** (2.2 g, 6.34 mmol) were dissolved in N,N-dimethylformamide : water = 5 : 1 (20 mL). Potassium phosphate (3.11 g, 14.64 mmol) and 1,1-bis(diphenylphosphino)ferrocene palladium dichloride (360 mg, 0.488 mmol) were added. After nitrogen displacement, the reaction flask was placed in an oil bath at 90°C for a reaction. After 4 hours, the reaction mixture was diluted with water (20 mL) and extracted with ethyl acetate (3 × 20 mL). The organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (dichloromethane : methanol = 40 : 1) to obtain compound **11d** (as a yellow solid, 620 mg, yield 35%).

LCMS m/z = 367.10 [M+1].

### Step 4

### 5-(3-Chloro-4-((4-methylpyrimidin-2-yl)oxy)phenyl)-6-iodo-7-methyl-7H-pyrrolo[2,3-d]pyrimidin-4-amine 11e

Compound **11d** (610 mg, 1.66 mmol) was dissolved in dichloromethane (5 mL). Trifluoroacetic acid (568 mg, 4.98 mmol) was added. After cooling to 0°C, N-iodosuccinimide (561 mg, 2.49 mmol) was added. After the reaction flask underwent nitrogen displacement, a reaction was carried out for 3 hours. The reaction system was quenched with an aqueous sodium sulfite solution and extracted with dichloromethane (3 × 5 mL). The organic phase was washed with a saturated aqueous sodium chloride solution (3 × 5 mL), dried over anhydrous sodium sulfate, and filtered. Organic phases were combined and concentrated under reduced pressure. The crude product was purified by column chromatography (dichloromethane : methanol = 40 : 1) to obtain compound **11e** (as a yellow solid, 500 mg, yield 61%).

LCMS m/z = 493.10 [M+1].

### Step 5

### N-(4-(4-amino-5-(3-chloro-4-((4-methylpyrimidin-2-yl)oxy)phenyl)-7-methyl-7H-pyrrolo[2,3-d]pyrimidin-6-yl)phenyl]-2-cyclopropylacrylamide compound 11

Compound **11e** (200 mg, 0.405 mmol) and compound **1c** (160 mg, 0.5 mmol) were dissolved in N,N-dimethylformamide : water = 20 : 1 (4 mL). Potassium phosphate (263 mg, 1.24 mmol) and 1,1-bis(diphenylphosphino)ferrocene palladium dichloride (30 mg, 0.041 mmol) were added. After nitrogen displacement, the reaction flask was placed in an oil bath at 90°C for a reaction. After 2 hours of reaction, the reaction mixture was quenched with water (5 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 11** (as a white solid, 27 mg, yield 12%).

¹H NMR (400 MHz, DMSO-d₆) δ 10.02 (s, 1H), 8.46 (d, *J* = 4.0 Hz, 2H), 7.83 - 7.75 (m, 2H), 7.44 (d, *J* = 2.4 Hz, 1H), 7.39 - 7.29 (m, 3H), 7.29 - 7.11 (m, 2H), 7.01 (s, 2H), 5.63 (s, 1H), 5.31 - 5.26 (m, 1H), 3.65 (s, 3H), 2.42 (s, 3H), 1.79 - 1.74 (m, 1H), 0.83 - 0.72 (m, 2H), 0.63 - 0.49 (m, 2H).

LCMS m/z = 552.20 [M+1].

### Example 12

### N-(4-(4-amino-5-(3-chloro-5-fluoro-4-((4-methylpyrimidin-2-yl)oxy)phenyl)-7-methyl-7H-pyrrolo[2,3-d]pyrimidin-6-yl)phenyl]-2-cyclopropylacrylamide compound 12

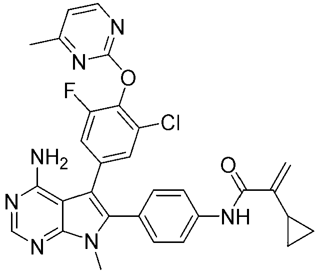

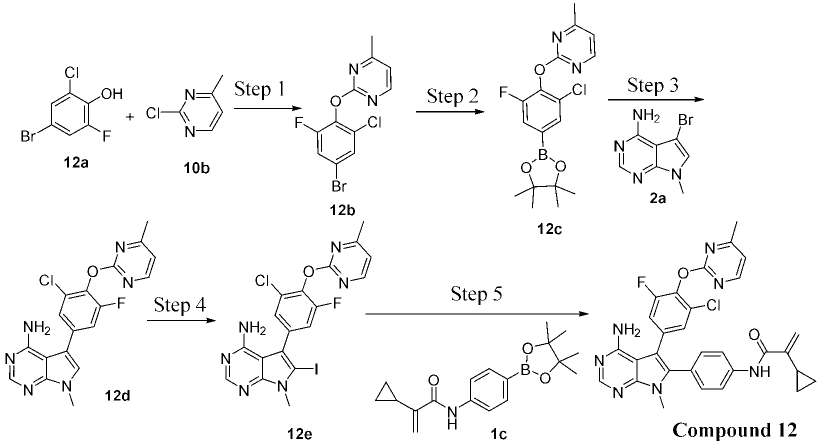

### Step 1

### 2-(4-Bromo-2-chloro-6-fluorophenoxy)-4-methylpyrimidine 12b

Compound **12a** (5.0 g, 22.17 mmol), compound **10b** (2.85 g, 22.17 mmol), and potassium hydroxide (1.25 g, 22.17 mmol) were added to dimethyl sulfoxide (40 mL). The mixture was heated in an oil bath at 150°C and reacted for 4 hours. The mixture was cooled to room temperature and reacted. Water (40 mL) and ethyl acetate (3 × 20 mL) were added, and layers were separated. The organic layer was washed with brine (40 mL), dried, and concentrated. The crude product was purified by column chromatography (petroleum ether : ethyl acetate = 5 : 1) to obtain compound **12b** (as a yellow liquid, 3.6 g, yield 51%).

LCMS m/z = 317.10 [M+1].

### Step 2

### 2-(2-Chloro-6-fluoro-4-(4,4,5,5-tetramethyl-1,3,2-dioxaborolan-2-yl)phenoxy)-4-methylpyrimidine 12c

Compound **12b** (2.6 g, 8.18 mmol) and bis(pinacolato)diboron (3.12 g, 12.28 mmol) were dissolved in 1,4-dioxane (30 mL). Potassium acetate (2.41 g, 24.56 mmol) and 1,1-bis(diphenylphosphino)ferrocene palladium dichloride (600 mg, 0.82 mmol) were added. After nitrogen displacement, the reaction flask was placed in an oil bath at 95°C for a reaction. After 5 hours, the reaction solution was concentrated. The crude product was purified by column chromatography (petroleum ether : ethyl acetate = 8 : 1) to obtain compound **12c** (as a light yellow solid, 2.6 g, yield 86%).

LCMS m/z = 365.10 [M+1].

### Step 3

### 5-(3-Chloro-5-fluoro-4-((4-methylpyrimidin-2-yl)oxy)phenyl)-7-methyl-7H-pyrrolo[2,3-d]pyrimidin-4-amine 12d

Compound **2a** (1.24 g, 5.48 mmol) and compound **12c** (2.4 g, 6.58 mmol) were dissolved in N,N-dimethylformamide : water = 5 : 1 (20 mL). Potassium phosphate (3.5 g, 16.45 mmol) and 1,1-bis(diphenylphosphino)ferrocene palladium dichloride (402 mg, 0.548 mmol) were added. After nitrogen displacement, the reaction flask was placed in an oil bath at 90°C for a reaction. After 4 hours, the reaction mixture was diluted with water (20 mL) and extracted with ethyl acetate (3 × 20 mL). The organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (dichloromethane : methanol = 40 : 1) to obtain compound **12d** (as a yellow solid, 1.3 g, yield 62%).

LCMS m/z = 385.10 [M+1].

### Step 4

### 5-(3-Chloro-5-fluoro-4-((4-methylpyrimidin-2-yl)oxy)phenyl)-6-iodo-7-methyl-7H-pyrrolo[2,3-d]pyrimidin-4-amine 12e

Compound **12d** (1 g, 2.6 mmol) was dissolved in dichloromethane (5 mL). Trifluoroacetic acid (889 mg, 7.8 mmol) was added. After cooling to 0°C, N-iodosuccinimide (877 mg, 3.9 mmol) was added. After the reaction flask underwent nitrogen displacement, a reaction was carried out for 3 hours. The reaction system was quenched with an aqueous sodium sulfite solution and extracted with dichloromethane (3 × 5 mL). The organic phase was washed with a saturated aqueous sodium chloride solution (3 × 5 mL), dried over anhydrous sodium sulfate, and filtered. Organic phases were combined and concentrated under reduced pressure. The crude product was purified by column chromatography (dichloromethane : methanol = 40 : 1) to obtain compound **12e** (as a white solid, 580 mg, yield 44%).

LCMS m/z = 511.10 [M+1].

### Step 5

### N-(4-(4-amino-5-(3-chloro-5-fluoro-4-((4-methylpyrimidin-2-yl)oxy)phenyl)-7-methyl-7H-pyrrolo[2,3-d]pyrimidin-6-yl)phenyl]-2-cyclopropylacrylamide compound 12

Compound **12e** (200 mg, 0.39 mmol) and compound **1c** (160 mg, 0.51 mmol) were dissolved in N,N-dimethylformamide : water = 20 : 1 (4 mL). Potassium phosphate (250 mg, 1.17 mmol) and 1,1-bis(diphenylphosphino)ferrocene palladium dichloride (30 mg, 0.04 mmol) were added. After nitrogen displacement, the reaction flask was placed in an oil bath at 90°C for a reaction. After 2 hours of reaction, the reaction mixture was quenched with water (5 mL) and extracted with ethyl acetate (3 × 10 mL). After the extraction was complete, the organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 12** (as a white solid, 22 mg, yield 10%).

¹H NMR (400 MHz, DMSO-d₆) δ 10.05 (s, 1H), 8.52 - 8.45 (m, 2H), 7.85 - 7.72 (m, 2H), 7.41 - 7.34 (m, 2H), 7.37 - 7.20 (m, 3H), 7.01 (s, 2H), 5.64 (s, 1H), 5.29 (s, 1H), 3.64 (s, 3H), 2.43 (s, 3H), 1.79 - 1.73 (m, 1H), 0.83 - 0.74 (m, 2H), 0.61 - 0.52 (m, 2H).

LCMS m/z = 570.20 [M+1].

### Example 13

### N-(4-(4-amino-5-(3,5-difluoro-4-((4-methylpyrimidin-2-yl)oxy)phenyl)-7-methyl-7H-pyrrolo[2,3-d]pyrimidin-6-yl)phenyl]-2-cyclopropylacrylamide compound 13

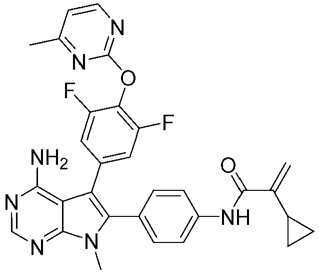

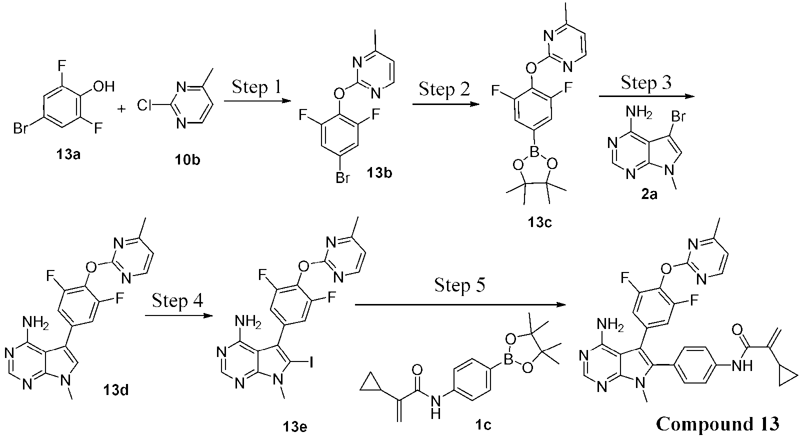

### Step 1

### 2-(4-Bromo-2,6-difluorophenoxy)-4-methylpyrimidine 13b

Compound 13a (3.0 g, 14.35 mmol), compound **10b** (1.85 g, 14.35 mmol), and potassium hydroxide (0.81 g, 14.35 mmol) were added to dimethyl sulfoxide (40 mL). The mixture was heated in an oil bath at 150°C and reacted for 4 hours. The mixture was cooled to room temperature and reacted. Water (40 mL) and ethyl acetate (3 × 20 mL) were added, and layers were separated. The organic layer was washed with brine (40 mL), dried, and concentrated. The crude product was purified by column chromatography (petroleum ether : ethyl acetate = 5 : 1) to obtain compound **13b** (as a yellow solid, 1.2 g, yield 27%).

LCMS m/z = 301.10 [M+1].

### Step 2

### 2-(2,6-Difluoro-4-(4,4,5,5-tetramethyl-1,3,2-dioxaborolan-2-yl)phenoxy)-4-methylpyrimidine 13c

Compound **13b** (1.2 g, 3.98 mmol) and bis(pinacolato)diboron (1.52 g, 5.97 mmol) were dissolved in 1,4-dioxane (10 mL). Potassium acetate (1.17 g, 11.95 mmol) and 1,1-bis(diphenylphosphino)ferrocene palladium dichloride (292 mg, 0.398 mmol) were added. After nitrogen displacement, the reaction flask was placed in an oil bath at 95°C for a reaction. After 5 hours, the reaction solution was concentrated. The crude product was purified by column chromatography (petroleum ether : ethyl acetate = 8 : 1) to obtain compound **13c** (as a light yellow liquid, 1.38 g, yield 90%).

LCMS m/z = 349.10 [M+1].

### Step 3

### 5-(3,5-Difluoro-4-((4-methylpyrimidin-2-yl)oxy)phenyl)-7-methyl-7H-pyrrolo[2,3-d]pyrimidin-4-amine 13d

Compound **2a** (749 mg, 3.3 mmol) and compound **13c** (1.68 g, 3.96 mmol) were dissolved in N,N-dimethylformamide : water = 5 : 1 (10 mL). Potassium phosphate (2.1 g, 9.9 mmol) and 1,1-bis(diphenylphosphino)ferrocene palladium dichloride (241 mg, 0.33 mmol) were added. After nitrogen displacement, the reaction flask was placed in an oil bath at 90°C for a reaction. After 4 hours, the reaction mixture was diluted with water (20 mL) and extracted with ethyl acetate (3 × 20 mL). The organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (dichloromethane : methanol = 40 : 1) to obtain compound **13d** (as a yellow solid, 400 mg, yield 33%).

LCMS m/z = 369.10 [M+1].

### Step 4

### 5-(3,5-Difluoro-4-((4-methylpyrimidin-2-yl)oxy)phenyl)-6-iodo-7-methyl-7H-pyrrolo[2,3-d]pyrimidin-4-amine 13e

Compound **13d** (400 mg, 1.08 mmol) was dissolved in dichloromethane (5 mL). Trifluoroacetic acid (372 mg, 3.26 mmol) was added. After cooling to 0°C, N-iodosuccinimide (367 mg, 1.63 mmol) was added. After the reaction flask underwent nitrogen displacement, a reaction was carried out for 3 hours. The reaction system was quenched with an aqueous sodium sulfite solution and extracted with dichloromethane (3 × 5 mL). The organic phase was washed with a saturated aqueous sodium chloride solution (3 × 5 mL), dried over anhydrous sodium sulfate, and filtered. Organic phases were combined and concentrated under reduced pressure. The crude product was purified by column chromatography (dichloromethane : methanol = 40 : 1) to obtain compound **13e** (as a white solid, 280 mg, yield 52%).

LCMS m/z = 495.10 [M+1].

### Step 5

### N-(4-(4-amino-5-(3,5-difluoro-4-((4-methylpyrimidin-2-yl)oxy)phenyl)-7-methyl-7H-pyrrolo[2,3-d]pyrimidin-6-yl)phenyl]-2-cyclopropylacrylamide compound 13

Compound **13e** (200 mg, 0.404 mmol) and compound **1c** (165 mg, 0.526 mmol) were dissolved in N,N-dimethylformamide : water = 20 : 1 (4 mL). Potassium phosphate (258 mg, 1.21 mmol) and 1,1-bis(diphenylphosphino)ferrocene palladium dichloride (30 mg, 0.04 mmol) were added. After nitrogen displacement, the reaction flask was placed in an oil bath at 90°C for a reaction. After 2 hours, the reaction mixture was quenched with water (5 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 13** (as a white solid, 110 mg, yield 49%).

¹H NMR (400 MHz, DMSO-d₆) δ 10.05 (s, 1H), 8.53 - 8.46 (m, 2H), 7.85 - 7.77 (m, 2H), 7.41 - 7.33 (m, 2H), 7.25 (d, *J* = 4.8 Hz, 1H), 7.18 - 7.07 (m, 2H), 7.01 (s, 2H), 5.64 (s, 1H), 5.29 (d, *J* = 2.0 Hz, 1H), 3.65 (s, 3H), 2.44 (s, 3H), 1.79 - 1.74 (m, 1H), 0.83 - 0.72 (m, 2H), 0.63 - 0.52 (m, 2H).

LCMS m/z = 554.20 [M+1].

### Example 14

### N-(4-(4-amino-5-(3-fluoro-4-((4-(trifluoromethyl)pyrimidin-2-yl)oxy)phenyl)-7-methyl-7H-pyrrolo[2,3-d]pyrimidin-6-yl)phenyl]-2-cyclopropylacrylamide compound 14

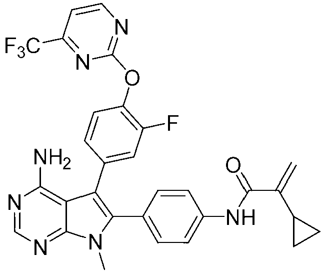

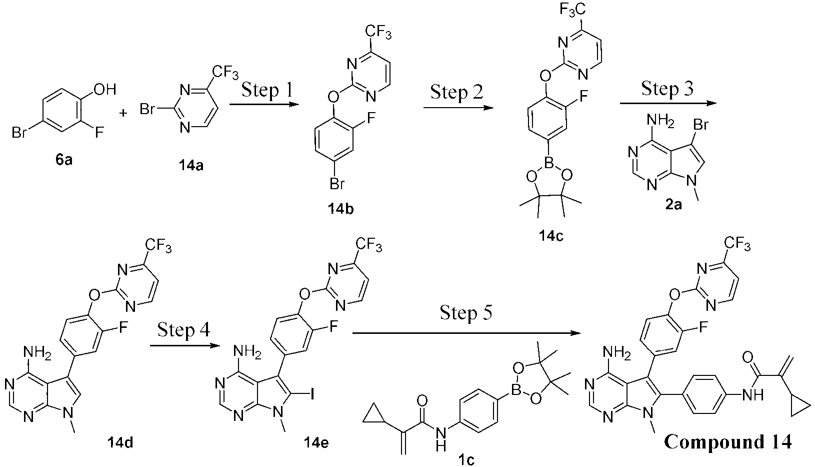

### Step 1

### 2-(4-Bromo-2-fluorophenoxy)-4-(trifluoromethyl)pyrimidine 14b

Compound **6a** (4.1 g, 21.58 mmol), compound **14a** (4.9 g, 21.58 mmol), and potassium hydroxide (1.3 g, 21.58 mmol) were added to dimethyl sulfoxide (40 mL). The mixture was heated in an oil bath at 100°C and reacted for 4 hours. The mixture was cooled to room temperature and reacted. Water (40 mL) and ethyl acetate (3 × 20 mL) were added, and layers were separated. The organic layer was washed with brine (40 mL), dried, and concentrated. The crude product was purified by column chromatography (petroleum ether : ethyl acetate = 10 : 1) to obtain compound **14b** (as a white solid, 4.4 g, yield 60%).

LCMS m/z = 336.90 [M+1].

### Step 2

### 2-(2-Fluoro-4-(4,4,5,5-tetramethyl-1,3,2-dioxaborolan-2-yl)phenoxy)-4-(trifluoromethyl)pyrimidine 14c

Compound **14b** (2.7 g, 8.0 mmol) and bis(pinacolato)diboron (3.05 g, 12.0 mmol) were dissolved in 1,4-dioxane (30 mL). Potassium acetate (2.36 g, 24.0 mmol) and 1,1-bis(diphenylphosphino)ferrocene palladium dichloride (586 mg, 0.8 mmol) were added. After nitrogen displacement, the reaction flask was placed in an oil bath at 95°C for a reaction. After 5 hours, the reaction solution was concentrated. The crude product was purified by column chromatography (petroleum ether : ethyl acetate = 10 : 1) to obtain compound **14c** (as a white solid, 2.3 g, yield 75%).

LCMS m/z = 385.10 [M+1].

### Step 3

### 5-(3-Fluoro-4-((4-(trifluoromethyl)pyrimidin-2-yl)oxy)phenyl)-7-methyl-7H-pyrrolo[2,3-d]pyrimidin-4-amine 14d

Compound **2a** (985 mg, 4.34 mmol) and compound **14c** (2.0 g, 5.2 mmol) were dissolved in N,N-dimethylformamide : water = 5 : 1 (10 mL). Potassium phosphate (2.76 g, 13.20 mmol) and 1,1-bis(diphenylphosphino)ferrocene palladium dichloride (318 mg, 0.434 mmol) were added. After nitrogen displacement, the reaction flask was placed in an oil bath at 90°C for a reaction. After 4 hours, the reaction mixture was diluted with water (20 mL) and extracted with ethyl acetate (3 × 20 mL). The organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (dichloromethane : methanol = 40 : 1) to obtain compound **14d** (as a yellow solid, 880 mg, yield 50%).

LCMS m/z = 405.10 [M+1].

### Step 4

### 5-(3-Fluoro-4-((4-(trifluoromethyl)pyrimidin-2-yl)oxy)phenyl)-6-iodo-7-methyl-7H-pyrrolo[2,3-d]pyrimidin-4-amine 14e

Compound **14d** (880 mg, 2.17 mmol) was dissolved in dichloromethane (5 mL). Trifluoroacetic acid (744 mg, 6.53 mmol) was added. After cooling to 0°C, N-iodosuccinimide (734 mg, 3.26 mmol) was added. After the reaction flask underwent nitrogen displacement, a reaction was carried out for 3 hours. The reaction system was quenched with an aqueous sodium sulfite solution and extracted with dichloromethane (3 × 5 mL). The organic phase was washed with a saturated aqueous sodium chloride solution (3 × 5 mL), dried over anhydrous sodium sulfate, and filtered. Organic phases were combined and concentrated under reduced pressure. The crude product was purified by column chromatography (dichloromethane : methanol = 40 : 1) to obtain compound **14e** (as a white solid, 600 mg, yield 52%).

LCMS m/z = 531.10 [M+1].

### Step 5

### N-(4-(4-amino-5-(3-fluoro-4-((4-(trifluoromethyl)pyrimidin-2-yl)oxy)phenyl)-7-methyl-7H-pyrrolo[2,3-d]pyrimidin-6-yl)phenyl]-2-cyclopropylacrylamide compound 14

Compound **14e** (200 mg, 0.377 mmol) and compound 1c (154 mg, 0.49 mmol) were dissolved in N,N-dimethylformamide : water = 20 : 1 (4 mL). Potassium phosphate (240 mg, 1.13 mmol) and 1,1-bis(diphenylphosphino)ferrocene palladium dichloride (28 mg, 0.037 mmol) were added. After nitrogen displacement, the reaction flask was placed in an oil bath at 90°C for a reaction. After 2 hours, the reaction mixture was quenched with water (5 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 14** (as a white solid, 8 mg, yield 4%).

¹H NMR (400 MHz, DMSO-d₆) δ 10.02 (s, 1H), 9.04 (d, *J =* 4.8 Hz, 1H), 8.48 (s, 1H), 7.86 (d, *J* = 4.0 Hz, 1H), 7.82 - 7.74 (m, 2H), 7.46 (t, *J* = 8.4 Hz, 1H), 7.38 - 7.23 (m, 3H), 7.16 - 7.08 (m, 3H), 5.62 (s, 1H), 5.29 (d, *J =* 2.0 Hz, 1H), 3.66 (s, 3H), 1.81 - 1.69 (m, 1H), 0.84 - 0.71 (m, 2H), 0.63 - 0.49 (m, 2H).

LCMS m/z = 590.20 [M+1].

### Example 15

### N-(4-(4-amino-5-(3-fluoro-4-((4-(difluoromethyl)pyrimidin-2-yl)oxy)phenyl)-7-methyl-7H-pyrrolo[2,3-d]pyrimidin-6-yl)phenyl]-2-cyclopropylacrylamide compound 15

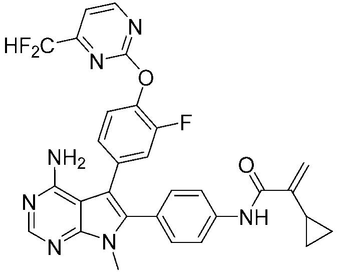

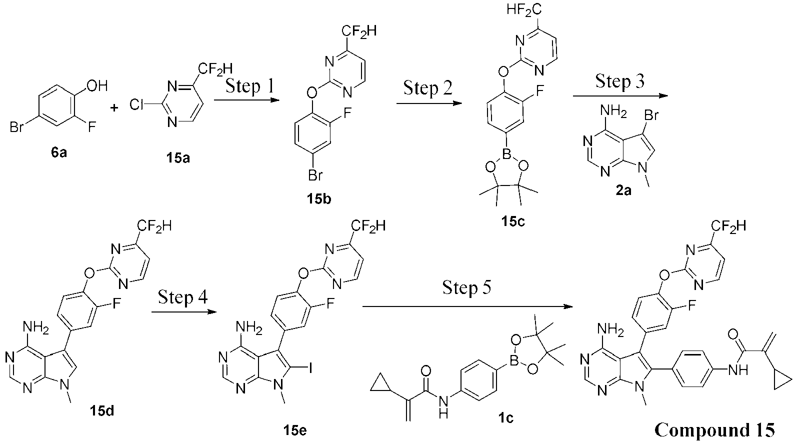

### Step 1

### 2-(4-Bromo-2-fluorophenoxy)-4-(difluoromethyl)pyrimidine 15b

Compound **6a** (1.04 g, 5.46 mmol), compound **15a** (900 mg, 5.46 mmol), and potassium hydroxide (307 mg, 5.46 mmol) were added to dimethyl sulfoxide (10 mL). The mixture was heated in an oil bath at 100°C and reacted for 4 hours. The mixture was cooled to room temperature and reacted. The reaction mixture was extracted with water (10 mL) and ethyl acetate (3 × 10 mL). The organic layer was washed with a saturated aqueous sodium chloride solution (10 mL), dried, and concentrated. The crude product was purified by column chromatography (petroleum ether : ethyl acetate = 10 : 1) to obtain compound **15b** (as a white solid, 1.4 g, yield 80%).

LCMS m/z = 318.90 [M+1].

### Step 2

### 4-(Difluoromethyl)-2-(2-fluoro-4-(4,4,5,5-tetramethyl-1,3,2-dioxaborolan-2-yl)phenoxy)pyrimidine 15c

Compound **15b** (1.4 g, 4.38 mmol) and bis(pinacolato)diboron (1.67 g, 6.58 mmol) were dissolved in 1,4-dioxane (20 mL). Potassium acetate (1.3 g, 13.16 mmol) and 1,1-bis(diphenylphosphino)ferrocene palladium dichloride (321 mg, 0.438 mmol) were added. After nitrogen displacement, the reaction flask was placed in an oil bath at 95°C for 5 hours of reaction. After the reaction was complete, the reaction mixture was cooled. The reaction solution was concentrated. The crude product was purified by column chromatography (petroleum ether : ethyl acetate = 10 : 1) to obtain compound **15c** (as a white solid, 1.2 g, yield 75%).

LCMS m/z = 367.10 [M+1].

### Step 3

### 5-(4-((4-(Difluoromethyl)pyrimidin-2-yl)oxy)-3-fluorophenyl)-7-methyl-7H-pyrrolo[2,3-d]pyrimidin-4-amine 15d

5-Bromo-7-methyl-4-aminopyrrolo[2,3-d]pyrimidine **2a** (568 mg, 2.5 mmol) and compound **15c** (1.1 g, 5.2 mmol) were dissolved in N,N-dimethylformamide : water = 5 : 1 (10 mL). Potassium phosphate (1.6 g, 7.5 mmol) and 1,1-bis(diphenylphosphino)ferrocene palladium dichloride (183 mg, 0.25 mmol) were added. After nitrogen displacement, the reaction flask was placed in an oil bath at 90°C for a reaction. After 4 hours of reaction, the reaction mixture was cooled to room temperature, diluted with water (20 mL), and extracted with ethyl acetate (3 × 10 mL). The organic phase was washed with a saturated aqueous sodium chloride solution (3 × 10 mL), dried over anhydrous sodium sulfate, filtered, and concentrated under reduced pressure. The crude product was purified by column chromatography (dichloromethane : methanol = 40 : 1) to obtain compound **15d** (as a yellow solid, 320 mg, yield 33%).

LCMS m/z = 387.10 [M+1].

### Step 4

### 5-(4-((4-(Difluoromethyl)pyrimidin-2-yl)oxy)-3-fluorophenyl)-6-iodo-7-methyl-7H-pyrrolo[2,3-d]pyrimidin-4-amine 15e

Compound **15d** (320 mg, 0.83 mmol) was dissolved in dichloromethane (5 mL). Trifluoroacetic acid (283 mg, 2.48 mmol) was added. After cooling to 0°C, N-iodosuccinimide (280 mg, 1.24 mmol) was added. After the reaction flask underwent nitrogen displacement, a reaction was carried out for 3 hours. The reaction system was quenched with an aqueous sodium sulfite solution and extracted with dichloromethane (3 × 5 mL). The organic phase was washed with a saturated aqueous sodium chloride solution (3 × 5 mL), dried over anhydrous sodium sulfate, and filtered. Organic phases were combined and concentrated under reduced pressure. The crude product was purified by column chromatography (dichloromethane : methanol = 40 : 1) to obtain compound **15e** (as a white solid, 160 mg, yield 38%).

LCMS m/z = 513.10 [M+1].

### Step 5

### N-(4-(4-amino-5-(3-fluoro-4-((4-(difluoromethyl)pyrimidin-2-yl)oxy)phenyl)-7-methyl-7H-pyrrolo[2,3-d]pyrimidin-6-yl)phenyl]-2-cyclopropylacrylamide compound 15

Compound **15e** (200 mg, 0.39 mmol) and **1c** (159 mg, 0.5 mmol) were dissolved in N,N-dimethylformamide : water = 20 : 1 (4 mL). Potassium phosphate (249 mg, 1.17 mmol) and 1,1-bis(diphenylphosphino)ferrocene palladium dichloride (29 mg, 0.039 mmol) were added. After nitrogen displacement, the reaction flask was placed in an oil bath at 90°C for a reaction. After 2 hours of reaction, the reaction mixture was cooled to room temperature. The reaction mixture was quenched with water (5 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 15** (as a white solid, 20 mg, yield 10%).

¹H NMR (400 MHz, DMSO-d₆) δ 10.02 (s, 1H), 8.89 (d, *J* = 4.0 Hz, 1H), 8.47 (s, 1H), 7.74 (dd, *J* = 30.4, 7.9 Hz, 2H), 7.59 (d, *J =* 4.0 Hz, 1H), 7.43 (t*, J =* 8.4 Hz, 1H), 7.31 (dd, *J* = 26.4, 9.6 Hz, 3H), 7.22 - 7.05 (m, 4H), 5.63 (s, 1H), 5.28 (s, 1H), 3.67 (s, 3H), 1.79 - 1.69 (m, 1H), 0.82 - 0.71 (m, 2H), 0.59 - 0.49 (m, 2H).

LCMS m/z = 572.20 [M+1].

### Example 16

### N-(4-(4-amino-5-(3-fluoro-4-((4-methylpyrimidin-2-yl)oxy)phenyl)-7-methyl-7H-pyrrolo[2,3-d]pyrimidin-6-yl)-2-chlorophenyl)-2-cyclopropylacrylamide compound 16

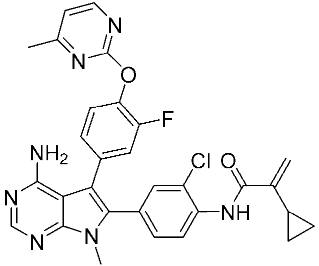

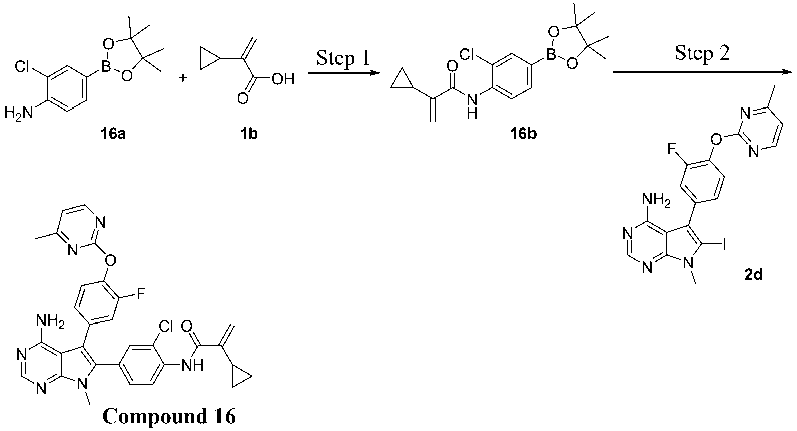

### Step 1

### N-(2-chloro-4-(4,4,5,5-tetramethyl-1,3,2-dioxaborolan-2-yl)phenyl)-2-cyclopropylacrylamide 16b

Compound **16a** (376 mg, 1.48 mmol) and 2-cyclopropylacrylic acid **1b** (200 mg, 1.78 mmol) were dissolved in acetonitrile (8 mL). N,N,N',N'-tetramethylchloroformamidinium hexafluorophosphate (541 mg, 1.93 mmol) and N-methylimidazole (611 mg, 7.4 mmol) were added. After nitrogen displacement, the reaction flask was placed at room temperature for a reaction. After the reaction was complete, the reaction solution was concentrated. After concentration, water (20 mL) was added, followed by ethyl acetate (3 × 15 mL) for extraction. Organic phases were combined and concentrated under reduced pressure to obtain a crude product, which was purified by column chromatography (petroleum ether : ethyl acetate = 4 : 1) to obtain compound **16b** (as a white solid, 300 mg, yield 58.17%).

LCMS m/z = 348.15 [M+1].

### Step 2

### N-(4-(4-amino-5-(3-fluoro-4-((4-methylpyrimidin-2-yl)oxy)phenyl)-7-methyl-7H-pyrrolo[2,3-d]pyrimidin-6-yl)-2-chlorophenyl)-2-cyclopropylacrylamide compound 16

Compound **2d** (316 mg, 0.66 mmol) and compound **16b** (300 mg, 0.86 mmol) were dissolved in N,N-dimethylformamide : water = 20 : 1 (4 mL). Potassium phosphate (422 mg, 1.99 mmol) and 1,1-bis(diphenylphosphino)ferrocene palladium dichloride (48 mg, 0.06 mmol) were added. After nitrogen displacement, the reaction flask was placed in an oil bath at 95°C for a reaction. After the reaction was complete, the reaction mixture was quenched with water (5 mL) and extracted with ethyl acetate (3 × 15 mL). After the extraction was complete, the organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (ethyl acetate : methanol = 20 : 1) to obtain **compound 16** (as a white solid, 61 mg, yield 16.1%).

¹H NMR (400 MHz, DMSO-d₆) δ 9.38 (s, 1H), 8.43 (d, *J* = 4.0 Hz, 1H), 8.18 (s, 1H), 7.87 (d, *J* = 8.4 Hz, 1H), 7.52 (d, *J* = 1.6 Hz, 1H), 7.41 - 7.28 (m, 2H), 7.20 (dd, *J* = 11.2, 2.0 Hz, 1H), 7.14 (d, *J* = 4.0 Hz, 1H), 7.11 - 7.04 (m, 1H), 6.02 (s, 2H), 5.83 (s, 1H), 5.32 (d, *J* = 1.2 Hz, 1H), 3.59 (s, 3H), 2.37 (s, 3H), 1.72 (d, *J* = 8.6, 5.2 Hz, 1H), 0.85 - 0.74 (m, 2H), 0.62 - 0.50 (m, 2H).

LCMS m/z = 570.17 [M+1].

### Example 17

### N-(4-(4-amino-5-(3-fluoro-4-((4-vinylpyrimidin-2-yl)oxy)phenyl)-7-methyl-7H-pyrrolo[2,3-d]pyrimidin-6-yl)phenyl)-2-cyclopropylacrylamide compound 17

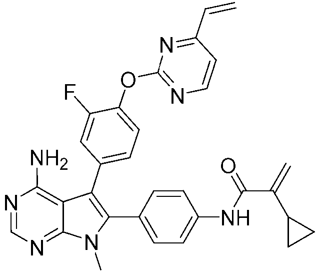

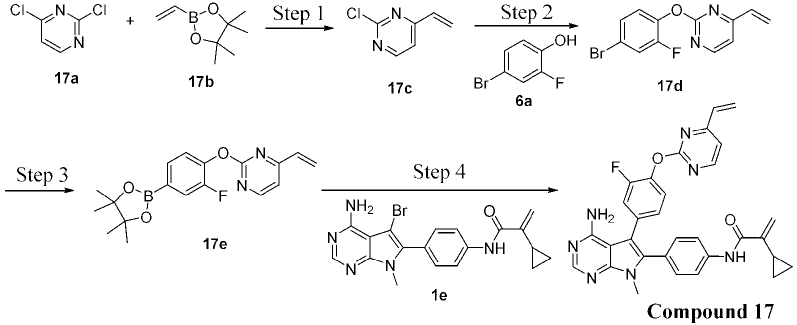

### Step 1

### 2-Chloro-4-vinylpyrimidine 17c

Compound **17a** (4 g, 26.8 mmol) and vinylboronic acid pinacol ester **17b** (4.7 g, 30.8 mmol) were dissolved in 1,4-dioxane : water = 4 : 1 (80 mL). 1,1-Bis(diphenylphosphino)ferrocene palladium dichloride (2.17 g, 2.68 mmol) and sodium carbonate (8.4 g, 80.4 mmol) were added. After nitrogen displacement, the reaction flask was placed in an oil bath at 90°C for a reaction. After the reaction was complete, the reaction mixture was cooled, quenched with water (80 mL), and extracted with ethyl acetate (3 × 50 mL). The organic phase was washed with a saturated aqueous sodium chloride solution (3 × 40 mL), dried over anhydrous sodium sulfate, and filtered. Organic phases were combined and concentrated under reduced pressure. The crude product was purified by column chromatography (petroleum ether : ethyl acetate = 15 : 1) to obtain compound **17c** (as a yellow liquid, 1.2 g, 31.7%).

LCMS m/z = 296.98 [M+1].

### Step 2

### 2-(4-Bromo-2-fluorophenoxy)-4-vinylpyrimidine 17d

Compound **17c** (1.2 g, 8.5 mmol) was dissolved in acetonitrile (25 mL). Compound **6a** (0.8 g, 4.2 mmol) and potassium carbonate (1.18 g, 3.488 mmol) were added. The mixture was heated to 85°C and reacted under reflux for 4 hours. The reaction mixture was quenched with water (25 mL) and extracted with ethyl acetate (3 × 30 mL). The organic phase was washed with a saturated aqueous sodium chloride solution (3 × 30 mL), dried over anhydrous sodium sulfate, and filtered. Organic phases were combined and concentrated under reduced pressure. The crude product was purified by column chromatography (petroleum ether : ethyl acetate = 30 : 1) to obtain compound **17d** (as a yellow solid, 1.2 g, yield 96.7%).

LCMS m/z = 140.01 [M+1].

### Step 3

### 2-(2-Fluoro-4-(4,4,5,5-tetramethyl-1,3,2-dioxaborolan-2-yl)phenoxy)-4-vinylpyrimidine 17e

Compound **17d** (1.2 g, 4.09 mmol), bis(pinacolato)diboron (1.64 g, 6.48 mmol), and potassium acetate (1.27 g, 12.94 mmol) were dissolved in 1,4-dioxane (30 mL). 1,1-Bis(diphenylphosphino)ferrocene palladium dichloride (0.3 g, 0.43 mmol) was then added. After nitrogen displacement, the reaction flask was placed in an oil bath at 95°C for 3 hours of reaction. After the reaction was complete, the reaction mixture was quenched with water (30 mL) and extracted with ethyl acetate (3 mL × 30). The organic phase was washed with a saturated aqueous sodium chloride solution (3 × 30 mL), dried over anhydrous sodium sulfate, and filtered. Organic phases were combined and concentrated under reduced pressure. The crude product was purified by column chromatography (petroleum ether : ethyl acetate = 10 : 1) to obtain compound **17e** (as a yellow solid, 600 mg, yield 40.5%).

LCMS m/z = 343.16 [M+1].

### Step 4

### N-(4-(4-amino-5-(3-fluoro-4-((4-vinylpyrimidin-2-yl)oxy)phenyl)-7-methyl-7H-pyrrolo[2,3-d]pyrimidin-6-yl)phenyl)-2-cyclopropylacrylamide compound 17

Compound **1e** (200 mg, 0.47 mmol) and compound **17e** (187 mg, 0.54 mmol) were dissolved in N,N-dimethylformamide : water = 10 : 1 (4 mL). Potassium phosphate (308 mg, 1.45 mmol) and 1,1-bis(diphenylphosphino)ferrocene palladium dichloride (40 mg, 0.04 mmol) were added. After nitrogen displacement, the reaction flask was placed in an oil bath at 95°C for a reaction. After the reaction was complete, the reaction mixture was quenched with water (5 mL) and extracted with ethyl acetate (3 × 10 mL). The organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (ethyl acetate : methanol = 20 : 1) to obtain **compound 17** (as a yellow solid, 12 mg, yield 8%).

¹H NMR (400 MHz, DMSO-d₆) δ 9.98 (s, 1H), 8.64 (d, *J* = 5.2 Hz, 1H), 8.21 (s, 1H), 7.76 (d, *J =* 8.4 Hz, 2H),7.54 (d, *J=* 11.2 Hz, 1H), 7.41 - 7.27 (m, 3H), 7.20 (d, *J =* 11.2 Hz, 1H), 7.11 (d, *J =* 8.4 Hz, 1H), 6.74 (dd, *J =* 17.2, 10.6 Hz, 1H), 6.32 (d, *J =* 17.2 Hz, 1H), 6.02 (s, 2H), 5.73 (d, *J= 10.4* Hz, 1H), 5.62 (s, 1H), 5.28 (s, 1H), 3.60 (s, 3H), 1.81 - 1.71 (m, 1H), 0.78 (d, *J=* 8.4 Hz, 2H), 0.56 (d, *J =* 4.8 Hz, 2H).

LCMS m/z = 548.21 [M+1].

### Example 18

### 4-(4-Amino-6-(4-(2-cyclopropylacrylamido)phenyl)-7-methyl-7H-pyrrolo[2,3-d]pyrimidin-5-yl)-2-fluorophenyl azetidine-1-carboxylate compound 18

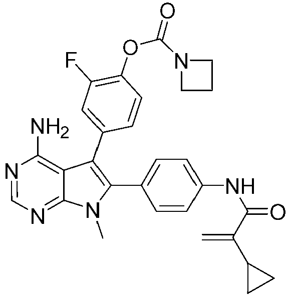

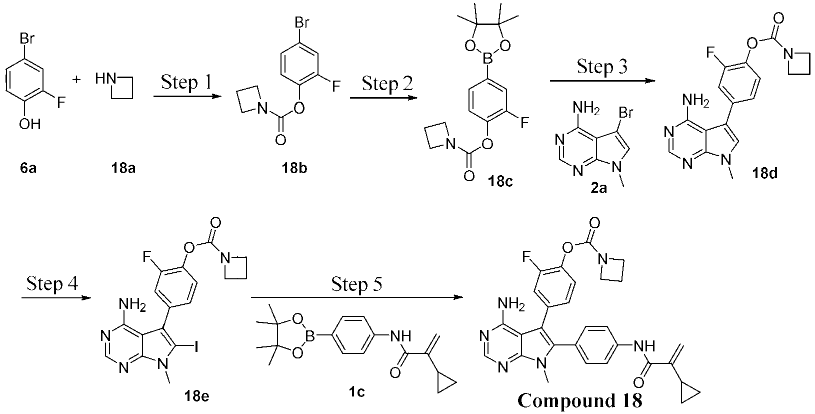

### Step 1

### 4-Bromo-2-fluorophenyl azetidine-1-carboxylate 18b

Triphosgene (6.6 g, 22.26 mmol) was dissolved in dichloromethane (40 mL). At 0°C, a solution of compound **6a** (12 g, 63.6 mmol) and N,N-diisopropylethylamine (8 g, 63.6 mmol) in dichloromethane (40 mL) was slowly dropwise added thereto. The mixture was then heated to room temperature and reacted for 2 hours. A solution of compound **18a** (4 g, 70 mmol) and N,N-diisopropylethylamine (8 g, 63.6 mmol) in dichloromethane (40 mL) was then slowly dropwise added to the reaction solution at 0°C. The mixture was then heated to room temperature and reacted for 2 hours. After cooling, the reaction solution was concentrated. The crude product was purified by column chromatography (petroleum ether : ethyl acetate = 8 : 1) to obtain compound **18b** (as a white solid, 7.811 g, yield 60%).

LCMS m/z = 273.98 [M+1].

### Step 2

### 2-Fluoro-4-(4,4,5,5-tetramethyl-1,3,2-dioxaborolan-2-yl)phenyl azetidine-1-carboxylate 18c

Compound **18b** (7.811 g, 28.5 mmol) and bis(pinacolato)diboron (10.86 g, 42.76 mmol) were dissolved in 1,4-dioxane (80 mL). Potassium acetate (8.38 g, 85.52 mmol) and 1,1-bis(diphenylphosphino)ferrocene palladium dichloride (2.086 g, 2.85 mmol) were added. After nitrogen displacement, the reaction flask was placed in an oil bath at 95°C for a reaction. After 5 hours, the reaction mixture was cooled to room temperature. The reaction solution was concentrated. The crude product was purified by column chromatography (petroleum ether : ethyl acetate = 8 : 1) to obtain compound **18c** (as an orange-yellow oil, 7.953 g, yield 87%).

LCMS m/z = 322.20 [M+1].

### Step 3

### 4-(4-Amino-7-methyl-7H-pyrrolo[2,3-d]pyrimidin-5-yl)-2-fluorophenyl azetidine-1-carboxylate 18d

Compound **2a** (5.62 g, 24.8 mmol) and **18c** (7.953g, 24.8 mmol) were dissolved in N,N-dimethylformamide : water = 5 : 1 (66 mL). Potassium phosphate (15.75 g, 74.3 mmol) and 1,1-bis(diphenylphosphino)ferrocene palladium dichloride (1.82 g, 2.78 mmol) were added. After nitrogen displacement, the reaction flask was placed in an oil bath at 90°C for a reaction. After 4 hours, the reaction mixture was cooled to room temperature, diluted with water (50 mL), and extracted with ethyl acetate (3 × 30 mL). The organic phase was washed with a saturated aqueous sodium chloride solution (3 × 30 mL), dried over anhydrous sodium sulfate, and filtered. Organic phases were combined and concentrated under reduced pressure. The crude product was purified by column chromatography (dichloromethane : methanol = 30 : 1) to obtain compound **18d** (as a yellow solid, 3.971 g, yield 47%).

LCMS m/z = 342.13 [M+1].

### Step 4

### 4-(4-Amino-6-iodo-7-methyl-7H-pyrrolo[2,3-d]pyrimidin-5-yl)-2-fluorophenyl azetidine-1-carboxylate 18e

Compound **18d** (1.97 g, 5.78 mmol) was dissolved in dichloromethane (20 mL). Trifluoroacetic acid (1.98 g, 17.33 mmol) was added. After cooling to 0°C, N-iodosuccinimide (1.94 g, 8.66 mmol) was added. After the reaction flask underwent nitrogen displacement, a reaction was carried out for 3 hours. The reaction mixture was quenched with an aqueous sodium sulfite solution and extracted with dichloromethane (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 40 : 1) to obtain compound **18e** (as a white solid, 1.2 g, yield 44%).

LCMS m/z = 468.10 [M+1].

### Step 5

### 4-(4-Amino-6-(4-(2-cyclopropylacrylamido)phenyl)-7-methyl-7H-pyrrolo[2,3-d]pyrimidin-5-yl)-2-fluorophenyl azetidine-1-carboxylate compound 18

Compound **18e** (300 mg, 0.64 mmol) and compound **1c** (201 mg, 0.64 mmol) were dissolved in N,N-dimethylformamide : water = 20 : 1 (5 mL). Potassium phosphate (408 mg, 1.69 mmol) and 1,1-bis(diphenylphosphino)ferrocene palladium dichloride (47 mg, 0.064 mmol) were added. After nitrogen displacement, the reaction flask was placed in an oil bath at 90°C for a reaction. After 2 hours, the reaction mixture was cooled to room temperature. The reaction mixture was quenched with water (5 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 18** (as a white solid, 150 mg, yield 44%).

¹H NMR (400 MHz, DMSO-d₆) δ 9.94 (s, 1H), 8.20 (s, 1H), 7.72 (d, *J* = 8.4 Hz, 2H), 7.25 (dd, *J* = 12.0, 8.4 Hz, 3H), 7.12 (dd, *J* = 11.2, 2.0 Hz, 1H), 7.03 (dd, *J* = 8.4, 1.9 Hz, 1H), 5.95 (s, 2H), 5.63 (s, 1H), 5.27 (s, 1H), 4.16 (t, *J* = 7.2 Hz, 2H), 3.99 (t, *J* = 7.2 Hz, 2H), 3.59 (s, 3H), 2.28 (q, *J =* 7.6 Hz, 2H), 1.76 (tt, *J =* 8.4, 5.2 Hz, 1H), 0.83 - 0.72 (m, 2H), 0.61 - 0.51 (m, 2H).

LCMS m/z = 527.21 [M+1].

### Example 19

### 4-(4-Amino-6-(4-(2-cyclopropylacrylamido)phenyl)-7-methyl-7H-pyrrolo[2,3-d]pyrimidin-5-yl)-2-fluorophenyl-3-fluoropyrrolidine-1-carboxylate compound 19

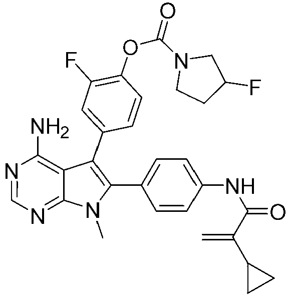

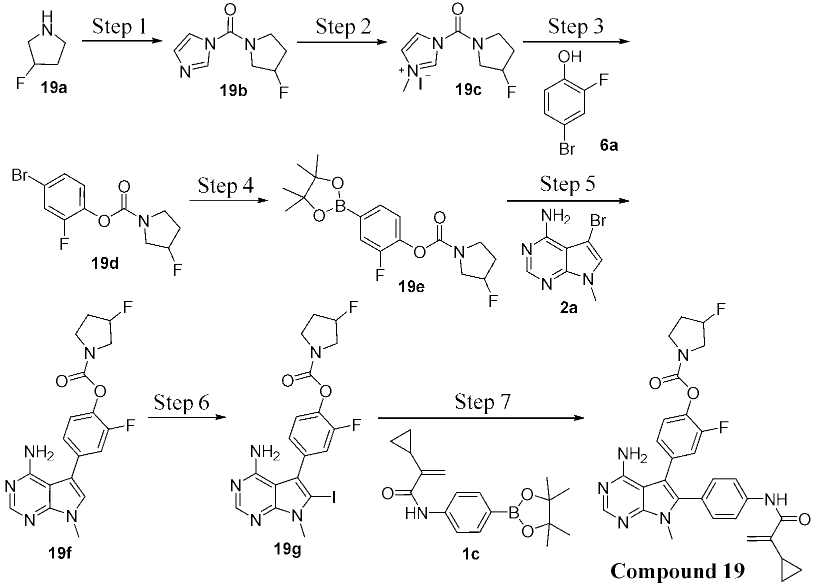

### Step 1

### (3-Fluoropyrrolidin-1-yl)(1H-imidazol-1-yl)methanone 19b

Compound **19a** (2 g, 16 mmol) and N,N'-carbonyldiimidazole (2.84 g, 17.5 mmol) were dissolved in tetrahydrofuran (40 mL). After reaction at 75°C under reflux for 24 hours, the reaction mixture was concentrated under reduced pressure, dissolved with dichloromethane, and extracted with water (3 × 50 mL). The organic phases were combined, dried over anhydrous sodium sulfate, and filtered. Organic phases were combined and concentrated under reduced pressure to obtain compound **19b** (as a yellow liquid, 1.855 g, yield 63%).

LCMS m/z = 184.10 [M+1].

### Step 2

### 1-(3-Fluoropyrrolidine-1-carbonyl)-3-methyl-1H-imidazol-3-ium 19c

Compound **19b** (1.855 g, 21.8 mmol) was dissolved in acetonitrile (20 mL). Iodomethane (2.6 mL, 40.5 mmol) was slowly dropwise added to the system. The system was reacted at room temperature for 24 hours and concentrated under reduced pressure to obtain compound **19c** (as a yellow oil, 3.1 g, yield 94%).

LCMS m/z = 199.10 [M+1].

### Step 3

### 4-Bromo-2-fluorophenyl 3-fluoropyrrolidine-1-carboxylate 19d

Compound **19c** (3.1 g, 9.54 mmol) was dissolved in dichloromethane (40 mL). Compound **6a** (1.46 g, 7.63 mmol) and triethylamine (0.78 g, 7.63 mmol) were added at room temperature. After 3 hours of reaction, the reaction mixture was concentrated under reduced pressure. The crude product was purified by column chromatography (petroleum ether : ethyl acetate = 10 : 1) to obtain compound **19d** (as a yellow liquid, 2.9 g, yield 99%).

LCMS m/z = 305.99 [M+1].

### Step 4

### 2-Fluoro-4-(4,4,5,5-tetramethyl-1,3,2-dioxaborolan-2-yl)phenyl 3-fluoropyrrolidine-1-carboxylate 19e

Compound **19d** (2.9 g, 9.54 mmol), bis(pinacolato)diboron (3.63 g, 14.31 mmol), and potassium acetate (2.8 g, 28.62 mmol) were dissolved in 1,4-dioxane (40 mL). 1,1-Bis(diphenylphosphino)ferrocene palladium dichloride (0.7 g, 0.954 mmol) was then added. After nitrogen displacement, the reaction flask was placed in an oil bath at 90°C for a reaction. After 3 hours of reaction, the reaction mixture was cooled to room temperature, quenched with water (100 mL), and extracted with ethyl acetate (3 × 80 mL). The organic phase was washed with a saturated aqueous sodium chloride solution (3 × 50 mL), dried over anhydrous sodium sulfate, and filtered. Organic phases were combined and concentrated under reduced pressure. The crude product was purified by column chromatography (petroleum ether : ethyl acetate = 10 : 1) to obtain compound **19e** (as a yellow solid, 3.3 g, yield 98%).

LCMS m/z = 354.10 [M+1].

### Step 5

### 4-(4-Amino-7-methyl-7H-pyrrolo[2,3-d]pyrimidin-5-yl)-2-fluorophenyl 3-fluoropyrrolidine-1-carboxylate 19f

Compound **19e** (6.5 g, 18.4 mmol), compound 2a (4.18 g, 18.4 mmol), and potassium phosphate (11.7 g, 55.2 mmol) were dissolved in N,N-dimethylformamide : water = 20 : 1 (60 mL). 1,1-Bis(diphenylphosphino)ferrocene palladium dichloride (1.35 g, 1.84 mmol) was added. After nitrogen displacement, the reaction flask was placed in an oil bath at 90°C for a reaction. After 3 hours, the reaction mixture was cooled to room temperature, quenched with water (50 mL), and extracted with ethyl acetate (3 × 50 mL). The organic phase was washed with a saturated aqueous sodium chloride solution (3 × 50 mL), dried over anhydrous sodium sulfate, and filtered. Organic phases were combined and concentrated under reduced pressure. The crude product was purified by column chromatography (ethyl acetate : methanol = 50 : 1) to obtain compound **19f** (as a yellow solid, 5.758 g, yield 84%).

LCMS m/z = 374.10 [M+1].

### Step 6

### 4-(4-Amino-6-iodo-7-methyl-7H-pyrrolo[2,3-d]pyrimidin-5-yl)-2-fluorophenyl 3-fluoropyrrolidine-1-carboxylate 19g

Compound **19f** (2 g, 5.36 mmol) was dissolved in dichloromethane (20 mL). Trifluoroacetic acid (1.83 g, 16 mmol) was added to the system. After the system was cooled to 0°C, N-iodosuccinimide (1.8 g, 8 mmol) was slowly added. After the reaction system was heated to room temperature and reacted for 2 hours, the reaction mixture was quenched with a saturated aqueous sodium sulfite solution (20 mL). The system was extracted with a dichloromethane (3 × 30 mL). The organic phase was washed with a saturated aqueous sodium chloride solution (3 × 30 mL), dried over anhydrous sodium sulfate, and filtered. Organic phases were combined and concentrated under reduced pressure. The crude product was purified by column chromatography (ethyl acetate : methanol = 30 : 1) to obtain compound **19g** (as a yellow solid, 1.428 g, yield 53%).

LCMS m/z = 500.10 [M+1].

### Step 7

### 4-(4-Amino-6-(4-(2-cyclopropylacrylamido)phenyl)-7-methyl-7H-pyrrolo[2,3-d]pyrimidin-5-yl)-2-fluorophenyl-3-fluoropyrrolidine-1-carboxylate compound 19

Compound **19g** (300 mg, 0.6 mmol) and compound **1c** (188 mg, 0.6 mmol) were dissolved in N,N-dimethylformamide : water = 20 : 1 (5.5 mL). Potassium phosphate (382 mg, 1.8 mmol) and 1,1-bis(diphenylphosphino)ferrocene palladium dichloride (45 mg, 0.06 mmol) were added. After nitrogen displacement, the reaction flask was placed in an oil bath at 90°C for a reaction. After 3 hours of reaction, the reaction mixture was quenched with water (10 mL) and extracted with ethyl acetate (3 × 15 mL). The organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (dichloromethane : methanol = 10 : 1) to obtain **compound 19** (as a white solid, 70 mg, yield 21%).

¹H NMR (400 MHz, DMSO-d₆) δ 9.99 (s, 1H), 8.48 (s, 1H), 7.76 (d, *J* = 8.4 Hz, 2H), 7.31 (d, *J =* 8.4 Hz, 3H), 7.20 (d, *J =* 11.2 Hz, 1H), 7.03 (d, *J =* 8.0 Hz, 1H), 5.64 (s, 1H), 5.28 (s, 1H), 4.25 (s, 1H), 3.84 - 3.69 (m, 2H), 3.66 (s, 3H), 3.65 - 3.33 (m, 3H), 2.19 (m, *J =* 24.0, 14.2, 8.0 Hz, 2H), 1.76 (m, *J =* 13.2, 8.4, 5.2 Hz, 1H), 0.85 - 0.68 (m, 2H), 0.62 - 0.49 (m, 2H).

LCMS m/z = 559.20 [M+1].

### Example 20

### 4-(4-Amino-6-(4-(2-cyclopropylacrylamido)phenyl)-7-methyl-7H-pyrrolo[2,3-d]pyrimidin-5-yl)-2-fluorophenyl piperidine-1-carboxylate compound 20

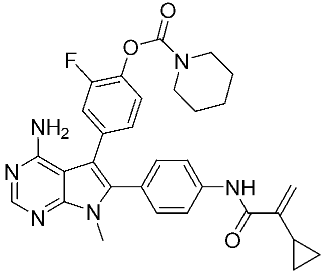

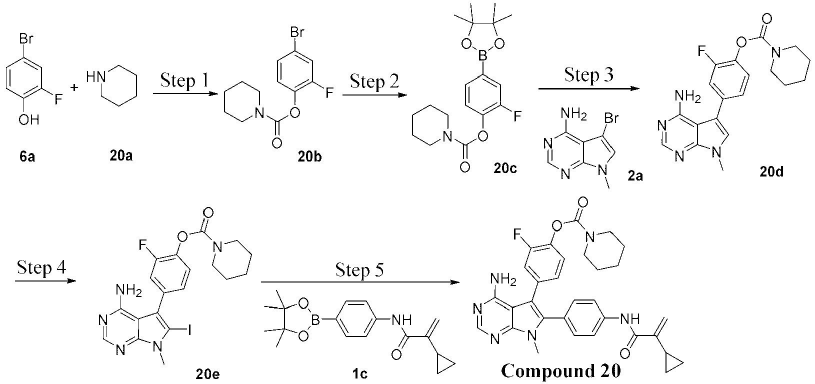

### Step 1

### 4-Bromo-2-fluorophenyl piperidine-1-carboxylate 20b

Triphosgene (3.3 g, 11.13 mmol) was dissolved in dichloromethane (10 mL). At 0°C, a solution of compound **6a** (6 g, 31.8 mmol) and N,N-diisopropylethylamine (4 g, 31.8 mmol) in dichloromethane (20 mL) was slowly dropwise added thereto. The mixture was heated to room temperature and reacted for 2 hours. A solution of compound **20a** (2.98 g, 34.98 mmol) and N,N-diisopropylethylamine (4 g, 31.8 mmol) in dichloromethane (20 mL) was then slowly dropwise added to the reaction solution at 0°C. The mixture was then heated to room temperature and reacted for 2 hours. The reaction solution was concentrated. The crude product was purified by column chromatography (petroleum ether : ethyl acetate = 8 : 1) to obtain compound **20b** (as a yellow liquid, 6.817 g, yield 71%).

LCMS m/z = 302.16 [M+1].

### Step 2

### 2-Fluoro-4-(4,4,5,5-tetramethyl-1,3,2-dioxaborolan-2-yl)phenylpiperidine-1-carboxylate 20c

Compound **20b** (6.817 g, 22.57 mmol) and bis(pinacolato)diboron (8.6 g, 33.86 mmol) were dissolved in 1,4-dioxane (80 mL). Potassium acetate (6.64 g, 67.7 mmol) and 1,1-bis(diphenylphosphino)ferrocene palladium dichloride (1.65 g, 2.257 mmol) were added. After nitrogen displacement, the reaction flask was placed in an oil bath at 95°C for a reaction. After 5 hours of reaction, the reaction solution was cooled to room temperature and concentrated. The crude product was purified by column chromatography (petroleum ether : ethyl acetate = 8 : 1) to obtain compound **20c** (as a white solid, 7.218 g, yield 91%).

LCMS m/z = 350.20 [M+1].

### Step 3

### 4-(4-Amino-7-methyl-7H-pyrrolo[2,3-d]pyrimidin-5-yl)-2-fluorophenyl piperidine-1-carboxylate 20d

Compound **2a** (5.43 g, 23.9 mmol) and compound **20c** (7.218g, 23.9 mmol) were dissolved in N,N-dimethylformamide : water = 5 : 1 (66 mL). Potassium phosphate (15.2 g, 71.7 mmol) and 1,1-bis(diphenylphosphino)ferrocene palladium dichloride (1.75 g, 2.39 mmol) were added. After nitrogen displacement, the reaction flask was placed in an oil bath at 90°C for a reaction. After 4 hours, the reaction mixture was cooled to room temperature, diluted with water (50 mL), and extracted with ethyl acetate (3 × 30 mL). The organic phase was washed with a saturated aqueous sodium chloride solution (3 × 30 mL), dried over anhydrous sodium sulfate, and filtered. Organic phases were combined and concentrated under reduced pressure. The crude product was purified by column chromatography (dichloromethane : methanol = 30 : 1) to obtain compound **20d** (as a white solid, 3.422 g, yield 39%).

LCMS m/z = 370.20 [M+1].

### Step 4

### 4-(4-Amino-6-iodo-7-methyl-7H-pyrrolo[2,3-d]pyrimidin-5-yl)-2-fluorophenyl piperidine-1-carboxylate 20e

Compound **20d** (1.42 g, 3.85 mmol) was dissolved in dichloromethane (15 mL). Trifluoroacetic acid (1.32 g, 11.54 mmol) was added. After cooling to 0°C, N-iodosuccinimide (1.3 g, 5.77 mmol) was added. After the reaction flask underwent nitrogen displacement, a reaction was carried out for 3 hours. The reaction system was quenched with an aqueous sodium sulfite solution and extracted with dichloromethane (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 40 : 1) to obtain compound 20e (as a white solid, 900 mg, yield 47%).

LCMS m/z = 496.10 [M+1].

### Step 5

### 4-(4-Amino-6-(4-(2-cyclopropylacrylamido)phenyl)-7-methyl-7H-pyrrolo[2,3-d]pyrimidin-5-yl)-2-fluorophenyl piperidine-1-carboxylate compound 20

Compound **20e** (300 mg, 0.61mmol) and compound **1c** (190 mg, 0.61 mmol) were dissolved in N,N-dimethylformamide : water = 20 : 1 (4 mL). Potassium phosphate (390 mg, 1.83 mmol) and 1,1-bis(diphenylphosphino)ferrocene palladium dichloride (48 mg, 0.061 mmol) were added. After nitrogen displacement, the reaction flask was placed in an oil bath at 90°C for a reaction. After 2 hours of reaction, the reaction mixture was quenched with water (5 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 20** (as a white solid, 100 mg, yield 30%).

¹H NMR (400 MHz, DMSO-d₆) δ 10.01 (s, 1H), 8.52 (s, 1H), 8.09 - 7.42 (d, *J =* 8.4 Hz, 4H), 7.36 - 7.14 (m, 4H), 7.02 (dd, *J* = 8.2, 2.0 Hz, 1H), 5.64 (s, 1H), 5.28 (s, 1H), 3.67 (s, 3H), 3.40 (d, *J* = 6.4 Hz, 4H), 1.75 (dd, *J* = 8.4, 4.4 Hz, 1H), 1.67 - 1.37 (m, 6H), 0.77 (dd, *J* = 8.4, 3.2 Hz, 2H), 0.66 - 0.43 (m, 2H).

LCMS *m*/*z* = 555.20 [M+1].

### Example 21

### N-(4-(4-amino-5-(3-fluoro-4-((4-methylpyrimidin-2-yl)oxy)phenyl)-7-methyl-7H-pyrrolo[2,3-d]pyrimidin-6-yl)-3-methylphenyl)-2-cyclopropylacrylamide compound 21

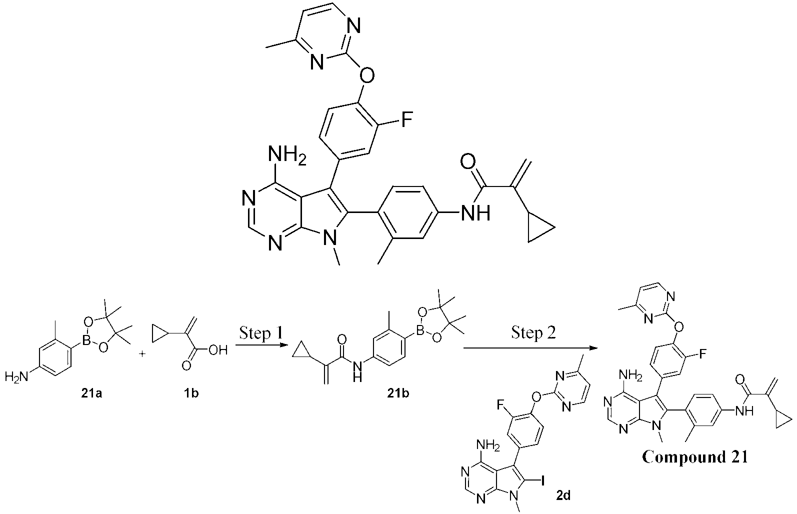

### Step 1

### 2-Cyclopropyl-N-(3-methyl-4-(4,4,5,5-tetramethyl-1,3,2-dioxaborolan-2-yl)phenyl)acrylamide 21b

Compound **1b** (288 mg, 2.57 mmol) was dissolved in N,N-dimethylformamide (10 mL). 2-(7-Azobenzotriazole)-N,N,N',N'-tetramethyluronium hexafluorophosphate (1.22 g, 3.21 mmol) and triethylamine (651 mg, 6.43 mmol) were added. After the mixture was reacted at room temperature for 30 minutes, compound **21a** (500 mg, 2.14 mmol) was added. After nitrogen displacement in a reaction flask at room temperature for 2 hours, the mixture was diluted with water (10 mL) and extracted with ethyl acetate (3 × 20 mL). The organic phase was washed with a saturated aqueous sodium chloride solution (3 × 20 mL), dried over anhydrous sodium sulfate, and filtered. The organic phases were combined and concentrated under reduced pressure to obtain compound **21b** (as a white solid, 412 mg, yield 58.7%).

LCMS m/z = 328.31 [M+1].

### Step 2

### N-(4-(4-amino-5-(3-fluoro-4-((4-methylpyrimidin-2-yl)oxy)phenyl)-7-methyl-7H-pyrrolo[2,3-d]pyrimidin-6-yl)-3-methylphenyl)-2-cyclopropylacrylamide compound 21

Compound **2d** (500 mg, 1.05mmol) and compound **21b** (412 mg, 1.26 mmol) were dissolved in N,N-dimethylformamide : water = 20 : 1 (6 mL). Potassium phosphate (668 mg, 3.15 mmol) and 1,1-bis(diphenylphosphino)ferrocene palladium dichloride (153 mg, 0.21 mmol) were added. After nitrogen displacement, the reaction flask was placed in an oil bath at 90°C for a reaction. After the reaction was complete, the reaction mixture was quenched with water (10 mL) and extracted with ethyl acetate (3 × 15 mL). The organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (ethyl acetate : methanol = 20 : 1) to obtain **compound 21** (as a white solid, 120 mg, yield 20.8%).

¹H NMR (400 MHz, DMSO-*d₆*) δ 9.94 (s, 1H), 8.50 (s, 1H), 8.47 (s, 1H), 7.94 (m, 2H), 7.71 - 7.62 (m, 2H), 7.33 (dd, *J* = 8.4, 4.4 Hz, 2H), 7.23 - 7.14 (m, 2H), 7.03 (dd, *J* = 8.4, 2.0 Hz, 1H), 5.63 (s, 1H), 5.28 (s, 1H), 3.50 (s, 3H), 2.40 (s, 3H), 1.94 (s, 3H), 1.75 (m, 1H), 0.82 - 0.73 (m, 2H), 0.60 - 0.51 (m, 2H).

LCMS m/z = 550.20 [M+1].

### Example 22

### N-(4-(4-amino-5-(3-fluoro-4-((4-methylpyrimidin-2-yl)oxy)phenyl)-7-methyl-7H-pyrrolo[2,3-d]pyrimidin-6-yl)-3-cyanophenyl)-2-cyclopropylacrylamide compound 22

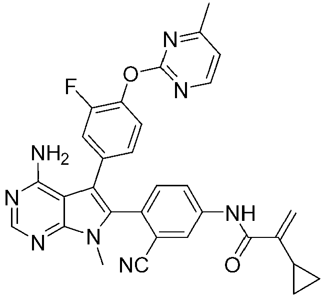

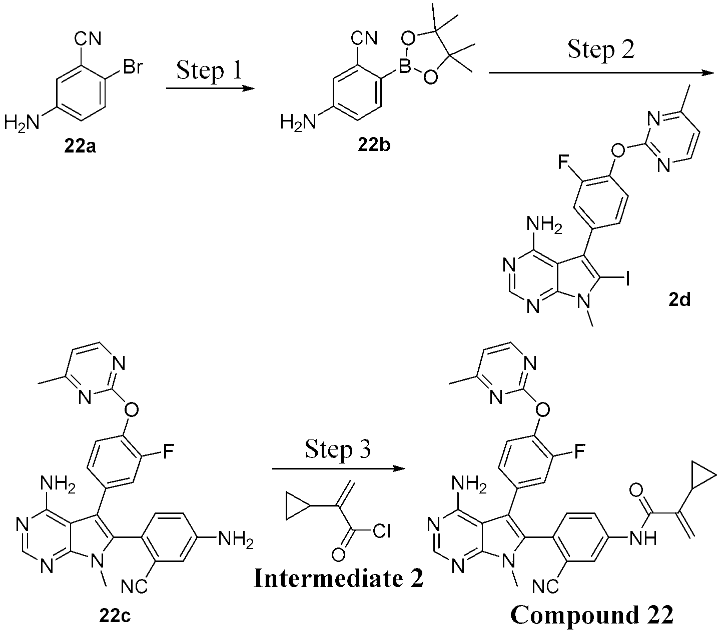

### Step 1

### 5-Amino-2-(4,4,5,5-tetramethyl-1,3,2-dioxaborolan-2-yl)benzonitrile 22b

Compound **22a** (3 g, 15.2 mmol) and bis(pinacolato)diboron (5.8 g, 22.8 mmol) were dissolved in 1,4-dioxane (60 mL). Potassium acetate (4.47 g, 45.6 mmol) and 1,1-bis(diphenylphosphino)ferrocene palladium dichloride (1.11 g, 1.52 mmol) were added. After nitrogen displacement, the reaction flask was placed in an oil bath at 95°C for a reaction. After 5 hours of reaction, the reaction solution was concentrated. The crude product was purified by column chromatography (petroleum ether : ethyl acetate = 5 : 1) to obtain compound **22b** (as a yellow solid, 3.36 g, yield 91%).

LCMS m/z = 245.10 [M+1].

### Step 2

### 5-Amino-2-(4-amino-5-(3-fluoro-4-((4-methylpyrimidin-2-yl)oxy)phenyl)-7-methyl-7H-pyrrolo[2,3-d]pyrimidin-6-yl)benzonitrile 22c

Compound **2d** (6.16 g, 12.95 mmol) and compound **22b** (3.16 g, 12.95 mmol) were dissolved in N,N-dimethylformamide : water = 20 : 1 (50 mL). Potassium phosphate (8.23 g, 38.8 mmol) and 1,1-bis(diphenylphosphino)ferrocene palladium dichloride (1.42 g, 1.94 mmol) were added. After nitrogen displacement, the reaction flask was placed in an oil bath at 90°C for a reaction. After the reaction was complete, the reaction mixture was quenched with water (50 mL) and extracted with ethyl acetate (3 × 50 mL). The organic phase was washed with a saturated aqueous sodium chloride solution (3 × 50 mL), dried over anhydrous sodium sulfate, and filtered. Organic phases were combined and concentrated under reduced pressure. The crude product was purified by column chromatography (ethyl acetate : methanol = 5 : 1) to obtain **compound 22c** (as a white solid, 1.44 g, yield 24%).

LCMS m/z = 467.20 [M+1].

### Step 3

### N-(4-(4-amino-5-(3-fluoro-4-((4-methylpyrimidin-2-yl)oxy)phenyl)-7-methyl-7H-pyrrolo[2,3-d]pyrimidin-6-yl)-3-cyanophenyl)-2-cyclopropylacrylamide compound 22

Compound **22c** (1.24 g, 2.66 mmol) and pyridine (2.14 mL, 26.6 mmol) were dissolved in dichloromethane (10 mL). The system was cooled to zero degree Celsius. **Intermediate 2** (520 mg, 3.99 mmol) was slowly added. After the addition was complete, the mixture was heated to room temperature and reacted. After the reaction was complete, the reaction mixture was quenched with water (10 mL) and extracted with dichloromethane (3 × 15 mL). The organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (ethyl acetate : methanol = 100 : 7) to obtain **compound 22** (as a white solid, 300 mg, yield 20%).

¹H NMR (400 MHz, DMSO-d6) δ 10.29 (s, 1H), 8.47 (d, J = 5.0 Hz, 1H), 8.26 (d, J = 2.5 Hz, 2H), 8.05 (dd, J = 8.6, 2.2 Hz, 1H), 7.67 (d, J = 8.6 Hz, 1H), 7.35 (t, J = 8.4 Hz, 1H), 7.21 - 7.09 (m, 2H), 7.04 (dd, J = 8.4, 2.0 Hz, 1H), 6.14 (s, 2H), 5.69 (s, 1H), 5.35 (s, 1H), 3.54 (s, 3H), 2.40 (s, 3H), 1.75 (m, 1H), 0.86 - 0.70 (m, 2H), 0.63 - 0.50 (m, 2H).

LCMS m/z = 561.20 [M+1].

### Example 23

### N-(6-(4-amino-5-(3-fluoro-4-((4-methylpyrimidin-2-yl)oxy)phenyl)-7-methyl-7H-pyrrolo[2,3-d]pyrimidin-6-yl)-5-chloropyridin-3-yl)-2-cyclopropylacrylamide compound 23

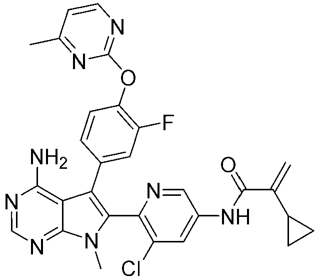

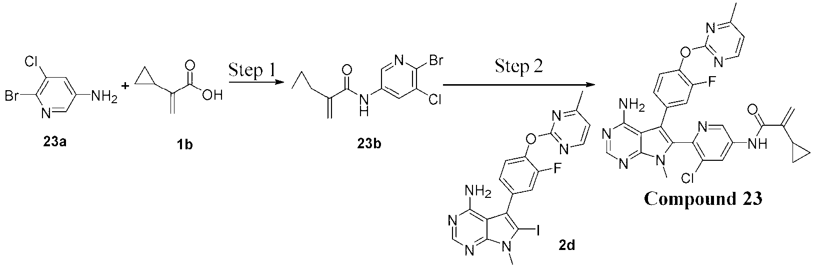

### Step 1

### N-(6-bromo-5-chloropyridin-3-yl)-2-cyclopropylacrylamide 23b

Compound **1b** (1.0 g, 8.9 mmol) was added to dichloromethane (27 mL). Oxalyl chloride (1.01 g, 8.0 mmol) and 1 drop of N,N-dimethylformamide were slowly dropwise added under ice bath condition. The mixture was reacted at room temperature until no gas was generated. Then, the mixture was slowly added to a solution of compound **23a** (1.85 g, 8.9 mmol) and triethylamine (9.0 g, 89 mmol) in dichloromethane (27 mL) under ice bath condition. The mixture was reacted at room temperature for 4 hours. Water (40 mL) was added, and layers were separated. The organic layer was washed with brine (40 mL), dried, and concentrated. The crude product was purified by column chromatography (petroleum ether : ethyl acetate = 6 : 1) to obtain compound **23b** (as a white solid, 1.36 g, yield 51%).

LCMS m/z = 302.90 [M+1].

### Step 2

### N-(6-(4-amino-5-(3-fluoro-4-((4-methylpyrimidin-2-yl)oxy)phenyl)-7-methyl-7H-pyrrolo[2,3-d]pyrimidin-6-yl)-5-chloropyridin-3-yl)-2-cyclopropylacrylamide compound 23

Compound **2d** (1.05 g, 2.2 mmol) and compound **23b** (1.0 g, 3.3 mmol) were dissolved in N,N-dimethylformamide : water = 20 : 1 (20 mL). Potassium phosphate (1.88 g, 8.86 mmol), 1,1-bis(diphenylphosphino)ferrocene palladium dichloride (243 mg, 0.33 mmol), and bis(pinacolato)diboron (845 mg, 3.3 mmol) were added. After nitrogen displacement, the reaction flask was placed in an oil bath at 90°C for a reaction. After 16 hours, the reaction mixture was quenched with water (40 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 23** (as a white solid, 20 mg, yield 1.6%).

¹H NMR (400 MHz, DMSO-*d₆*) δ 10.45 (s, 1H), 8.98 (d, *J* = 2.2 Hz, 1H), 8.51 - 8.44 (m, 4H), 7.35 (t, *J* = 8.4 Hz, 1H), 7.24 - 7.17 (m, 3H), 7.04 - 7.00 (m, 1H), 5.74 (s, 1H), 5.40 (s, 1H), 3.60 (s, 3H), 2.41 (s, 3H), 1.78 - 1.72 (m, 1H), 0.83 - 0.77 (m, 2H), 0.60 - 0.55 (m, 2H).

LCMS m/z = 571.20 [M+1].

### Example 24

### N-(5-(4-amino-5-(3-fluoro-4-((4-methylpyrimidin-2-yl)oxy)phenyl)-7-methyl-7H-pyrrolo[2,3-d]pyrimidin-6-yl)-6-chloropyridin-2-yl)-2-cyclopropylacrylamide compound 24

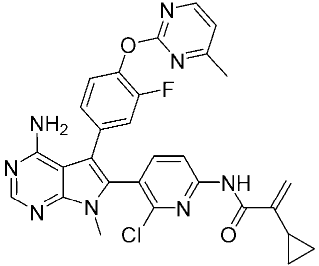

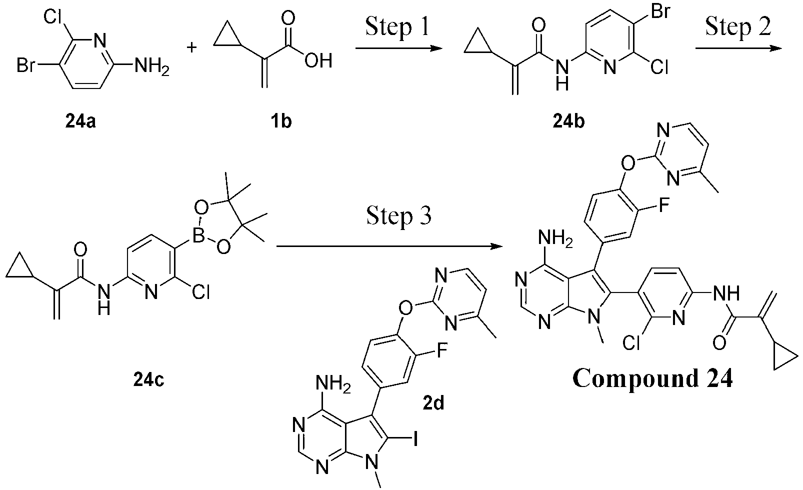

### Step 1

### N-(5-bromo-6-chloropyridin-2-yl)-2-cyclopropylacrylamide 24b

Compound **1b** (1.00 g, 8.90 mmol) was added to dichloromethane (27 mL). Oxalyl chloride (1.01 g, 8.00 mmol) and 1 drop of N,N-dimethylformamide were slowly dropwise added under ice bath condition. The mixture was reacted at room temperature until no gas was generated. Then, the mixture was slowly added to compound 24a (1.85 g, 8.90 mmol) and triethylamine (9.00 g, 89 mmol) in dichloromethane (27 mL) under ice bath condition. The mixture was reacted at room temperature for 4 hours. Water (40 mL) was added, and layers were separated. The organic layer was washed with brine (40 mL), dried, and concentrated. The crude product was purified by column chromatography (petroleum ether : ethyl acetate = 10 : 1) to obtain compound **24b** (as a white solid, 1.00 g, yield 37.0%).

LCMS m/z = 302.90 [M+1].

### Step 2

### N-(6-chloro-5-(4,4,5,5-tetramethyl-1,3,2-dioxaborolan-2-yl)pyridin-2-yl)-2-cyclopropylacrylamide 24c

Compound **24b** (1.00 g, 3.30 mmol) and bis(pinacolato)diboron (1.27 g, 5.00 mmol) were dissolved in 1,4-dioxane (15 mL). Potassium acetate (981 mg, 10.0 mmol) and 1,1-bis(diphenylphosphino)ferrocene palladium dichloride (244 mg, 0.33 mmol) were added. After nitrogen displacement, the reaction flask was placed in an oil bath at 95°C for a reaction. After 5 hours of reaction, the reaction solution was concentrated and extracted with water (40 mL) and ethyl acetate (3 × 15 mL). The organic layer was washed with water (40 mL), dried, and concentrated. The crude product was purified by column chromatography (petroleum ether : ethyl acetate = 6 : 1) to obtain compound **24c** (as a white solid, 1.0 g, yield 87%).

LCMS m/z = 349.10 [M+1].

### Step 3

### N-(5-(4-amino-5-(3-fluoro-4-((4-methylpyrimidin-2-yl)oxy)phenyl)-7-methyl-7H-pyrrolo[2,3-d]pyrimidin-6-yl)-6-chloropyridin-2-yl)-2-cyclopropylacrylamide compound 24

Compound **2d** (523 mg, 1.10 mmol) and compound **24c** (500 mg, 1.43 mmol) were dissolved in tetrahydrofuran : water = 10 : 1 (10 mL). Potassium carbonate (460 mg, 3.30 mmol) and XPhos Pd G4 (95 mg, 0.11 mmol) were added. After nitrogen displacement, the reaction flask was placed in an oil bath at 60°C. After 2 hours of reaction, the reaction mixture was quenched with water (10 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 24** (as a white solid, 50 mg, yield 9.6%).

¹H NMR (400 MHz, DMSO-*d₆*) δ 10.78 (s, 1H), 8.51 (s, 1H), 8.46 (d, *J =* 5.0 Hz, 1H), 8.21 (d, *J* = 8.4 Hz, 1H), 8.03 (d, *J* = 8.3 Hz, 1H), 7.38 (t, *J* = 8.3 Hz, 1H), 7.25 (dd, *J* = 11.3, 2.0 Hz, 1H), 7.19 (d, *J* = 5.0 Hz, 1H), 7.07 (dd, *J* = 8.3, 2.0 Hz, 1H), 7.02 (s, 2H), 5.82 (s, 1H), 5.36 (s, 1H), 3.61 (s, 3H), 2.40 (s, 3H), 1.83 - 1.75 (m, 1H), 0.79 - 0.74 (m, 2H), 0.57 - 0.51 (m, 2H).

LCMS m/z = 571.10 [M+1].

### Example 25

### N-(4-(4-amino-5-(3-fluoro-4-((4-methylpyrimidin-2-yl)oxy)phenyl)-7-methyl-7H-pyrrolo[2,3-d]pyrimidin-6-yl)-3-methoxyphenyl)-2-cyclopropylacrylamide compound 25

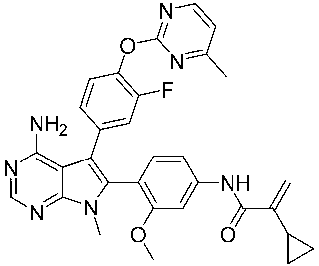

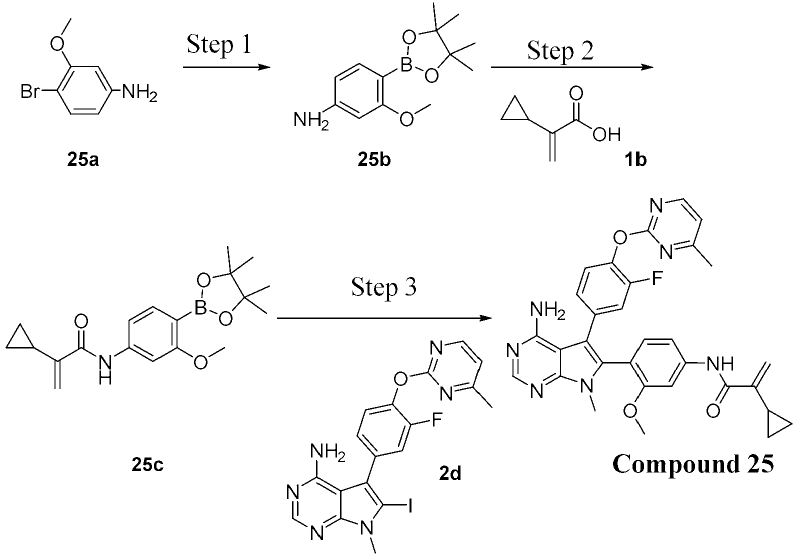

### Step 1

### 3-Methoxy-4-(4,4,5,5-tetramethyl-1,3,2-dioxaborolan-2-yl)aniline 25b

Compound **25a** (2.02 g, 10.0 mmol) and bis(pinacolato)diboron (3.81 g, 15.0 mmol) were added to triethylamine (40 mL). Potassium acetate (3.00 g, 30.0 mmol) and bis(triphenylphosphine)palladium dichloride (702 mg, 1.00 mmol) were added. After nitrogen displacement, the reaction flask was placed in an oil bath at 90°C. After 8 hours of reaction, the reaction solution was filtered, concentrated, and extracted with water (30 mL) and ethyl acetate (3 × 15 mL). The organic layer was washed with brine (40 mL), dried, and concentrated. The crude product was purified by column chromatography (petroleum ether : ethyl acetate = 2 : 1) to obtain compound **25b** (as a white solid, 1.0 g, yield 40%).

LCMS m/z = 250.10 [M+1].

### Step 2

### 2-Cyclopropyl-N-(3-methoxy-4-(4,4,5,5-tetramethyl-1,3,2-dioxaborolan-2-yl)phenyl)acrylamide 25c

Compound **1b** (584 mg, 5.20 mmol) was dissolved in N,N-dimethylformamide (20 mL). 2-(7-Azobenzotriazole)-N,N,N',N'-tetramethyluronium hexafluorophosphate (1.83 g, 4.80 mmol) and triethylamine (1.00 g, 10.0 mmol) were added. After reaction at room temperature for 30 minutes, compound **25b** (1.00 g, 4.0 mmol) was added. After nitrogen displacement in a reaction flask at room temperature for 2 hours, the reaction mixture was diluted with water (30 mL) and extracted with ethyl acetate (3 × 20 mL). The organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (petroleum ether : ethyl acetate = 6 : 1) to obtain compound 25c (as a white solid, 600 mg, yield 43.8%).

LCMS m/z = 344.20 [M+1].

### Step 3

### N-(4-(4-amino-5-(3-fluoro-4-((4-methylpyrimidin-2-yl)oxy)phenyl)-7-methyl-7H-pyrrolo[2,3-d]pyrimidin-6-yl)-3-methoxyphenyl)-2-cyclopropylacrylamide compound 25

Compound **2d** (200 mg, 0.42 mmol) and compound 25c (187 mg, 0.54 mmol) were dissolved in N,N-dimethylformamide : water = 20 : 1 (5 mL). Potassium phosphate (267 mg, 1.26 mmol) and 1,1-bis(diphenylphosphino)ferrocene palladium dichloride (31 mg, 0.042 mmol) were added. After nitrogen displacement, the reaction flask was placed in an oil bath at 90°C for a reaction. After 2 hours, the reaction mixture was quenched with water (10 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 25** (as a white solid, 80 mg, yield 33.7%).

¹H NMR (400 MHz, DMSO-*d₆*) δ 10.02 (s, 1H), 8.49 (s, 1H), 8.46 (d, *J* = 5.0 Hz, 1H), 7.65 (d, *J* = 2.0 Hz, 1H), 7.38 (dd, *J =* 8.4, 2.0 Hz, 1H), 7.33 (t, *J =* 8.4 Hz, 1H), 7.21 - 7.15 (m, 5H), 7.05 - 7.01 (m, 1H), 5.64 (s, 1H), 5.30 (s, 1H), 3.70 (s, 3H), 3.56 (s, 3H), 2.40 (s, 3H), 1.80 - 1.71 (m, 1H), 0.81 - 0.75 (m, 2H), 0.58 - 0.54 (m, 2H).

LCMS m/z = 566.20 [M+1].

### Example 26

### N-(5-(4-amino-5-(3-fluoro-4-((4-methylpyrimidin-2-yl)oxy)phenyl)-7-methyl-7H-pyrrolo[2,3-d]pyrimidin-6-yl)-4-chloropyridin-2-yl)-2-cyclopropylacrylamide compound 26

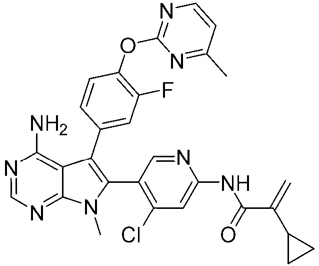

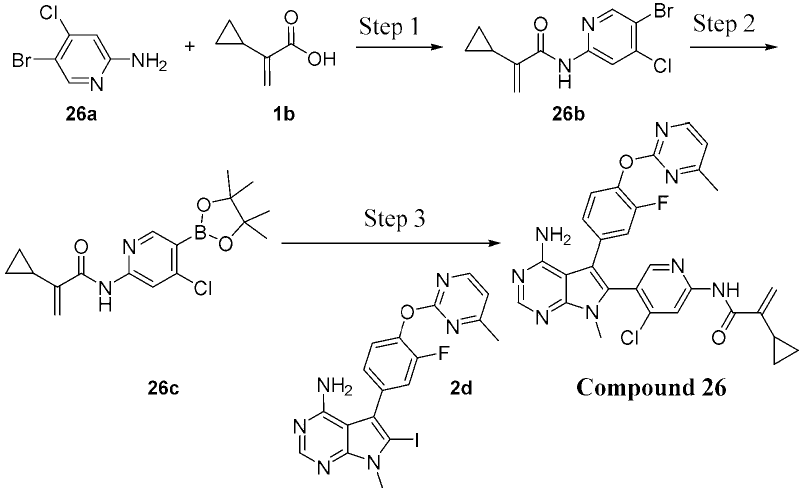

### Step 1

### N-(5-bromo-4-chloropyridin-2-yl)-2-cyclopropylacrylamide 26b

Compound **1b** (1.00 g, 8.90 mmol) was added to dichloromethane (27 mL). Oxalyl chloride (1.01 g, 8.00 mmol) and 1 drop of N,N-dimethylformamide were slowly dropwise added under ice bath condition. The mixture was reacted at room temperature until no gas was generated. Then, the mixture was slowly added to compound **26a** (1.85 g, 8.90 mmol) and triethylamine (9.00 g, 89 mmol) in dichloromethane (27 mL) under ice bath condition. The mixture was reacted at room temperature for 4 hours. Water (40 mL) was added for extraction, and layers were separated. The organic layer was washed with brine (40 mL), dried, and concentrated. The crude product was purified by column chromatography (petroleum ether : ethyl acetate = 10 : 1) to obtain compound 26b (as a white solid, 1.75 g, yield 65.5%).

LCMS m/z = 302.90 [M+1].

### Step 2

### N-(4-chloro-5-(4,4,5,5-tetramethyl-1,3,2-dioxaborolan-2-yl)pyridin-2-yl)-2-cyclopropylacrylamide 26c

Compound **26b** (1.00 g, 3.30 mmol) and bis(pinacolato)diboron (1.27 g, 5.00 mmol) were dissolved in 1,4-dioxane (15 mL). Potassium acetate (981 mg, 10.0 mmol) and 1,1-bis(diphenylphosphino)ferrocene palladium dichloride (244 mg, 0.33 mmol) were added. After nitrogen displacement, the reaction flask was placed in an oil bath at 95°C for a reaction. After 5 hours of reaction, the reaction solution was concentrated and extracted with water (40 mL) and ethyl acetate (3 × 15 mL). The organic layer was washed with water (40 mL), dried, and concentrated. The crude product was purified by column chromatography (petroleum ether : ethyl acetate = 6 : 1) to obtain compound **26c** (as a white solid, 440 mg, yield 38%).

LCMS m/z = 349.10 [M+1].

### Step 3

### N-(5-(4-amino-5-(3-fluoro-4-((4-methylpyrimidin-2-yl)oxy)phenyl)-7-methyl-7H-pyrrolo[2,3-d]pyrimidin-6-yl)-4-chloropyridin-2-yl)-2-cyclopropylacrylamide compound 26

Compound **2d** (470 mg, 1.10 mmol) and compound **26c** (500 mg, 1.43 mmol) were dissolved in tetrahydrofuran : water = 10 : 1 (10 mL). Potassium carbonate (415 mg, 3.00 mmol) and XPhos Pd G4 (86 mg, 0.10 mmol) were added. After nitrogen displacement, the reaction flask was placed in an oil bath at 60°C. After 2 hours of reaction, the reaction mixture was quenched with water (10 mL) and extracted with ethyl acetate (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1) to obtain **compound 26** (as a white solid, 30 mg, yield 5.3%).

¹H NMR (400 MHz, DMSO-*d₆*) δ 10.64 (s, 1H), 8.46 (d, J = 5.0 Hz, 1H), 8.41 (d, J = 4.4 Hz, 1H), 8.36 (d, J = 6.4 Hz, 1H), 8.24 (s, 1H), 7.35 (t, J = 8.4 Hz, 1H), 7.24 - 7.69 (m, 2H), 7.18 (d, J = 5.0 Hz, 1H), 7.08 - 7.04 (m, 2H), 5.82 (s, 1H), 5.37 (s, 1H), 3.53 (s, 3H), 2.40 (s, 3H), 1.82 - 1.75 (m, 1H), 0.81 - 0.75 (m, 2H), 0.57 - 0.53 (m, 2H).

LCMS m/z = 571.10 [M+1].

### Example 27

### N-(4-(4-amino-5-(3-fluoro-4-((4-methylpyrimidin-2-yl)oxy)phenyl)-7-methyl-7H-pyrrolo[2,3-d]pyrimidin-6-yl)-3-(trifluoromethyl)phenyl)-2-cyclopropylacrylamide compound 27

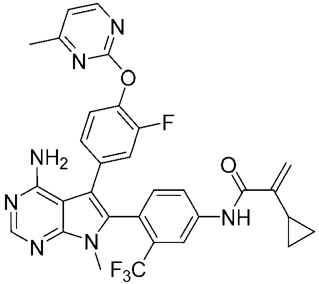

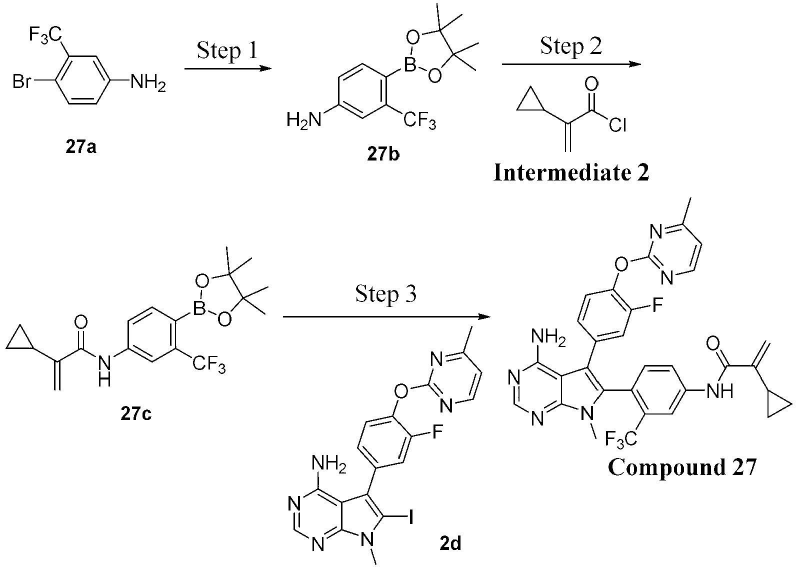

### Step 1

### 4-(4,4,5,5-Tetramethyl-1,3,2-dioxaborolan-2-yl)-3-(trifluoromethyl)aniline 27b

Compound **27a** (25 g, 87.1 mmol) and bis(pinacolato)diboron (33.2 g, 130 mmol) were added to triethylamine (40 mL). Potassium acetate (25.6 g, 261 mmol) and bis(triphenylphosphine)palladium dichloride (6.37 g, 8.71 mmol) were added. After nitrogen displacement, the reaction flask was placed in an oil bath at 90°C. After 8 hours, the reaction solution was filtered, and concentrated. Water (300 mL) and ethyl acetate (3 × 150 mL) were added, and layers were separated. The organic layer was washed with brine (400 mL), dried, and concentrated. The crude product was purified by column chromatography (petroleum ether : ethyl acetate = 2 : 1) to obtain compound **27b** (as a white solid, 12.5 g, yield 50%).

LCMS m/z = 288.10 [M+1].

### Step 2

### 2-Cyclopropyl-N-(4-(4,4,5,5-tetramethyl-1,3,2-dioxaborolan-2-yl)-3-(trifluoromethyl)phenyl)acrylamide 27c

Compound **27b** (5.6 g, 19.5 mmol) and pyridine (16 mL, 195 mmol) were dissolved in dichloromethane (100 mL). The system was cooled to zero degree Celsius. **Intermediate 2** (3.8 g, 29.2 mmol) was slowly added. The system was heated to room temperature and reacted. After the reaction was complete, the reaction mixture was quenched with water (100 mL) and extracted with dichloromethane (3 × 50 mL). The organic phase was washed with a saturated aqueous sodium chloride solution (3 × 50 mL), dried over anhydrous sodium sulfate, and filtered. Organic phases were combined and concentrated under reduced pressure. The crude product was purified by column chromatography (petroleum ether : ethyl acetate = 10 : 1) to obtain compound **27c** (as a yellow oil, 1.62 g, yield 22%).

LCMS m/z = 382.20 [M+1].

### Step 3

### N-(4-(4-amino-5-(3-fluoro-4-((4-methylpyrimidin-2-yl)oxy)phenyl)-7-methyl-7H-pyrrolo[2,3-d]pyrimidin-6-yl)-3-(trifluoromethyl)phenyl)-2-cyclopropylacrylamide compound 27

Compound **2d** (520 mg, 1.09 mmol) and compound **27c** (500 mg, 1.31 mmol) were dissolved in 1,4-dioxane : water = 2 : 1 (7.5 mL). Potassium carbonate (605 mg, 4.37 mmol) and XPhos Pd G4 (94 mg, 0.109 mmol) were added. After nitrogen displacement, the reaction flask was placed in an oil bath at 90°C for a reaction. After 2 hours of reaction, the reaction mixture was quenched with water (10 mL) and extracted with ethyl acetate (3 × 10 mL). After the extraction was complete, the organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (ethyl acetate : methanol = 30 : 1) to obtain **compound 27** (as a white solid, 60 mg, yield 9%).

¹H NMR (400 MHz, DMSO-*d₆*) δ 10.31 (s, 1H), 8.45 (d, J = 5.0 Hz, 1H), 8.28 (d, J = 2.1 Hz, 1H), 8.22 (s, 1H), 8.14 - 8.05 (m, 1H), 7.69 (d, J = 8.4 Hz, 1H), 7.31 (t, J = 8.4 Hz, 1H), 7.16 (d, J = 5.1 Hz, 1H), 7.12 (dd, J = 11.5, 2.0 Hz, 1H), 7.04 (dd, J = 8.3, 2.0 Hz, 1H), 6.08 (s, 2H), 5.71 (s, 1H), 5.34 (s, 1H), 2.45 (s, 3H), 2.39 (s, 3H), 1.76 (m, 1H), 0.86 - 0.74 (m, 2H), 0.62 - 0.51 (m, 2H).

LCMS m/z = 604.20 [M+1].

### Example 28

### N-(4-(4-amino-5-(6-fluoro-5-((4-methylpyrimidin-2-yl)oxy)pyridin-2-yl)-7-methyl-7H-pyrrolo[2,3-d]pyrimidin-6-yl)-3-chlorophenyl)-2-cyclopropylacrylamide compound 28

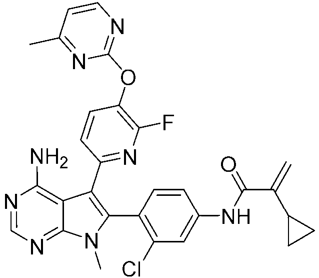

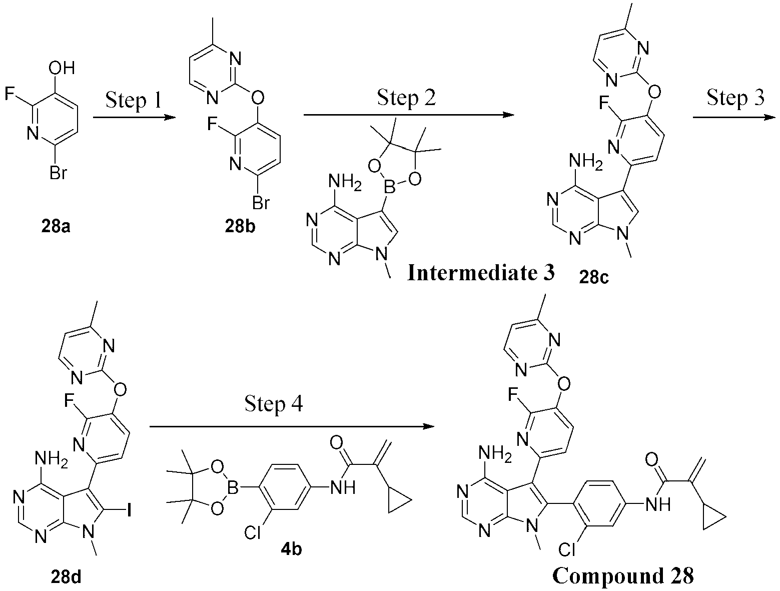

### Step 1

### 2-((6-Bromo-2-fluoropyridin-3-yl)oxy)-4-methylpyrimidine 28b

Compound **28a** (5 g, 26 mmol), 2-chloro-4-methylpyrimidine (3.35 g, 26 mmol), and potassium hydroxide (1.46 g, 26 mmol) were added to dimethyl sulfoxide (100 mL). The mixture was heated in an oil bath at 100°C and reacted for 4 hours. The mixture was cooled to room temperature and reacted. Water (100 mL) and ethyl acetate (3 × 100 mL) were added, and layers were separated. The organic layer was washed with brine (100 mL), dried, and concentrated. The crude product was purified by column chromatography (petroleum ether : ethyl acetate = 6 : 1) to obtain compound **28b** (as a white solid, 2 g, yield 25%).

LCMS m/z = 283.90 [M+1].

### Step 2

### 5-(6-Fluoro-5-((4-methylpyrimidin-2-yl)oxy)pyridin-2-yl)-7-methyl-7H-pyrrolo[2,3-d]pyrimidin-4-amine 28c

**Intermediate 3** (6.34 g, 17 mmol) and compound **28b** (3.2 g, 11.3 mmol) were dissolved in N,N-dimethylformamide : water = 5 : 1 (55 mL). Potassium phosphate (7.2 g, 33.9 mmol) and 1,1-bis(diphenylphosphino)ferrocene palladium dichloride (830 mg, 1.13 mmol) were added. After nitrogen displacement, the reaction flask was placed in an oil bath at 90°C for a reaction. After 4 hours of reaction, the reaction mixture was diluted with water (100 mL) and extracted with ethyl acetate (3 × 50 mL). The organic phase was washed with a saturated aqueous sodium chloride solution (3 × 50 mL), dried over anhydrous sodium sulfate, and filtered. Organic phases were combined and concentrated under reduced pressure. The crude product was purified by column chromatography (petroleum ether : ethyl acetate = 1 : 1) to obtain compound **28c** (as a yellow solid, 2.1 g, yield 41%).

LCMS m/z = 352.10 [M+1].

### Step 3

### 5-(6-Fluoro-5-((4-methylpyrimidin-2-yl)oxy)pyridin-2-yl)-6-iodo-7-methyl-7H-pyrrolo[2,3-d]pyrimidin-4-amine 28d

Compound **28c** (2.085 g, 4.6 mmol) was dissolved in dichloromethane (20 mL). Trifluoroacetic acid (1.1 mL, 13.8 mmol) was added. After cooling to 0°C, N-iodosuccinimide (1.56 g, 6.9 mmol) was added. After the reaction flask underwent nitrogen displacement, a reaction was carried out for 3 hours. The reaction system was quenched with an aqueous sodium sulfite solution and extracted with dichloromethane (3 × 10 mL). The organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (ethyl acetate : methanol = 8 : 1) to obtain compound **28d** (as a yellow solid, 600 mg, yield 27%).

LCMS m/z = 478.10 [M+1].

### Step 4

### N-(4-(4-amino-5-(6-fluoro-5-((4-methylpyrimidin-2-yl)oxy)pyridin-2-yl)-7-methyl-7H-pyrrolo[2,3-d]pyrimidin-6-yl)-3-chlorophenyl)-2-cyclopropylacrylamide compound 28

Compound **28d** (550 mg, 1.15 mmol) and compound **4b** (600 mg, 1.73 mmol) were dissolved in 1,4-dioxane : water = 10 : 1 (11 mL). Cesium carbonate (1.13 g, 3.46 mmol) and 1,1-bis(diphenylphosphino)ferrocene palladium dichloridedichloromethane complex (95 mg, 0.115 mmol) were added. After nitrogen displacement, the reaction flask was placed in an oil bath at 90°C for a reaction. After 2 hours of reaction, the reaction mixture was quenched with water (5 mL) and extracted with ethyl acetate (3 × 10 mL). After the extraction was complete, the organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (dichloromethane : methanol = 30 : 1), followed by purification by reversed-phase column chromatography to **compound 28** (as a white solid, 110 mg, yield 17%).

¹H NMR (400 MHz, DMSO-*d₆*) δ 10.26 (s, 1H), 8.49 (d, J = 4.9 Hz, 1H), 8.20 (s, 2H), 7.82 (m, 3H), 7.52 (d, J = 8.5 Hz, 2H), 7.22 (d, J = 5.0 Hz, 1H), 6.80 (d, J = 8.3 Hz, 1H), 5.69 (s, 1H), 5.35 (s, 1H), 3.45 (s, 3H), 2.42 (s, 3H), 1.77 (s, 1H), 0.80 (d, J = 8.1 Hz, 2H), 0.57 (d, J = 5.5 Hz, 2H).

LCMS m/z = 571.20 [M+1].

### Example 29

### N-(4-(4-amino-5-(3-fluoro-4-((4-methylpyrimidin-2-yl)oxy)phenyl)-7-methyl-7H-pyrrolo[2,3-d]pyrimidin-6-yl)-3-(difluoromethyl)phenyl)-2-cyclopropylacrylamide compound 29

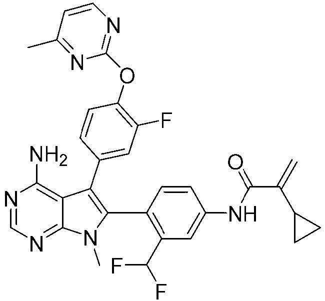

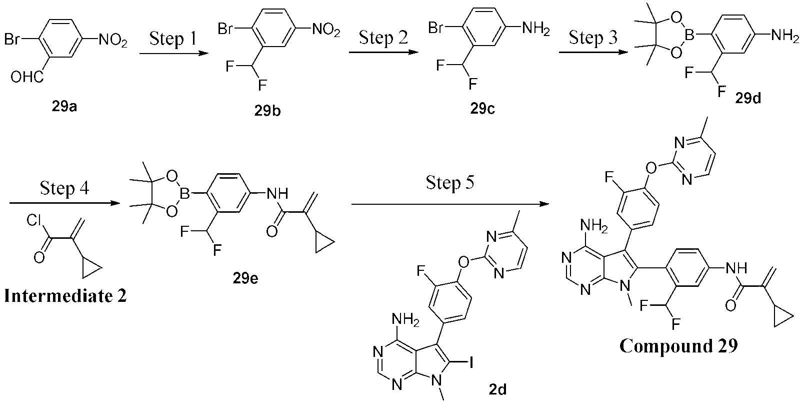

### Step 1

### 1-Bromo-2-(difluoromethyl)-4-nitrobenzene 29b

2-Bromo-5-nitrobenzaldehyde compound **29a** (5.0 g, 21.7 mmol) was added to dichloromethane (100 mL). Diethylaminosulfur trifluoride (DAST) (17.2 mL, 130 mmol) was slowly dropwise added at 0°C. The mixture was transferred to room temperature and reacted for 3 hours. The mixture was quenched with water (150 mL) and extracted with dichloromethane (200 mL × 2). The organic layer was washed with brine (200 mL), dried over anhydrous sodium sulfate, and concentrated to obtain crude compound **29b** (as a yellow liquid), which was directly subjected to the next reaction without purification.

LCMS m/z = 254.01 [M+2].

### Step 2

### 4-Bromo-3-(difluoromethyl)aniline 29c

Compound **29b** (5.6 g, 22.2 mmol) was dissolved in tetrahydrofuran : ethanol=1 : 1 (140 mL). Ammonium chloride (12.0 g, 222 mmol) and zinc powder (14.5 g, 222 mmol) were added. After the reaction flask underwent nitrogen displacement, a reaction was carried out at room temperature for 4 hours. After the reaction was complete, the reaction system was filtered. The reaction solution was concentrated. The crude product was purified by column chromatography (petroleum ether : ethyl acetate = 5 : 1) to obtain compound **29c** (as a brown solid, 1.6 g, yield 32.7%).

LCMS m/z = 222.90 [M+2].

### Step 3

### 3-(Difluoromethyl)-4-(4,4,5,5-tetramethyl-1,3,2-dioxaborolan-2-yl)aniline 29d

Compound **29c** (1.4 g, 6.3 mmol) was dissolved in triethylamine (30 mL). Bis(pinacolato)diboron (2.4 g, 9.5 mmol), bis(triphenylphosphine)palladium dichloride (892 mg, 1.2 mmol), and potassium acetate (1.87 g, 19 mmol) were added. After nitrogen displacement, the reaction flask was placed in an oil bath at 90°C for a reaction. After 2 hours of reaction, the reaction mixture was quenched with water (10 mL) and extracted with ethyl acetate (3 × 10 mL). The organic phase was washed with a saturated aqueous sodium chloride solution (3 × 10 mL), dried over anhydrous sodium sulfate, and filtered. The organic phases were combined and concentrated under reduced pressure to obtain compound **29d** (as a brown liquid, 1.9 g, yield 90%).

LCMS m/z = 270.91 [M+1].

### Step 4

### 2-Cyclopropyl-N-(3-(difluoromethyl)-4-(4,4,5,5-tetramethyl-1,3,2-dioxaborolan-2-yl)phenyl)acrylamide 29e

Compound **29d** (1.4 g, 5.2 mmol) and pyridine (4.2 mL, 52 mmol) were dissolved in dichloromethane (25 mL). The system was cooled to zero degree Celsius. **Intermediate 2** (1.015 g, 8.8 mmol) was slowly added. The system was heated to room temperature and reacted. After the reaction was complete, the reaction mixture was quenched with water (30 mL) and extracted with dichloromethane (3 × 20 mL). After the extraction was complete, the organic phase was washed with a saturated aqueous sodium chloride solution (3 × 30 mL), dried over anhydrous sodium sulfate, and filtered. Organic phases were combined and concentrated under reduced pressure. The crude product was purified by column chromatography (petroleum ether : ethyl acetate = 10 : 1) to obtain compound **29e** (as a yellow solid, 400 mg, yield 20%).

LCMS m/z = 384.20 [M+1].

### Step 5

### N-(4-(4-amino-5-(3-fluoro-4-((4-methylpyrimidin-2-yl)oxy)phenyl)-7-methyl-7H-pyrrolo[2,3-d]pyrimidin-6-yl)-3-(difluoromethyl)phenyl)-2-cyclopropylacrylamide compound 29

Compound **2d** (414 mg, 0.87 mmol) and compound **29e** (400 mg, 1.0 mmol) were dissolved in 1,4-dioxane : water = 2 : 1 (5 mL). Potassium carbonate (480 mg, 3.48 mmol) and XPhos Pd G4 (75 mg, 0.08 mmol) were added. After nitrogen displacement, the reaction flask was placed in an oil bath at 100°C for a reaction. After the reaction was complete, the reaction mixture was quenched with water (8 mL) and extracted with ethyl acetate (3 × 10 mL). After the extraction was complete, the organic phase was washed with a saturated aqueous sodium chloride solution (3 × 10 mL), dried over anhydrous sodium sulfate, and filtered. Organic phases were combined and concentrated under reduced pressure. The crude product was purified by column chromatography (ethyl acetate : methanol = 30 : 1) to obtain **compound 29** (as a white solid, 20 mg, yield 3.9%).

¹H NMR (400 MHz, Chloroform-d) δ 8.38 (s, 1H), 8.33 (d, *J=* 4.0 Hz, 1H), 8.24 (s, 1H), 8.07 (d, *J* = 8.4 Hz, 1H), 7.78 (d, *J =* 2.4 Hz, 1H), 7.38 (d, *J =* 8.4 Hz, 1H), 7.22 (d, *J =* 8.4 Hz, 1H), 7.02 (d, *J* = 8.0 Hz, 2H), 6.93 (d, *J* = 4.0 Hz, 1H), 6.29 (s, 1H), 6.16 (d, *J* = 2.4 Hz, 1H), 5.71 (s, 2H), 5.44 (d, *J* = 1.2 Hz, 1H), 3.56 (s, 3H), 2.48 (s, 3H), 1.66 (d, *J* = 7.6 Hz, 1H), 1.01 - 0.93 (m, 2H), 0.72 - 0.63 (m, 2H).

LCMS m/z = 586.20 [M+1].

### Example 30

### N-(4-(4-amino-5-(3-fluoro-4-((1-methyl-2-oxo-1,2-dihydropyridin-3-yl)oxy)phenyl)-7-methyl-7H-pyrrolo[2,3-d]pyrimidin-6-yl)phenyl]-2-cyclopropylacrylamide compound 30

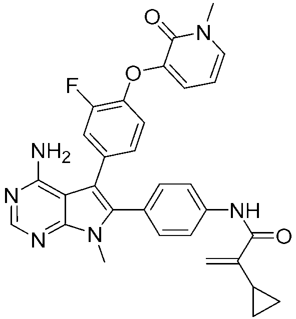

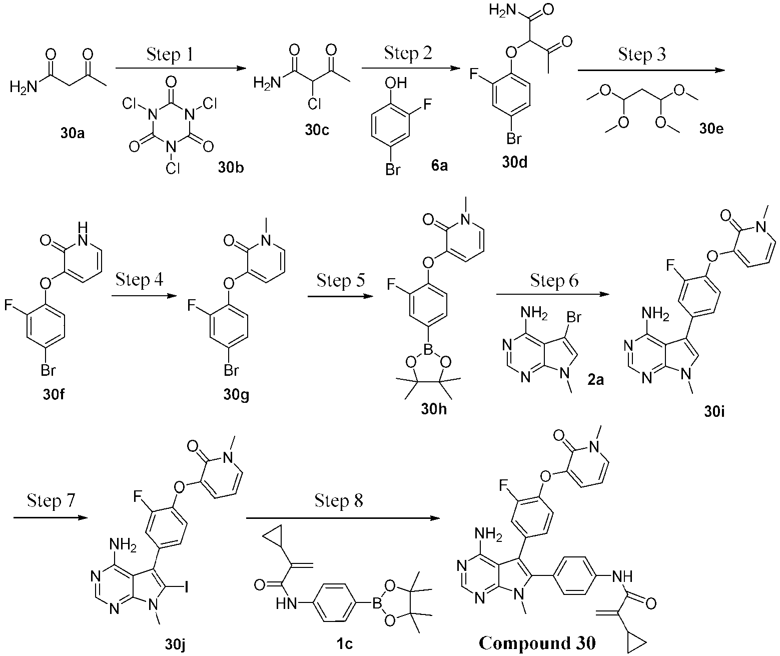

### Step 1:

### 2-Chloro-3-oxobutanamide 30c

At 0°C, trichloroisocyanuric acid compound **30b** (2.33 g, 10.3 mmol) was added to a solution of acetoacetate amide compound **30a** (3.03 g, 30 mmol), sodium bisulfate (1.02 g, 7.5 mmol), and tetrahydrofuran (70 mL) and stirred at 0°C for 1 hour. The mixture was filtered and concentrated under reduced pressure to obtain 2-chloro-3-oxobutanamide compound **30c** (as a white solid, 4.2 g, yield 99%).

LCMS m/z = 136.01 [M+1].

### Step 2:

### 2-(4-Bromo-2-fluorophenoxy)-3-oxobutanamide 30d

Under nitrogen, 2-chloro-3-oxobutanamide compound **30c** (1 g, 7.4 mmol), 4-bromo-2-fluorophenol compound **6a** (1.42 g, 7.4 mmol), and triethylamine (1.2 mL, 8.14 mmol) were added to N,N-dimethylformamide (20 mL) and stirred at 80°C for 6 hours. The reaction mixture was cooled to room temperature. Ethyl acetate (80 mL) and a saturated aqueous sodium chloride solution (30 mL) were added. After extraction, the organic phase was washed twice with an aqueous hydrochloric acid solution (20 mL, 1 mol/L) and once with a saturated sodium chloride solution (20 mL). The organic phases were combined, dried over anhydrous sodium sulfate, and concentrated. The crude product was purified by column chromatography to obtain compound **30d** (as a pink solid, 1.254 g, yield 58%).

LCMS m/z = 289.97 [M+1].

### Step 3:

### 3-(4-Bromo-2-fluorophenoxy)pyridin-2 (1H)-one 30f

In a nitrogen atmosphere, 2-(4-bromo-2-fluorophenoxy)-3-oxobutanamide **30d** (3.654 g, 16.024 mmol) and 1,1,3,3-tetramethoxypropane **30e** (3.955 g, 24.09 mmol) were added to a solution of 25% hydrogen bromide in acetic acid (50 mL) and stirred at 100°C for 2 hours. Ethyl acetate (150 mL) and a saturated aqueous sodium chloride solution (50 mL) were added to the reaction mixture. After extraction, the organic phase was washed twice with a saturated aqueous sodium bicarbonate solution (30 mL) and once with a saturated sodium chloride solution (50 mL). The organic phases were combined, then dried over anhydrous sodium sulfate, and concentrated, followed by purification by column chromatography to obtain compound **30f** (as a white solid, 3.915 g, yield 86%).

LCMS m/z = 283.96 [M+1].

### Step 4:

### 3-(4-Bromo-2-fluorophenoxy)-1-methylpyridin-2(1H)-one 30g

In a round-bottomed flask, compound **30f** (530 mg, 1.76 mmol) was dissolved in acetone (18 mL). Subsequently, potassium carbonate (980 mg, 7.04 mmol) and iodomethane (380 mg, 2.64 mmol) were added to the system and reacted at room temperature for 12 hours. After filtration, concentration under reduced pressure was carried out to obtain compound **30g** (as a gray solid, 530 mg, yield 99%).

LCMS m/z = 297.98 [M+1].

### Step 5:

### 3-(2-Fluoro-4-(4,4,5,5-tetramethyl-1,3,2-dioxaborolan-2-yl)phenoxy)-1-methylpyridin-2(1H)-one 30h

Compound **30g** (530 mg, 1.76 mmol), bis(pinacolato)diboron (670 mg, 2.64 mmol), and potassium acetate (520 mg, 5.28mmol) were dissolved in 1,4-dioxane (10 mL). 1,1-Bis(diphenylphosphino)ferrocene palladium dichloride (130 mg, 0.176 mmol) was then added. After nitrogen displacement, the reaction flask was placed in an oil bath at 90°C. After 3 hours of reaction, the reaction mixture was quenched with water (20 mL), and extracted with ethyl acetate (3×20 mL). The organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (petroleum ether : ethyl acetate = 1 : 1) to obtain compound **30h** (as a yellow oil, 160 mg, yield 26%).

LCMS m/z = 346.18 [M+1].

### Step 6:

### 3-(4-(4-Amino-7-methyl-7H-pyrrolo[2,3-d]pyrimidin-5-yl)-2-fluorophenoxy)-1-methylpyridin-2(1H)-one 30i

Compound **30h** (680 mg, 1.97 mmol), compound **2a** (450 mg, 1.97 mmol), and potassium phosphate (1.25 g, 5.9 mmol) were dissolved in N,N-dimethylformamide : water = 20 : 1 (11 mL). 1,1-Bis(diphenylphosphino)ferrocene palladium dichloride (150 mg, 0.197 mmol) was added. After nitrogen displacement, the reaction flask was placed in an oil bath at 90°C for a reaction. After 3 hours of reaction, the reaction mixture was quenched with water (20 mL) and extracted with ethyl acetate (3 × 20 mL). After the extraction was complete, the organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (dichloromethane : methanol = 20 : 1) to obtain compound **30i** (as a gray solid, 400 mg, yield 55%).

LCMS m/z = 366.13 [M+1].

### Step 7:

### 3-(4-(4-Amino-6-iodo-7-methyl-7H-pyrrolo[2,3-d]pyrimidin-5-yl)-2-fluorophenoxy)-1-methylpyridin-2 (1H)-one 30j

Compound **30i** (400 mg, 1.069 mmol) was dissolved in dichloromethane (12 mL). Trifluoroacetic acid (375 mg, 3.287 mmol) was added to the system. After the system was cooled to 0°C, N-iodosuccinimide (372 mg, 1.64 mmol) was slowly added. The system was heated to room temperature and reacted for 2 hours. The reaction mixture was quenched with a saturated aqueous sodium sulfite solution (20 mL). The system was extracted with dichloromethane (3 × 20 mL). After the extraction was complete, the organic phase was washed with a saturated aqueous sodium chloride solution (3 × 20 mL), dried over anhydrous sodium sulfate, and filtered. Organic phases were combined and concentrated under reduced pressure. The crude product was purified by column chromatography (dichloromethane : methanol = 20 : 1) to obtain compound **30j** (as a brown solid, 100 mg, yield 24%).

LCMS m/z = 492.10 [M+1].

### Step 8:

### N-(4-(4-amino-5-(3-fluoro-4-((1-methyl-2-oxo-1,2-dihydropyridin-3-yl)oxy)phenyl)-7-methyl-7H-pyrrolo[2,3-d]pyrimidin-6-yl)phenyl]-2-cyclopropylacrylamide compound 30

Compound **30j** (100 mg, 0.2 mmol) and compound **1c** (64 mg, 0.2 mmol) were dissolved in N,N-dimethylformamide : water = 20 : 1 (5.5 mL). Potassium phosphate (130 mg, 0.6 mmol) and 1,1-bis(diphenylphosphino)ferrocene palladium dichloride (15 mg, 0.02 mmol) were added. After nitrogen displacement, the reaction flask was placed in an oil bath at 90°C for a reaction. After 3 hours of reaction, the reaction mixture was quenched with water (10 mL) and extracted with ethyl acetate (3 × 15 mL). After the extraction was complete, the organic phase was washed with a saturated aqueous sodium chloride solution (3 × 15 mL), dried over anhydrous sodium sulfate, and filtered. Organic phases were combined and concentrated under reduced pressure. The crude product was purified by column chromatography (dichloromethane : methanol = 10 : 1) to obtain a crude product. The crude product was purified by reversed-phase column chromatography to obtain **compound 30** (as a white solid, 30 mg, yield 27%).

¹H NMR (400 MHz, DMSO-d6) δ 9.96 (s, 1H), 8.19 (s, 1H), 7.73 (d, J = 8.3 Hz, 2H), 7.58 (dd, J = 6.8, 1.8 Hz, 1H), 7.28 (d, J = 8.4 Hz, 2H), 7.21 (dd, J = 7.4, 1.8 Hz, 1H), 7.13 (dd, J = 11.9, 2.0 Hz, 1H), 7.04 - 6.81 (m, 2H), 6.19 (t, J = 7.1 Hz, 1H), 6.15 - 5.75 (s, 2H), 5.64 (s, 1H), 5.27 (s, 1H), 3.58 (s, 3H), 3.49 (s, 3H), 1.75 (dq, J = 8.5, 5.2, 4.4 Hz, 1H), 0.82 - 0.73 (m, 2H), 0.59 - 0.51 (m, 2H).

LCMS m/z = 551.20 [M+1].

### Biological Test

### 1. Research on FGFR1-4 enzymology

The inhibitory effects of compounds on the enzymatic activities of FGFR1, 2, 3, and 4 were detected by using HTRF method (PerkinElmer, 62TK0PEJ). 2× Enzyme and 2× substrate /ATP mixed solutions were prepared using a buffer solution. The final concentrations of FGFR1 enzyme (Cama, 08-133), the substrate (TK), and ATP were 0.10 nM, 1 µM, and 40 µM, respectively; the final concentrations of FGFR2 (Carna, 08-134), the substrate (TK), and ATP were 0.08 nM, 1 µM, and 20 µM, respectively; the final concentrations of FGFR3 (Cama, 08-135), the substrate (TK), and ATP were 0.30 nM, 1 µM, and 50 µM, respectively; and the final concentrations of FGFR4 (Carna, 08-136), the substrate (TK), and ATP were 10 nM, 1 µM, and 50 µM, respectively. The compound stock solution was diluted to 100× working concentration using DMSO. 50 nL/well of the compound was transferred to a 384-well plate using Echo, with 2 replicate wells per concentration. 2.5 µL/well of 2× kinase solution was added to reaction wells, mixed and incubated at 25°C for 10 min. 2.5 µL/well of 2× substrate/ATP solution was added to reaction wells and incubated at 25°C for 50 min. 2× Streptavidin-XL665/Cryptate-antibody mixed solution was prepared using a detection buffer solution (containing EDTA). 5 µL/well of a kinase detection reagent was added to the reaction wells and incubated at 25°C for 60 min. Fluorescence signals at 620 nm (Cryptate) and 665 nm (XL665) were read by a multifunctional microplate reader. IC₅₀ (median inhibitory concentration) of the compound was calculated using the GraphPad nonlinear fitting formula, Y=Bottom + (Top-Bottom)/(1+10^((LogIC50-X) ×HillSlope)), in which X: the logarithmic value of the concentration of the compound; and Y: the inhibition rate of the compound (% inhibition). The results are as below:

| **Inhibitory activity of test substances on FGFR1-4 kinases** | | | | |
|---|---|---|---|---|
| **Test substance** | FGFR1 IC₅₀ (nM) | FGFR2 IC₅₀ (nM) | FGFR3 IC₅₀ (nM) | FGFR4 IC₅₀ (nM) |
| **Comparative Example** | 553.2 | 8.4 | 612.4 | > 1000 |
| **Compound 1** | > 1000 | 23.8 | > 1000 | > 1000 |
| **Compound 2** | 407.4 | 12.0 | 149.1 | > 1000 |
| **Compound 4** | 164.9 | 1.7 | 64.2 | > 1000 |
| **Compound 5** | 550.1 | 7.3 | 246.2 | > 1000 |
| **Compound 9** | > 1000 | 65.5 | > 1000 | > 1000 |

| | | | | |
|---|---|---|---|---|
| Note: The comparative example was **compound 343** from the patent WO 2020231990 A1 and was obtained according to a method for preparing **compound 343.** | | | | |

The results show that compared with FGFR1 and FGFR4, the compound of the present invention has significant inhibitory activity and high selectivity for FGFR2 and/or FGFR3.

### 2. Research on FGFR2 and FGFR3 mutant enzymology

The inhibitory effects of the compounds on the enzymatic activities of FGFR2 and FGFR3 mutants by using HTRF method (PerkinElmer, 62TK0PEJ). 2× Enzyme and 2× substrate /ATP mixed solutions were prepared using a buffer solution. The specific reaction system is as shown in the following table.

| **Mutant kinase** | **Working concentration of kinase (nM)** | **Working concentration of substrate (µM)** | **Working concentration of ATP (µM)** |
|---|---|---|---|
| FGFR2 V564I | 0.03 | 1 | 20 |
| FGFR2 V564F | 0.02 | 1 | 15 |
| FGFR2 N549H | 0.02 | 1 | 9 |
| FGFR3 K650M | 0.10 | 1 | 12 |
| FGFR3 V555M | 0.55 | 1 | 20 |
| FGFR2 V564I | 0.03 | 1 | 20 |

The compound stock solution was diluted to 100× working concentration using DMSO. 50 nL/well of the compound was transferred to a 384-well plate using Echo, with 2 replicate wells per concentration. 2.5 µL/well of 2× kinase solution was added to reaction wells, mixed and incubated at 25°C for 10 min. 2.5 µL/well of 2× substrate/ATP solution was added to reaction wells and incubated at 25°C for 50 min. 2× Streptavidin-XL665/Cryptate-antibody mixed solution was prepared using a detection buffer solution (containing EDTA). 5 µL/well of a kinase detection reagent was added to the reaction wells and incubated at 25°C for 60 min. Fluorescence signals at 620 nm (Cryptate) and 665 nm (XL665) were read by a multifunctional microplate reader. According to the test results, the inhibition rate was calculated. Inhibition rate (% Inhibition) = (signal negative control - signal compound) / (signal negative control - signal positive control) * 100%. IC50 (median inhibitory concentration) of the compound was calculated using the GraphPad nonlinear fitting formula, Y = Bottom + (Top-Bottom)/(1+10^((LogIC50-X) ×HillSlope)), in which X: the logarithmic value of the concentration of the compound; and Y: the inhibition rate of the compound (% inhibition). The results are as below:

| **Mutant kinase** | **Comparative Example** | **Compound 4** |
|---|---|---|
| FGFR2 V564I IC₅₀ (nM) | 104.2 | 15.0 |
| FGFR2 V564F IC₅₀ (nM) | 0.9 | 0.5 |
| FGFR2 N549H IC₅₀ (nM) | 16.7 | 5.2 |
| FGFR3 K650M IC₅₀ (nM) | > 1000 | 270.7 |
| FGFR3 V555M IC₅₀ (nM) | 773.9 | 120.1 |

| | | |
|---|---|---|
| Note: The comparative example was **compound 343** from the patent WO 2020231990 A1 and was obtained according to a method for preparing **compound 343.** | | |

The results show that compared with the control compound, the compounds of the present invention have more significant inhibitory activity on different FGFR2 and FGFR3 mutants. This indicates that the compounds of the present invention can overcome acquired drug resistance.

### 3. Pharmacokinetic study on mouse brain tissue

An appropriate amount of the test substance was weighed and formulated into a 1 mg/mL transparent and clear solution using 10% DMSO + 5% HS-15 + 85% Saline for intragastric administration. 24 healthy male ICR mice (Charles River, SPF grade) were taken, with 3 animals per time point. Blood samples were collected at 15 min, 0.5, 1, 2, 4, 6, 8, and 24 h after intragastric administration of the test substance (10 mg/kg). The blood sample was anticoagulated with EDTA-K2 and then centrifuged at 6000 g at 4°C for 5 min to separate plasma. The plasma was stored at - 70°C for testing. After blood collection, the animal was sacrificed to collect cerebrospinal fluid, which was stored at -70°C for testing. The brain tissue was rinsed with ice-cold normal saline to remove the residual blood, blotted dry with absorbent paper, and then stored at -70°C for testing. The drug concentration of the specified compound in the plasma/cerebrospinal fluid/brain tissue was determined using LC-MS/MS method (with Gliclazide as an internal standard). The main pharmacokinetic parameters were calculated using a Winnolin 8.3 noncompartmental model. The results are as below:

| **Plasma/cerebrospinal fluid/brain tissue concentration data table in ICR mice after intragastric administration of test substance** | | | | | |
|---|---|---|---|---|---|
| **Test substance** | Site | t_{1/2} (h) | Tₘₐₓ (h) | Cₘₐₓ (ng/mL or ng/g) | AUC₀₋ₜ (h*ng/mL or h*ng/g) |
| **Comparati ve Example** | Plasma | 1.3 | 0.3 | 6097 | 9914 |
| | Cerebrospinal fluid | / | 0.5 | 9.5 | 4.9 |
| | Brain tissue | / | 0.5 | 38.8 | 48 |
| **Compound 1** | Plasma | 3.1 | 1 | 6240 | 50857 |
| | Cerebrospinal fluid | 6.3 | 0.5 | 44.5 | 59 |
| | Brain tissue | 3 | 1 | 116 | 524 |
| **Compound 4** | Plasma | 1.8 | 0.5 | 5010 | 21966 |
| | Cerebrospinal fluid | / | 1 | 6.3 | 9 |
| | Brain tissue | 1.7 | 1 | 97.3 | 277 |

| | | | | | |
|---|---|---|---|---|---|
| Note: The comparative example was **compound 343** from the patent WO 2020231990 A1 and was obtained according to a method for preparing **compound 343.** | | | | | |

The results show that compared with the control compound, the compounds of the present invention have superior pharmacokinetic characteristics and superior blood-brain barrier penetration ability.

### 4. Pharmacokinetic study on in rats in vivo

An appropriate amount of the test substance was weighed and formulated into a 0.5 mg/mL transparent and clear solution using 10% DMSO + 5% HS-15 + 85% Saline for intravenous administration. The test substance was formulated into a 1 mg/mL uniform suspension using 10% DMSO + 5% HS-15 + 85% Saline for intragastric administration. 6 healthy male SD rats (Charles River, SPF grade) were taken, with 3 animals assigned to the intravenous group and three to the intragastric group, respectively. With 3 animals per time point, blood samples were collected via the jugular vein. Blood samples were collected at 5 min, 15 min, 0.5, 1, 2, 4, 8, and 24 h after intravenous administration of the test substance (1 mg/kg), and blood samples were collected at 15 min, 0.5, 1, 2, 4, 6, 8, and 24 h after intragastric administration of the test substance (5 mg/kg). After anticoagulation with EDTA-K2, the blood samples were centrifuged at 6000 g at 4°C for 5 min to separate plasma, and all plasma samples were stored at -70°C for testing. The drug concentration of the test substance in plasma was determined by LC-MS/MS method (with gliclazide as an internal standard), and the main pharmacokinetic parameters were calculated by Winnolin 8.3 non-compartment model. The intragastric administration results are as follows:

| **Plasma drug concentration and PK parameters in SD rats after intragastric administration of 5 mg/kg of test substance** | | | | |
|---|---|---|---|---|
| **Test substance** | t_{1/2} (h) | Cₘₐₓ (ng/mL) | AUC₀₋ₜ (h*ng/mL) | F (%) |
| **Comparative Example** | 1.1 | 136 | 228 | 16.3 |
| **Compound 1** | 1.9 | 938 | 3193 | 90.8 |
| **Compound 4** | 0.9 | 273 | 787 | 24.2 |

| | | | | |
|---|---|---|---|---|
| Note: The comparative example was **compound 343** from the patent WO 2020231990 A1 and was obtained according to a method for preparing **compound 343.** | | | | |

The results show that compared with the control compound, the compounds of the present invention have superior pharmacokinetic characteristics.

### 5. Pharmacokinetic study on dogs in vivo

An appropriate amount of the test substance was weighed and formulated into a 0.5 mg/mL transparent and clear solution using 10% DMSO + 5% HS-15 + 85% Saline for intravenous administration. The test substance was formulated into a 0.6 mg/mL uniform suspension using 10% DMSO + 5% HS-15 + 85% Saline for intragastric administration. 6 healthy male Beagle dogs (Charles River, SPF grade) were taken, with 3 animals assigned to the intravenous group and three to the intragastric group, respectively. With 3 animals per time point, blood samples were collected via the forelimb vein. Blood samples were collected at 5 min, 15 min, 0.5, 1, 2, 4, 8, and 24 h after intravenous administration of the test substance (1 mg/kg), and blood samples were collected at 15 min, 0.5, 1, 2, 4, 6, 8, and 24 h after intragastric administration of the test substance (3 mg/kg). After anticoagulation with EDTA-K2, the blood samples were centrifuged at 3200 g at 4°C for 10 min to separate plasma, and all plasma samples were stored at -70°C for testing. The drug concentration of the test substance in plasma was determined by LC-MS/MS method (with gliclazide as an internal standard), and the main pharmacokinetic parameters were calculated by Winnolin 8.3 non-compartment model.

The results show that compared with the control compound, the compounds of the present invention have superior pharmacokinetic characteristics.

### 6. Study on serum phosphorus concentration in tumor-bearing mice

Human endometrial adenocarcinoma cell AN3CA (Procell, CL-0505) was cultured in an MEM medium containing 10% fetal bovine serum and 1% Penicillin-Streptomycin. When the cells were in the exponential growth phase, the cells were trypsinized, collected, counted, and then inoculated under the armpit of female BALB/c Nude mice. The amount of inoculated cells was 2.5-5 × 10⁶/animal and the inoculation volume was 100 µL. Once the tumor had grown to about 150 mm³, they were randomly divided into groups according to the tumor volume, with 6 animals per group. The test compound was formulated with 0.5% MC/2% TPGS and was intragastrically administered at an administration volume of 10 mL/kg. After the last administration, blood samples were collected from the orbital venous plexus of mice, and after centrifugation, serum was collected for phosphorus concentration detection. The grouping and administration regimen and the results are detailed in the following table.

| **Grouping and administration regimen** | | | | |
|---|---|---|---|---|
| Experime nt group | Treatment method | Test substance | Administration regimen | Results |
| Test I | Administration was performed for 6 days in total, and blood collection was performed 2 hours after the last drug administration. | **Blank group** | 0.5% MC/2% TPGS, BID | As shown in FIG. 1 |
| | | **Futibatinib** | 2 mg/kg, TID | |
| | | **Compound 1** | 30 mg/kg, BID | |
| Test II | Administration was performed for 13 days in total, and blood collection was performed 1 hours after the last drug administration. | **Blank group** | 0.5% MC/2% TPGS, BID | As shown in FIG. 2 |
| | | **Futibatinib** | 2 mg/kg, TID | |
| | | **Compound 4** | 10 mg/kg, BID | |

| | | | | |
|---|---|---|---|---|
| Note: The comparative example was **compound 343** from the patent WO 2020231990 A1 and was obtained according to a method for preparing **compound 343.** | | | | |

The results show that the serum phosphorus levels in the groups administered with the compounds of the present invention are both significantly lower than that in the first-generation FGFR2 inhibitor Futibatinib administration group. This indicates that the compound of the present invention has significant safety.

The specification of the present invention describes specific embodiments in detail, and those skilled in the art should realize that the above embodiments are exemplary and should not be understood as limiting the present invention. For those skilled in the art, without departing from the principle of the present invention, several improvements and modifications are made to the present invention, and the technical solutions obtained by these improvements and modifications also fall within the scope of protection of the claims of the present invention.

## Claims

1. A compound represented by general formula (I), or a pharmaceutically acceptable salt, stereoisomer, or deuterated compound thereof: 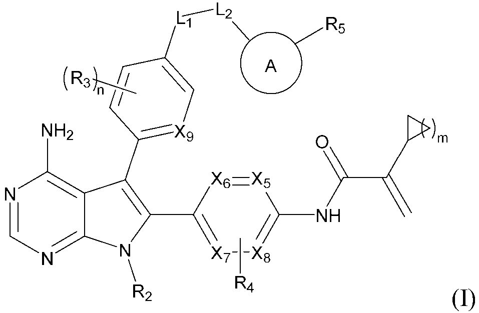 **characterized in that**:
X₅ is CH or N;
X₆ is CH or N;
X₇ is CH or N;
X₈ is CH or N;
X₉ is CH or N;
R₂ is C₁₋₆ alkyl;
each R₃ is independently H, halogen, cyano, C₁₋₆ alkyl, or C₁₋₆ alkoxy, wherein the C₁₋₆ alkyl is optionally substituted with 1 to 3 halogens;
R₄ is H, halogen, cyano, C₁₋₆ alkyl, or C₁₋₆ alkoxy, wherein the C₁₋₆ alkyl is optionally substituted with 1 to 3 halogens;
R₅ is H, halogen, cyano, C₁₋₆ alkyl, C₁₋₆ alkoxy, C₂₋₆ alkenyl, or C₂₋₆ alkynyl, wherein the C₁₋₆ alkyl, C₂₋₆ alkenyl, and C₂₋₆ alkynyl are optionally substituted with 1 to 3 substituents selected from halogen or C₁₋₆ alkyl;
L₁ is O, 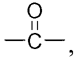 or -O(CO)-;
L₂ is a bond, 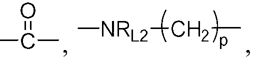 or C₁₋₆ alkyl;
R_{L2} is H or C₁₋₆ alkyl;
A is C₃₋₈ heterocycle or C₃₋₈ cycloalkyl, wherein the C₃₋₈ heterocycle contains 1 to 3 heteroatoms selected from N, O, or S, and the C₃₋₈ heterocycle is optionally substituted with carbonyl;
n is 0, 1, 2, or 3;
m is 1, 2, 3, or 4; and
p is 0, 1, or 2.

2. The compound or the pharmaceutically acceptable salt, stereoisomer, or deuterated compound thereof according to claim 1, **characterized in that**:
A is

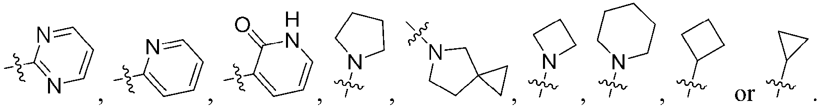

3. The compound or the pharmaceutically acceptable salt, stereoisomer, or deuterated compound thereof according to claim 1, **characterized in that** the compound has a structure represented by formula (I-1): 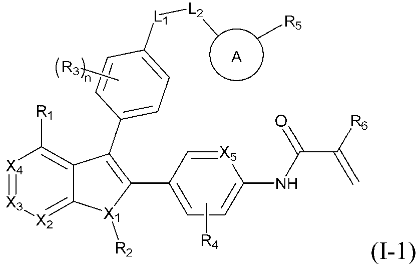 wherein:
X₁ is N;
X₂ is N;
X₃ is CH;
X₄ is N;
X₅ is CH or N;
R₁ is NH₂;
R₂ is C₁₋₆ alkyl;
each R₃ is independently H, halogen, cyano, or C₁₋₆ alkyl, wherein the C₁₋₆ alkyl is optionally substituted with 1 to 3 halogens;
R₄ is H or halogen;
R₅ is H, halogen, cyano, C₁₋₆ alkyl, C₂₋₆ alkenyl, or C₂₋₆ alkynyl, wherein the C₁₋₆ alkyl, C₂₋₆ alkenyl, and C₂₋₆ alkynyl are optionally substituted with 1 to 3 substituents selected from halogen or C₁₋₆ alkyl;
R₆ is C₃₋₆ cycloalkyl;
L₁ is O;
L₂ is a bond or 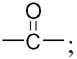
A is 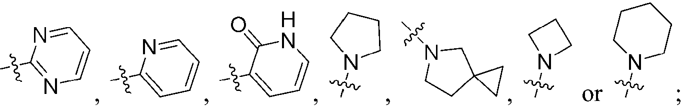 and
n is 0, 1, 2, or 3.

4. The compound or the pharmaceutically acceptable salt, stereoisomer, or deuterated compound thereof according to claim 3, **characterized in that** the compound has a structure represented by formula (I-2): 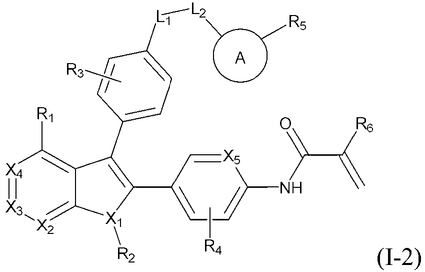 wherein:
X₁ is N;
X₂ is N;
X₃ is CH;
X₄ is N;
X₅ is CH or N;
R₁ is NH₂;
R₂ is C₁₋₆ alkyl;
R₃ is H, halogen, cyano, or C₁₋₆ alkyl, wherein the C₁₋₆ alkyl is optionally substituted with 1 to 3 halogens;
R₄ is H or halogen;
R₅ is H, halogen, cyano, C₁₋₆ alkyl, C₂₋₆ alkenyl, or C₂₋₆ alkynyl, wherein the C₁₋₆ alkyl, C₂₋₆ alkenyl, and C₂₋₆ alkynyl are optionally substituted with 1 to 3 substituents selected from halogen or C₁₋₆ alkyl;
R₆ is 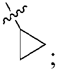
L₁ is O;
L₂ is a bond; and
A is

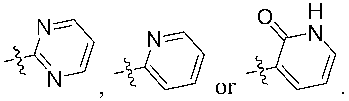

5. The compound or the pharmaceutically acceptable salt, stereoisomer, or deuterated compound thereof according to claim 1, **characterized in that** the compound has a structure represented by formula (1-2): 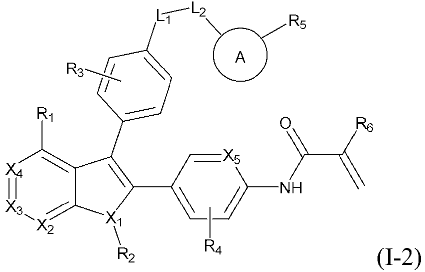 wherein:
X₁ is N;
X₂ is N;
X₃ is CH;
X₄ is N;
X₅ is CH or N;
R₁ is NH₂;
R₂ is C₁₋₆ alkyl;
R₃ is H, halogen, cyano, or C₁₋₆ alkyl, wherein the C₁₋₆ alkyl is optionally substituted with 1 to 3 halogens;
R₄ is H or halogen;
R₅ is H, halogen, cyano, C₁₋₆ alkyl, C₂₋₆ alkenyl, or C₂₋₆ alkynyl, wherein the C₁₋₆ alkyl, C₂₋₆ alkenyl, and C₂₋₆ alkynyl are optionally substituted with 1 to 3 substituents selected from halogen or C₁₋₆ alkyl;
R₆ is 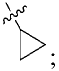
L₁ is -O(CO)-;
L₂ is a bond, NR_{L2}, or C₁₋₆ alkyl;
R_{L2} is H or C₁₋₆ alkyl; and
A is C₃₋₈ cycloalkyl or C₃₋₈ heterocycloalkyl, wherein the C₃₋₈ heterocycloalkyl contains 1 to 3 heteroatoms selected from N, O, or S.

6. The compound or the pharmaceutically acceptable salt, stereoisomer, or deuterated compound thereof according to any one of claims 1 to 5, **characterized in that** the compound is selected from the following structures:

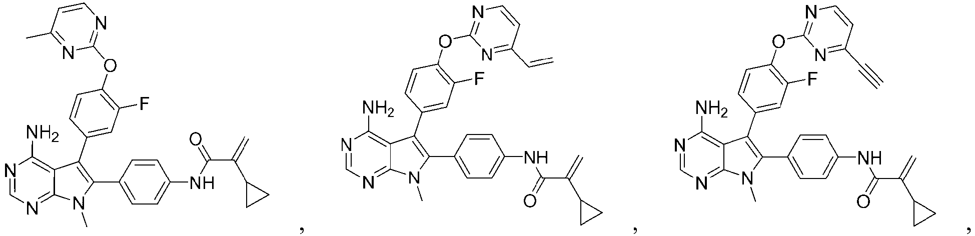

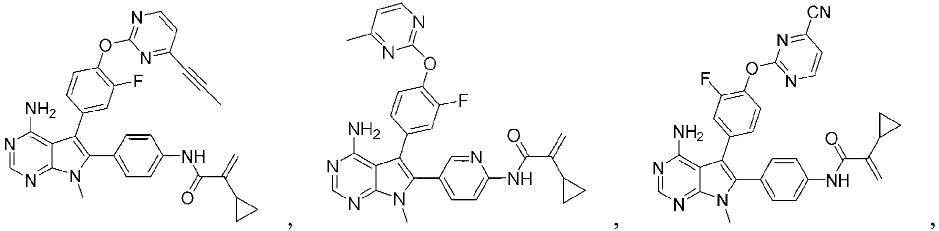

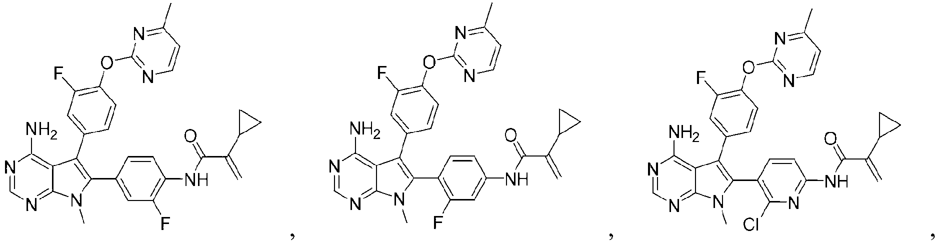

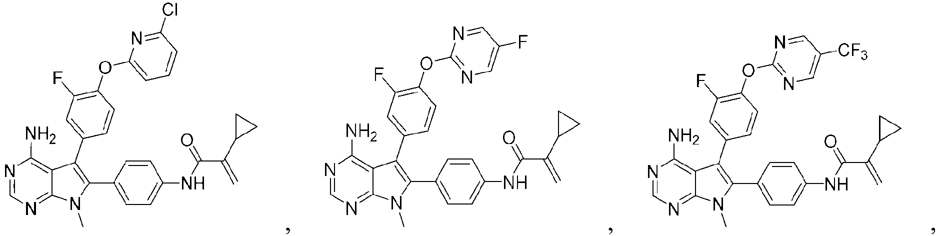

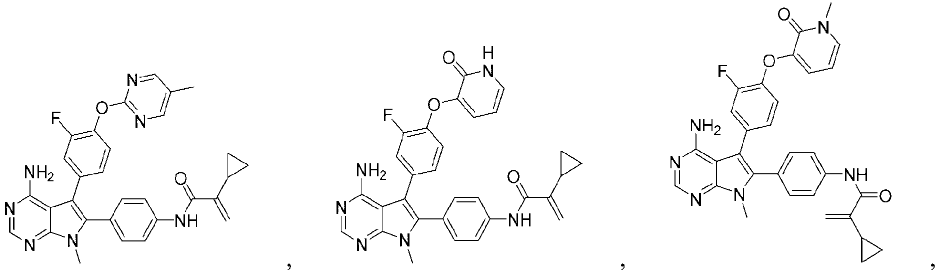

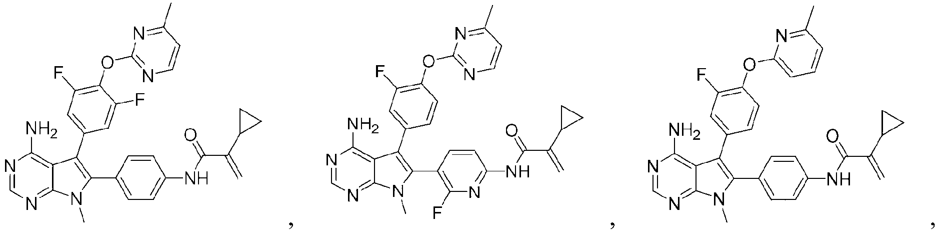

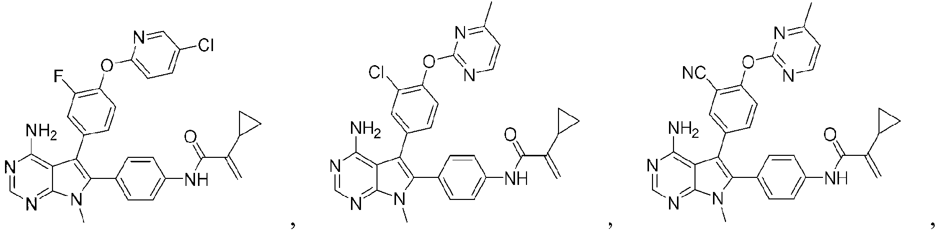

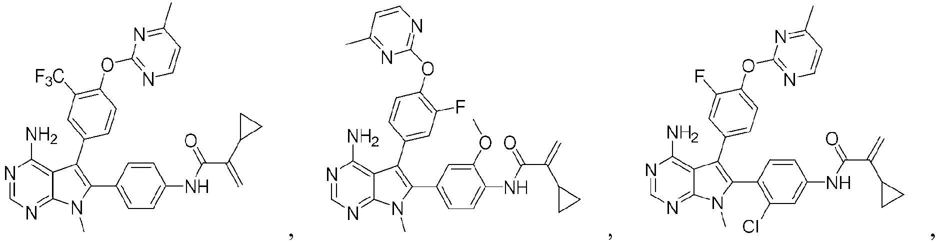

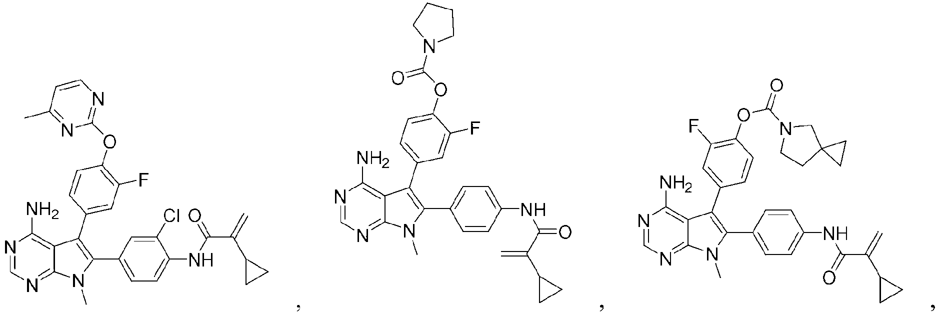

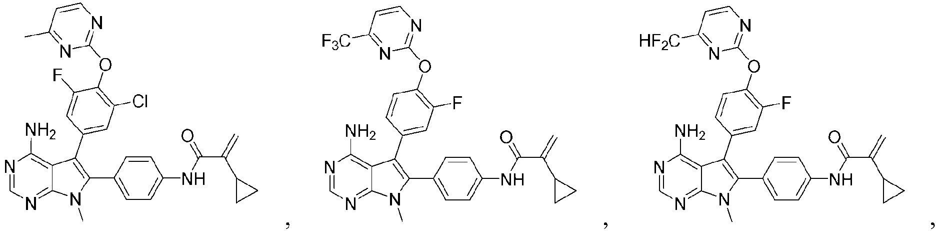

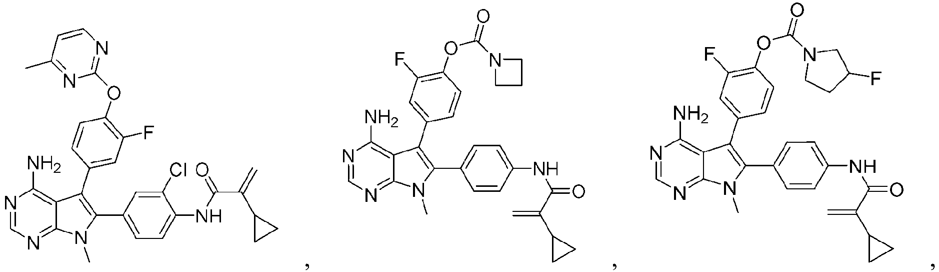

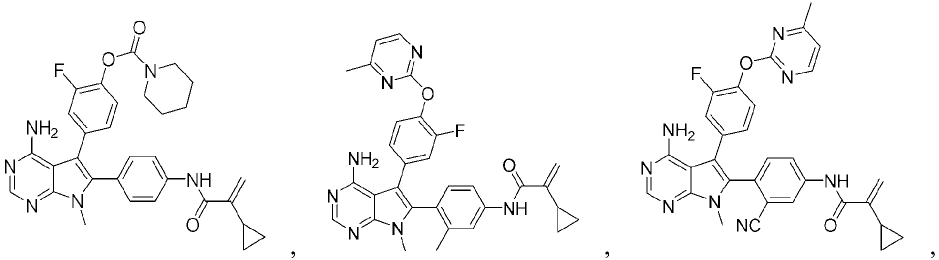

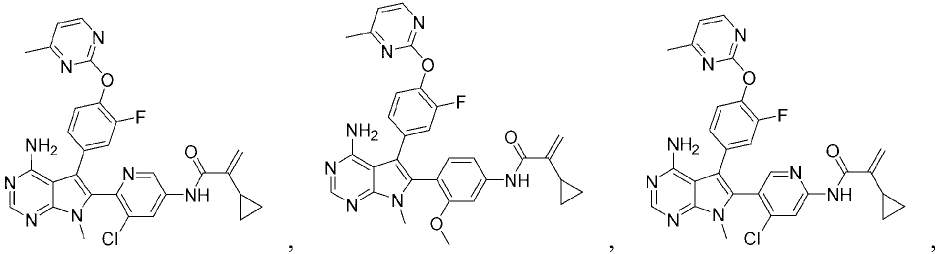

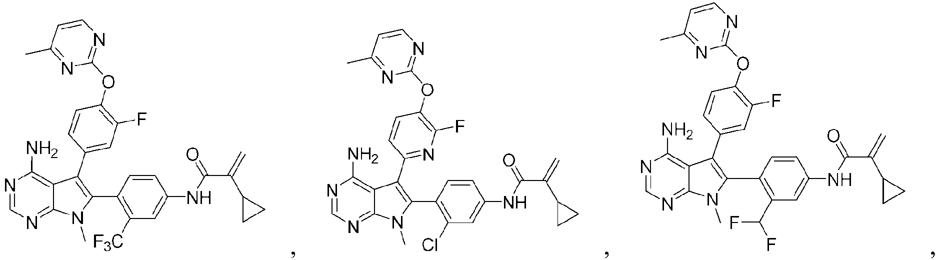

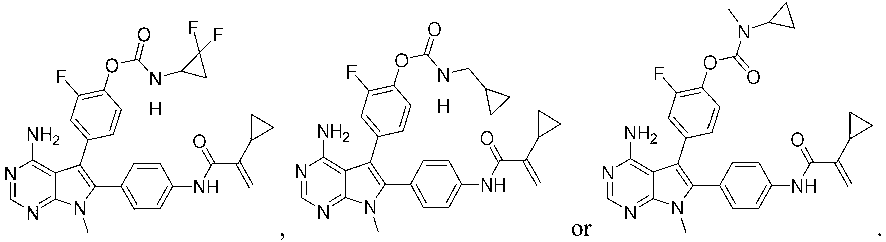

7. A pharmaceutical composition, **characterized by** comprising:
(1) the compound or the pharmaceutically acceptable salt, stereoisomer, or deuterated compound thereof according to any one of claims 1 to 6;
(2) optionally one or more additional active ingredients; and
(3) a pharmaceutically acceptable carrier and/or excipient.

8. Use of the compound or the pharmaceutically acceptable salt, stereoisomer, or deuterated compound thereof according to any one of claims 1 to 6 or the pharmaceutical composition according to claim 7 in the preparation of an anti-tumor drug.
